Europäisches Patentamt
European Patent Office
Office européen des brevets

(19)

(11) Veröffentlichungsnummer: **0 302 826 A2**

(12)

# EUROPÄISCHE PATENTANMELDUNG

(21) Anmeldenummer: 88810517.8

(22) Anmeldetag: 27.07.88

(51) Int. Cl.4: C 07 F 9/30
C 07 F 9/48, C 07 F 9/38,
A 61 K 31/66

(30) Priorität: 04.08.87 CH 2986/87

(43) Veröffentlichungstag der Anmeldung:
08.02.89 Patentblatt 89/06

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

(72) Erfinder: Allgeier, Hans, Dr.
Lichsenweg 20
D-7850 Lörrach-Haagen (DE)

Angst, Christof, Dr.
13 Lowell Avenue
Summit New Jersey (US)

Bold, Guido, Dr.
Hauptstrasse 125
CH-5262 Frick (CH)

Duthaler, Rudolf, Dr.
Girenhaldenweg 17
CH-4126 Bettingen (CH)

Heckendorn, Roland, Dr.
Blumenweg 20
CH-4144 Arlesheim (CH)

Togni, Antonio, Dr.
Im Lee 20
CH-4144 Arlesheim (CH)

Patentansprüche für folgende Vertragsstaaten: ES + GR.

(54) **Verfahren zur Herstellung neuer ungesättigter Aminosäureverbindungen.**

(57) Die Erfindung betrifft Verfahren zur Herstellung ungesättigter Aminosäureverbindungen der Formel

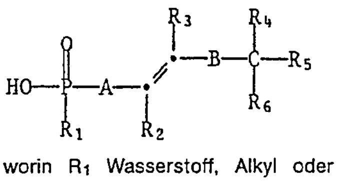

(I),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für gegebenenfalls verestertes oder amidiertes Carboxy steht, $R_6$ eine unsubstituierte durch Niederalkyl oder Arylniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, und ihrer Salze, dadurch gekennzeichnet, dass man in einer Verbindung der Formel

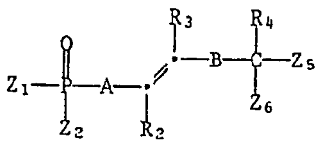

(II),

worin $Z_1$ gegebenenfalls geschütztes Hydroxy bedeutet, $Z_2$ eine Gruppe $R_1$ oder geschütztes Hydroxy bedeutet, $Z_5$ eine Gruppe $R_5$ oder geschütztes Carboxy bedeutet, $Z_6$ eine geschützte Gruppe $R_6$ darstellt und $R_1$, $R_2$, $R_3$, $R_4$, $R_5$, $R_6$, A und B die vorstehend angegebenen Bedeutungen haben, die geschützten Gruppe $Z_6$ und gegebenenfalls $Z_1$, $Z_2$ und/oder $Z_5$ durch Behandeln mit einem Triniederalkylhalogensilan freisetzt und gewünschtenfalls die erhaltene Verbindung in eine andere Verbindung der Formel I umwandelt, ein erhaltenes Gemisch optischer Isomeren in die Komponenten auftrennt und das gewünschte Isomer abtrennt und/oder eine erhaltene freie Verbindung in ein Salz oder ein erhaltenes Salz in die freie Verbindung oder in ein anderes Salz überführt. Die Erfindung betrifft auch den (2R)-E-2-Amino-4-methyl-5-phosphono-3-pentensaureethylester und seine Salze.

EP 0 302 826 A2

**Beschreibung**

## Verfahren zur Herstellung neuer ungesättigter Aminosäureverbindungen

Die Erfindung betrifft ein Verfahren zur Herstellung ungesättigter Aminosäureverbindungen der Formel

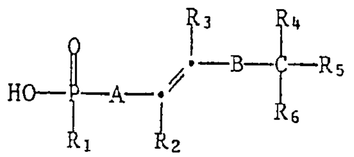

(I),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für gegebenenfalls verestertes oder amidiertes Carboxy steht, $R_6$ eine unsubstituierte oder durch Niederalkyl oder Arylniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, und ihrer Salze sowie (2R)-E-2-Amino-4-methyl-5-phosphono-3-pentensäureethylester als solchen.

Die Verbindungen der Formel I enthalten mindestens ein chirales Zentrum und können als Enantiomere oder als Enantiomerengemische, wie Racemate, vorliegen, sofern sie mehr als ein chirales Zentrum aufweisen, auch als Diastereomere oder als Diastereomerengemische. Die Kohlenstoff-Kohlenstoff-Doppelbindung der erfindungsgemässen Verbindungen ist bezüglich $R_2$ und $R_3$ beziehungsweise bezüglich A und B trans-konfiguriert, d.h. es handelt sich bei den Verbindungen der Formel I um die Verbindungen der E-Reihe.

Verbindungen der Formel I, in denen $R_1$ Wasserstoff bedeutet, sind Phosphonige Säuren (engl.: phosphonous acids), solche in denen $R_1$ Alkyl bedeutet, sind Phosphinsäuren (engl.: phosphinic acids), solche in denen $R_1$ für Hydroxy steht, sind Phosphonsäuren (engl.: phosphonic acids). Als Präfixe in der Benennung der Verbindungen der Formel I, die als substituierte Carbonsäuren anzusprechen sind, werden "Phosphino" ($R_1$ bedeutet Wasserstoff), "Phosphonyl" ($R_1$ bedeutet Alkyl) und "Phosphono" ($R_1$ bedeutet Hydroxy) verwendet.

Verestertes Carboxy ist beispielsweise mit einem aliphatischen oder araliphatischen Alkohol, wie einem gegebenenfalls substituierten Niederalkanol oder Phenylniederalkanol, verestertes Carboxy, wie entsprechendes Niederalkoxy- oder Phenylniederalkoxycarbonyl. Vorzugsweise handelt es sich hierbei um pharmazeutisch annehmbares verestertes Carboxy, wie z.B. verestertes Carboxy, das unter physiologischen Bedingungen in Carboxy übergeführt werden kann. Diese Ester der Formel I können auch als Prodrug-Ester bezeichnet werden.

In pharmazeutisch annehmbarer Weise verestertes Carboxy bedeutet beispielsweise Niederalkoxycarbonyl; in höherer als der α-Stellung durch Amino, Mono- oder Diniederalkylamino oder Hydroxy substituiertes Niederalkoxycarbonyl; durch Carboxy substituiertes Niederalkoxycarbonyl, z.B. α-Carboxy-substituiertes Niederalkoxycarbonyl; durch Niederalkoxycarbonyl substituiertes Niederalkoxycarbonyl, z.B. α-Niederalkoxycarbonyl-substituiertes Niederalkoxycarbonyl, Arylniederalkoxycarbonyl, z.B. unsubstituiertes oder substituiertes Benzyloxycarbonyl, oder Pydridylmethoxycarbonyl; Niederalkanoyloxy-substituiertes Methoxycarbonyl, z.B. Pivaloyloxymethoxycarbonyl; durch Niederalkanoyloxy oder Niederalkoxy substituiertes Niederalkoxymethoxycarbonyl; Bicyclo[2.2.1]heptyloxycarbonyl-substituiertes Methoxycarbonyl, wie Bornyloxycarbonylmethoxycarbonyl; 3-Phthalidoxycarbonyl; durch Niederalkyl, Niederalkoxy oder Halogen substituiertes 3-Phthalidoxycarbonyl; oder Niederalkoxycarbonyloxy-niederalkoxycarbonyl, z.B. 1-(Methoxy- oder Ethoxycarbonyloxy-)ethoxycarbonyl.

Besonders bevorzugte Prodrug-Ester sind z.B. die Niederalkylester mit bis zu vier Kohlenstoffatomen, wie z.B. Butyl- oder Ethylester, die Niederalkanoyloxymethylester, wie z.B. Pivaloyloxymethylester, die in höherer als der α-Stellung durch Diniederalkylamino substituierten Niederalkylester mit zwei bis vier Kohlenstoffatomen in jeder Niederalkylgruppe wie z.B. 2-Diethylaminoethylester, sowie Pyridylmethylester wie 3-Pyridylmethylester.

In amidiertem Carboxy bedeutet die Aminogruppe beispielsweise gegebenenfalls durch Hydroxy monosubstituiertes oder durch aliphatische Reste mono- oder disubstituiertes Amino, wie Amino, Hydroxyamino, Mono- oder Diniederalkylamino oder Niederalkylenamino mit 5 bis 7 Ringgliedern. Vorzugsweise handelt es sich hierbei um pharmazeutisch annehmbares amidiertes Carboxy, wie z.B. um amidiertes Carboxy, das unter physiologischen Bedingungen in Carboxy übergeführt werden kann.

Bevorzugte pharmazeutisch annehmbare Amide sind Verbindungen der Formel I, in denen $R_5$ Carbamoyl, Niederalkylcarbamoyl, z.B. Ethylcarbamoyl, Diniederalkylcarbamoyl, z.B. Diethylcarbamoyl, oder N-(Diniederalkylamino)niederalkylcarbamoyl, z.B. als N-(2-Diethylaminoethyl)carbamoyl oder als N-(3-Diethylaminopropyl)carbamoyl, vorliegt.

Alkyl bedeutet im Rahmen dieser Erfindung einen gesättigten aliphatischen Kohlenwasserstoffrest mit z.B. bis zu 12 Kohlenstoffatomen, insbesondere mit bis zu 8 Kohlenstoffatomen, wobei der letztgenannte Bereich auch durch den Begriff Niederalkyl wiedergegeben wird.

α,ω-Alkylen mit 1 bis 3 Kohlenstoffatomen ist Methylen, 1,2-Ethylen oder 1,3-Propylen. Durch Alkyl substituiertes α,ω-Alkylen ist an einer beliebigen Position substituiert. So bedeutet durch Alkyl substituiertes Methylen z.B. 1,1-Ethylen, 1,1-Butylen oder 1,1-Octylen, durch Alkyl substituiertes 1,2-Ethylen z.B. 1,2-Propylen, 1,2-Butylen, 2,3-Butylen, 1,2-Pentylen oder 1,2-Nonylen, und durch Alkyl substituiertes 1,3-Propylen z.B. 1,3-Butylen, 1,3-Pentylen oder 1,3-Decylen.

Durch Niederalkyl oder Arylniederalkyl substituiertes Amino ist Mono-oder Diniederalkylamino oder Arylniederalkylamino.

Aryl steht, auch in Definitionen wie Aroyl oder Arylniederalkoxycarbonyl, für aromatische Kohlenwasserstoffreste, die unsubstituiert oder durch Niederalkyl, Hydroxy, geschütztes Hydroxy, Niederalkoxy, Halogen, Amino, Halogenniederalkyl, Hydroxyniederalkyl, Aminoniederalkyl oder Nitro substituiert sind und bedeutet z.B. unsubstituiertes oder entsprechend substituiertes 1- oder 2-Naphthyl, vorzugsweise jedoch unsubstituiertes oder entsprechend substituiertes Phenyl, wie Phenyl, Niederalkylphenyl, z.B. Methylphenyl, Hydroxyphenyl, Halogenphenyl, z.B. 4-Halogenphenyl, wie 4-Chlorphenyl, Benzyloxyphenyl, Niederalkoxyphenyl, z.B. Methoxyphenyl, Hydroxymethylphenyl, Aminomethylphenyl oder Nitrophenyl.

Die vor- und nachstehend verwendeten Allgemeinbegriffe haben, sofern sie nicht abweichend definiert sind, die folgenden Bedeutungen:

Der Ausdruck "nieder" bedeutet, dass entsprechend definierte Gruppen oder Verbindungen bis und mit 8, vorzugsweise bis und mit 4 Kohlenstoffatome enthalten.

Niederalkyl bedeutet z.B. $C_1$-$C_4$-Alkyl, wie Methyl, Ethyl, n-Propyl, Isopropyl, n-Butyl, Isobutyl oder tert.-Butyl, kann aber auch $C_5$-$C_8$-Alkyl, wie n-Pentyl, n-Hexyl, n-Heptyl oder n-Octyl bedeuten, ist aber bevorzugt Methyl.

Arylniederalkyl bedeutet, auch in Definitionen wie Arylniederalkylamino, z.B. Aryl-$C_1$-$C_4$-alkyl, worin Aryl die vorstehend angegebenen Bedeutungen hat, und steht in erster Linie z.B. für unsubstitutiertes Phenyl-$C_1$-$C_4$-alkyl, wie Benzyl oder 1- oder 2-Phenylethyl.

Niederalkenyl enthält vorzugsweise bis zu 6 Kohlenstoffatome und ist über ein $sp^3$-hybridisiertès Kohlenstoffatom gebunden, und bedeutet z.B. 2-Propenyl, 2- oder 3-Butenyl oder 3-Pentenyl, kann aber auch Vinyl sein.

Niederalkoxy steht in erster Linie für $C_1$-$C_4$-Alkoxy, wie Methoxy, Ethoxy, n-Propoxy, Isopropoxy, n-Butoxy, Isobutoxy oder tert.-Butoxy.

Halogen hat vorzugsweise eine Atomnummer bis 35 und ist insbesondere Chlor, ferner Fluor oder Brom, kann jedoch auch Iod sein.

Halogenniederalkyl ist z.B. Halogen-$C_1$-$C_4$-alkyl, wie Fluormethyl, Trifluormethyl oder 1- oder 2-Chlorethyl.

Hydroxyniederalkyl ist z.B. Mono- oder Dihydroxy-$C_1$-$C_7$-alkyl, trägt die Hydroxygruppe(n) insbesondere in höherer als der α-Stellung und bedeutet z.B. Hydroxymethyl oder insbesondere Mono- oder Dihydroxy-$C_2$-$C_7$-alkyl, wie 2-Hydroxyethyl, 3-Hydroxy- oder 2,3-Dihydroxy-propyl, 4-Hydroxy- oder 2,4-Dihydroxybutyl, 5-Hydroxy-, 2,5-Dihydroxy-oder 3,5-Dihydroxy-pentyl.

Niederalkoxyniederalkyl ist z.B. Mono- oder Di-$C_1$-$C_4$-alkoxy-$C_1$-$C_7$-alkyl, trägt die Niederalkoxygruppe(n) beispielsweise insbesondere in höherer als der α-Stellung und ist insbesondere $C_1$-$C_4$-Alkoxy-$C_2$-$C_4$-alkyl, z.B. 2-Methoxy-, 2-Ethoxy-, 2-Propoxy- oder 2-Isopropoxy-ethyl, 3-Methoxy-oder 3-Ethoxy-propyl oder 3,3-Dimethoxy-, 3,3-Diethoxy-, 2,3-Dimethoxy-oder 2,3-Diethoxypropyl oder 4,4-Dimethoxy-butyl, kann aber auch Methoxy-, Ethoxy-, Dimethoxy- oder Propoxy-oder Isopropoxymethyl sein.

Mono- oder Diniederalkylamino ist z.B. N-$C_1$-$C_4$-Alkyl- oder N,N-Di-$C_1$-$C_4$-Alkylamino, wie Methylamino, Dimethylamino, Ethylamino, Diethylamino, Propylamino, Isopropylamino oder Butylamino.

Salze von erfindungsgemässen Verbindungen sind in erster Linie pharmazeutisch verwendbare, nichttoxische Salze von Verbindungen der Formel I. Solche Salze werden beispielsweise von der in Verbindungen der Formel I vorhandenen Carboxygruppe gebildet und sind in erster Linie Metall- oder Ammoniumsalze, wie Alkalimetall- und Erdalkalimetall-, z.B. Natrium-, Kalium-, Magnesium- oder Calziumsalze, sowie Ammoniumsalze mit Ammoniak oder geeigneten organischen Aminen, wie Niederalkylaminen, z.B. Methylamin, Diethylamin oder Triethylamin, Hydroxyniederalkylaminen, z.B. 2-Hydroxyethylamin, Bis-(2-hydroxyethyl)-amin, Tris-(hydroxymethyl)methylamin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Niederalkylendiaminen, z.B. Ethylendiamin, Cycloalkylaminen, z.B. Dicyclohexylamin, oder Benzylaminen, z.B. N,N′-Dibenzylethylendiamin, Benzyltrimethylammoniumhydroxid, Dibenzylamin oder N-Benzyl-β-phenylethylamin. Verbindungen der Formel I mit primärer oder sekundärer Aminogruppe können auch Säureadditionssalze, z.B. mit vorzugsweise pharmazeutisch annehmbaren anorganischen Säuren, wie Halogenwasserstoffsäuren, z.B. Salzsäure oder Bromwasserstoffsäure, Schwefelsäure, Salpetersäure oder Phosphorsäure, oder mit geeigneten organischen Carbon- oder Sulfonsäuren, z.B. Essigsäure, Propionsäure, Bernsteinsäure, Glykolsäure, Milchsäure, Fumarsäure, Maleinsäure, Weinsäure, Oxalsäure, Zitronensäure, Brenztraubensäure, Benzoesäure, Mandelsäure, Aepfelsäure, Ascorbinsäure, Pamoasäure, Nicotinsäure, Methansulfonsäure, Ethansulfonsäure, Hydroxyethansulfonsäure, Benzolsulfonsäure, 4-Toluolsulfonsäure oder Naphthalinsulfonsäure bilden.

Zur Isolierung oder Reinigung können auch pharmazeutisch ungeeignete Salze Verwendung finden. Zur

therapeutischen Anwendung gelangen nur die pharmazeutisch verwendbaren, nicht-toxischen Salze, die deshalb bevorzugt sind.

Die erfindungsgemäss bereitgestellten Verbindungen weisen wertvolle pharmakologische Eigenschaften auf. So sind sie beispielsweise aktive und selektive Antagonisten von für N-Methyl-D-asparaginsäure (NMDA) sensitiven excitatorischen Aminosäure-Rezeptoren in Säugern. Sie sind daher geeignet für die Behandlung von Krankheiten, die auf eine Blockierung von NMDA sensitiven Rezeptoren ansprechen, wie z.B. cerebrale Ischämie, Muskelspasmen (Spastizität), Konvulsionen (Epilepsie), Angst- und Schmerzzustände oder manische Zustände.

Diese vorteilhaften Wirkungen lassen sich in in vitro- oder in in vivo-Testanordnungen demonstrieren. Dabei werden vorzugsweise Säuger verwendet, z.B. Mäuse, Ratten oder Affen, oder Gewebe oder Enzympräparate von solchen Säugern. Die Verbindungen können enteral oder parenteral verabreicht werden, vorzugsweise oral; oder subkutan, intravenös oder intraperitoneal, z.B. in Gelatinekapseln oder in Form von wässrigen Suspensionen oder Lösungen. Die zu verwendende in vivo-Dosis kann sich zwischen 0,1 und 600 mg/kg bewegen, vorzugsweise zwischen 1 und 300 mg/kg. In vitro können die Verbindungen in Form wässriger Lösungen verwendet werden, wobei sich die Konzentrationen zwischen $10^{-4}$ molaren und $10^{-8}$ molaren Lösungen bewegen können.

Die inhibierende Wirkung auf NMDA-sensitiven excitatorischen Aminosäurerezeptoren lässt sich in vitro durch Messung bestimmen, in welchem Ausmass die Bindung von L-$^{3}$H-Glutaminsäure [G. Fagg und A. Matus, Proc. Nat. Acad. Sci., USA, 81, 6876-80 (1984)] oder $^{3}$H-3-(2-Carboxypiperazin-4-yl)propyl-1-phosphonsäure [Murphy et al., J. Pharmakol. exp. Ther. 240, 778-784 (1987)] an NMDA-sensitiven Rezeptoren inhibiert wird. In vivo lässt sich die inhibierende Wirkung auf NMDA-sensitive excitatorische Aminosäurerezeptoren anhand der Hemmung der NMA-induzierten Zunahme der enuronalen Entladungsfrequenz bei Ratten oder anhand der Hemmung von NMDA-induzierten Konvulsionen der Maus demonstrieren.

Die antikonvulsiven Eigenschaften der erfindungsgemässen Verbindungen lassen sich ferner durch ihre Wirksamkeit bei der Verhinderung von audiogen hervorgerufenen Anfällen in DBA/2 Mäusen zeigen (Chapman et al., Arzneimittel-Forsch. 34, 1261, 1984).

Die antikonvulsiven Eigenschaften lassen sich ferner durch die Wirksamkeit der erfindungsgemässen Verbindungen als Elektroschockantagonisten an der Maus oder an der Ratte zeigen.

Einen Hinweis auf die anxiolytische Aktivität der Verbindungen der vorliegenden Erfindung gibt ihre gute Wirksamkeit im Konflikt-Modell nach Cook/Davidson (Psychopharmacologia 15, 159-168 (1968)).

Die gute Wirksamkeit der Verbindungen der Formel I hängt in überraschend hohem Ausmass von der Konfiguration an der Doppelbindung ab. So erweist sich das Racemat der aus Agric. Biol. Chem. 41, 573 - 579 (1979), B. K. Park et al., bekannten D-2-Amino-5-phosphono-3-cis-pentensäure z.B. in seiner Fähigkeit, an den NMDA-sensitiven Rezeptor zu binden, dem Racemat der erfindungsgemäss bereitgestellten 2-Amino-5-phosphono-3-trans-pentensäure (im Beispielteil werden diese Verbindungen als Verbindungen der "E-Reihe" bezeichnet) als weit unterlegen. Als besonders wirksam haben sich Verbindungen der Formel I herausgestellt, in denen das die Gruppe $R_6$ tragende C-Atom R-Konfiguration besitzt.

Bevorzugt ist die Herstellung von Verbindungen der Formel I, worin $R_2$ für Wasserstoff, Alkyl oder Aryl steht.

Die Erfindung betrifft in erster Linie ein Verfahren zur Herstellung von Verbindungen der Formel I, worin $R_1$ Wasserstoff, Alkyl mit bis und mit 12 Kohlenstoffatomen oder Hydroxy bedeutet, $R_2$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R_3$ Wasserstoff, Niederalkyl oder unsubstituiertes oder substituiertes Phenyl bedeutet, $R_4$ für Wasserstoff oder Niederalkyl steht, $R_5$ freies oder pharmazeutisch annehmbares verestertes oder amidiertes Carboxy bedeutet, $R_6$ für Amino, Mono- oder Diniederalkylamino steht, A unsubstituiertes oder durch Niederalkyl substituiertes $\alpha,\omega$-Alkylen mit 1 bis 3 Kohlenstoffatomen oder eine Bindung bedeutet, und B Methylen oder eine Bindung bedeutet, mit der Massgabe, dass A von einer Bindung verschieden ist, wenn B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro und niedere Reste beispielsweise bis und mit 8 C-Atome aufweisen, sowie von pharmazeutisch annehmbaren Salzen davon.

Dabei ist die Anwendung des Verfahrens zur Herstellung von Verbindungen der Formel I bevorzugt, worin $R_1$ bis $R_4$, A und B wie vorstehend definiert sind und einerseits $R_5$ Niederalkoxycarbonyl oder durch Amino, Mono- oder Diniederalkylamino, Hydroxy oder Niederalkanoyloxy substituiertes Niederalkoxycarbonyl bedeutet und $R_6$ für Amino oder Niederalkylamino steht oder andrerseits $R_5$ Carboxy und $R_6$ Amino bedeutet, insbesondere von deren bezüglich des Atoms, das die Aminogruppe trägt, R-Enantiomeren, sowie von pharmazeutisch annehmbaren Salzen davon.

Ebenfalls bevorzugt ist die Anwendung des Verfahrens zur Herstellung von Verbindungen der Formel I, worin $R_1$, $R_4$ bis $R_6$ sowie A und B wie un mittelbar vorstehend definiert sind und worin $R_2$ und $R_3$ unabhängig voneinander Wasserstoff, Niederalkyl, Phenyl oder durch Niederalkyl, Hydroxy, Niederalkoxy, Halogen, Amino, Halogenniederalkyl, Hydroxyniederalkyl, Aminoniederalkyl oder Nitro substituiertes Phenyl bedeuten, insbesondere von deren bezüglich des Atoms, das die Aminogruppe trägt, R-Enantiomeren, und von pharmazeutisch annehmbaren Salzen davon.

Die Erfindung betrifft vor allem ein Verfahren zur Herstellung von Verbindungen der Formel I, worin $R_1$ für Wasserstoff, Alkyl mit bis und mit 12 Kohlenstoffatomen oder Hydroxy steht, $R_2$ für Wasserstoff, Niederalkyl,

Phenyl, Halogenphenyl oder Phenylniederalkyl steht, $R_3$ und $R_4$ Wasserstoff oder Niederalkyl bedeuten, und einerseits $R_5$ für Alkoxycarbonyl oder Hydroxyniederalkoxycarbonyl und $R_6$ für Amino oder Mononiederalkylamino steht oder andrerseits $R_5$ Carboxy und $R_6$ Amino bedeutet, wobei jeweils A für unsubstituiertes oder durch Niederalkyl substituiertes α,ω-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, insbesondere von deren bezüglich des Atoms, das die Aminogruppe trägt, R-Enantiomeren, und von pharmazeutisch annehmbaren Salzen davon.

Die Erfindung betrifft besonders ein Verfahren zur Herstellung von Verbindungen der Formel I, worin $R_1$ für Wasserstoff, Alkyl mit bis und mit 12 C-Atomen oder Hydroxy steht, $R_2$ Wasserstoff, Niederalkyl oder Halogenphenyl bedeutet, $R_3$ für Wasserstoff oder Halogenphenyl steht, $R_4$ Wasserstoff bedeutet und einerseits $R_5$ Niederalkoxycarbonyl oder Hydroxyniederalkoxycarbonyl und $R_6$ Amino oder Mononiederalkylamino bedeutet oder andrerseits $R_5$ Carboxy und $R_6$ Amino bedeutet, wobei jeweils A für α,ω-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, insbesondere von deren bezüglich des Atoms, das die Aminogruppe trägt, R-Enantiomeren, und von pharmazeutisch annehmbaren Salzen davon.

Die Erfindung betrifft insbesondere ein Verfahren zur Herstellung von Verbindungen der Formel I, worin $R_1$ für Wasserstoff, Niederalkyl oder Hydroxy steht, $R_2$ Wasserstoff oder Niederalkyl bedeutet, $R_3$ und $R_4$ für Wasserstoff stehen und einerseits $R_5$ für Niederalkoxycarbonyl oder andrerseits für Carboxy steht und $R_6$ Amino bedeutet, wobei jeweils A für α,ω-Alkylen mit 1 bis 3 Kohlenstoffatomen steht, B eine Bindung bedeutet, insbesondere von deren bezüglich des Atoms, das die Aminogruppe trägt, R-Enantiomeren sowie pharmazeutisch annehmbare Salze davon.

Die Erfindung betrifft in allererster Linie ein Verfahren zur Herstellung von Verbindungen der Formel I, worin $R_1$ Hydroxy bedeuten, $R_2$ für Wasserstoff oder Niederalkyl steht, $R_3$ und $R_4$ für Wasserstoff stehen und $R_5$ Niederalkoxycarbonyl oder Carboxy bedeutet, $R_6$ für Amino steht, A Methylen und B eine Bindung bedeutet, insbesondere von deren bezüglich des Atoms, das die Aminogruppe trägt, R-Enantiomeren, sowie von pharmazeutisch annehmbaren Salzen davon.

Die Erfindung betrifft namentlich die Herstellung der in den Beispielen genannten Verbindungen und ihrer pharmazeutisch annehmbaren Salze.

Das erfindungsgemässe Verfahren ist dadurch gekennzeichnet, dass man in einer Verbindung der Formel

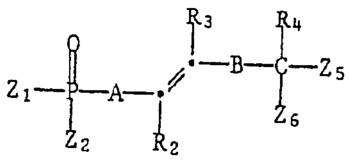

(II),

worin $Z_1$ gegebenenfalls geschütztes Hydroxy bedeutet, $Z_2$ eine Gruppe $R_1$ oder geschütztes Hydroxy bedeutet, $Z_5$ eine Gruppe $R_5$ oder geschütztes Carboxy bedeutet, $Z_6$ eine geschützte Gruppe $R_6$ darstellt und $R_1$, $R_2$, $R_3$, $R_4$, $R_5$, $R_6$, A und B die vorstehend angegebenen Bedeutungen haben, die geschützten Gruppen $Z_6$ und gegebenenfalls $Z_1$, $Z_2$ und/oder $Z_5$ durch Behandeln mit einem Triniederalkylhalogensilan freisetzt und gewünschtenfalls die erhaltene Verbindung in eine andere Verbindung der Formel I umwandelt, ein erhaltenes Gemisch optischer Isomeren in die Komponenten auftrennt und das gewünschte Isomer abtrennt und/oder eine erhaltene freie Verbindung in ein Salz oder ein erhaltenes Salz in die freie Verbindung oder in ein anderes Salz überführt.

Geschütztes Hydroxy $Z_1$ und/oder $Z_2$ in Zwischenprodukten der Formel II ist beispielsweise mit einem aliphatischen Alkohol verethertes Hydroxy, wie mit einem gegebenenfalls durch Halogen oder in höherer als der α-Stellung durch Hydroxy, Oxo, Niederalkoxy, Niederalkanoyloxy und/oder Mono- oder Diniederalkylamino substituierten Niederalkanol, Niederalkenol oder Niederalkinol verethertes Hydroxy und bedeutet beispielsweise Niederalkoxy, Halogenniederalkoxy, oder entsprechendes Hydroxy-, Oxo-, Niederalkoxy-, Niederalkanoyloxy oder Mono- oder Diniederalkylaminoniederalkoxy. Verbindungen der Formel II, in denen $Z_1$ und/oder $Z_2$ verethertes Hydroxy bedeuten, sind Ester der phosphorhaltigen Säuregruppe und je nach der Bedeutung von $R_1$ Phosphonigsäureester, Phosphinsäureester oder Phosphonsäureester. Bevorzugte Ester sind die jeweiligen Niederalkylester und Hydroxyniederalkylester.

Gruppen $Z_6$ in Zwischenprodukten der Formel II sind beispielsweise durch Acyl substituierte Gruppen $R_6$, d.h. gegebenenfalls durch Niederalkyl oder Arylniederalkyl N-substituiertes Acylamino, worin Acyl den Acylrest einer organischen Säure mit z.B. bis zu 18 Kohlenstoffatomen, insbesondere einer gegebenenfalls, z.B. durch Halogen, Amino oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Tod-, 2,2,2-Trifluor- oder 2,2,2-Trichloracetyl, Aroyl, wie gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder vor allem gegebenenfalls in 1- oder 2-Stellung substituiertes Niederalkoxycarbonyl geeignet, wie insbesondere Niederalkoxycarbonyl, z.B. Tertiärbutoxy-, ferner Methoxy- oder Ethoxycarbonyl, ferner gegebenenfalls substituiertes Benzyloxycarbonyl, z.B. Benzyloxycarbonyl oder 4-Nitrobenzyloxycarbonyl, oder Aroylmethoxycarbonyl, worin die Aroylgruppe gegebenenfalls, z.B. durch Halogen, wie Brom substituiertes Benzoyl darstellt, z.B. Phenacyloxycarbonyl oder Bromphenacyloxycarbonyl.

Ferner kann in einer entsprechenden Acylaminogruppe $Z_6$ Acyl für durch Amino und/oder Phenyl, Carbamoyl, Carboxy, Imidazolyl, Niederalkylthio, Tetrahydropyrrolyl, Hydroxy, Indolyl oder Hydroxyphenyl substituiertes Alkanoylamino stehen, so dass darunter z.B. die Acylreste von Aminosäuren zu verstehen sind, z.B. der natürlich vorkommenden Aminosäuren, wie Alanyl, Asparaginyl, Aspartyl, Glycyl, Histidyl, Isoleucyl, Leucyl, Lysyl, Methionyl, Phenylalanyl, Prolyl, Seryl, Threonyl, Tryptophyl, Tyrosyl oder Valyl; ebenso fallen darunter die Acylreste von Oligopeptiden, z.B. Di- oder Tripeptiden, wie Oligopeptide aus Alanin, Asparagin oder Asparaginsäure.

Weiterhin kann geschütztes Amino $Z_6$ eine Diacylaminogruppe darstellen. In dieser bedeutet Diacyl z.B. zwei Acylreste wie vorstehend definiert, oder Diacyl ist beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimido.

Darüberhinaus kann geschütztes Amino $Z_6$ auch durch substituiertes Niederalkoxycarbonyl substituiertes Amino sein, wie durch 2-Halogenniederalkoxycarbonyl, z.B. 2,2,2-Trichlorethoxycarbonyl, 2-Chlorethoxycarbonyl, 2-Bromethoxycarbonyl oder 2-Iodethoxycarbonyl, oder 2-(tris-substituiertes Silyl)-ethoxycarbonyl, wie 2-Triniederalkylsilylethoxycarbonyl, z.B. 2-Trimethylsilylethoxycarbonyl oder 2-(Di-n-butyl-methylsilyl)-ethoxycarbonyl, oder 2-Triarylsilylethoxycarbonyl, wie 2-Triphenylsilylethoxycarbonyl substituiertes Amino oder verethertes Mercaptoamino oder Silylamino, oder als Enamino-, Nitro- oder Azidogruppe vorliegen. Eine veretherte Mercaptoaminogruppe ist in erster Linie eine gegebenenfalls durch Niederalkyl, wie methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino. Eine Silylaminogruppe ist in erster Linie eine organische Silylaminogruppe. In diesen enthält das Siliciumatom vorzugsweise Niederalkyl, z.B. Methyl, Ethyl, n-Butyl oder tert.-Butyl, ferner Aryl, z.B. Phenyl als Substituenten. Geeignete Silylgruppen sind in erster Linie Triniederalkylsilyl, insbesondere Trimethylsilyl oder Dimethyl-tert.-butylsilyl. Enaminogruppen enthalten an der Doppelbindung in 2-Stellung einen elektronenziehenden Substituenten, beispielsweise eine Carbonylgruppe. Schutzgruppen dieser Art sind beispielsweise 1-Acyl niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -ethylhalbesters, und Niederalk-1-en insbesondere 1-Propen bedeutet. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Geschütztes Carboxy $Z_5$ ist üblicherweise in veresterter Form geschützt, wobei die Estergruppe unter reduktiven, wie hydrogenolytischen, oder solvolytischen, wie acidolytischen oder hydrolytischen, wie sauer, basisch oder neutral hydrolytischen Bedingungen spaltbar sein kann. Eine geschützte Carboxygruppe kann ferner eine unter physiologischen Bedingungen spaltbare oder eine leicht in eine andere funktionell abgewandelte Carboxygruppe, wie in eine andere veresterte Carboxygruppe, umwandelbare, veresterte Carboxygruppe darstellen. Solche veresterten Carboxygruppen enthalten als veresternde Gruppen in erster Linie Silylgruppen, insbesondere Triniederalkylsilylgruppen, ferner in 1- oder 2-Stellung geeignet substituierte Niederalkylgruppen. Bevorzugte in veresterter Form vorliegende Carboxygruppen sind unter anderem Triniederalkylsilyloxycarbonyl, z.B. Trimethylsilyloxycarbonyl, (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls, z.B. wie vorstehend erwähnt, substituiertes Benzyloxycarbonyl, z.B. 4-Nitrobenzyloxycarbonyl, gegebenenfalls, z.B. wie vorstehend erwähnt, substituiertes Diphenylmethoxycarbonyl, z.B. Diphenylmethoxycarbonyl, oder Triphenylmethoxycarbonyl, oder gegebenenfalls, z.B. wie vorstehend erwähnt, substituiertes Picolyloxycarbonyl, z.B. 4-Picolyloxycarbonyl, oder Furfuryloxycarbonyl, wie 2-Furfuryloxycarbonyl.

Insbesondere ist geschütztes Hydroxy $Z_1$ und/oder $Z_2$ Niederalkoxy, wie Methoxy, Ethoxy oder Isopropyloxy, geschütztes Carboxy Triniederalkyl silyloxycarbonyl, wie Trimethylsilyloxycarbonyl und geschütztes, gegebenenfalls durch Niederalkyl oder Arylniederalkyl substituiertes Amino $Z_6$ Niederalkanoylamino, vor allem Formylamino, N-Niederalkanoyl-N-niederalkylamino, vor allem N-Formyl-N-niederalkyl-amino, z.B. N-Formyl-N-methyl-amino, oder Niederalkoxycarbonylamino, vorzugsweise Tertiärbutyloxycarbonylamino.

Triniederalkylhalogensilane sind beispielsweise Triniederalkyljodsilane oder insbesondere Triniederalkylbromsilane, vorzugsweise solche, in denen die Niederalkylgruppen identische $C_1$-$C_4$-Alkylreste darstellen. Bevorzugt ist Trimethylbromsilan, in zweiter Linie Trimethyljodsilan.

Die Freisetzung der geschützten Gruppen, d.h. von Hydroxy aus geschützten Hydroxygruppen $Z_1$ und/oder $Z_2$, von Carboxy aus geschützten Carboxygruppen $Z_5$ und/oder von gegebenenfalls durch Niederalkyl oder

Phenylniederalkyl substituiertem Amino aus geschützten Aminogruppen $Z_6$ durch Behandeln mit einem Triniederalkylhalogensilan erfolgt beispielsweise in einem inerten Lösungsmittel, wie einem halogenierten, vorzugsweise aliphatischen Kohlenwasserstoff, z.B. in Dichlormethan oder in zweiter Linie Tri- oder Tetrachlormethan, Trichlorethan oder Tetrachlorethan, im Temperaturbereich von etwa -25° C bis + 50°C, vorzugsweise von etwa 0° C bis etwa 30° C, z.B. bei Temperaturen im Bereich Raumtemperatur, d.h. bei etwa 15° C bis etwa 25° C, vorteilhaft unter weitgehend wasserfreien Bedingungen und unter Inertgas, z.B. unter Argon oder Stickstoff.

Die Aufarbeitung erfolgt in an sich bekannter Weise, wobei insbesondere zwei Reinigungsoperationen sich als vorteilhaft erweisen. Entweder kann das Rohprodukt in ein leichtflüchtiges Derivat übergeführt werden, z.B. durch Silylieren, und als solches destillativ gewonnen, danach desilyliert werden. Oder das Rohprodukt kann mit einem Mittel versetzt werden, das mit überschüssiger Säure, wie Halogenwasserstoffsäure, reagiert und sie so entfernt. In Frage kommen z.B. Verbindungen, an die sich eine entsprechende Säure addieren kann, z.B. Niederalkylenoxide (Epoxide), wie Propylenoxid.

Sollen als Endstoffe Verbindungen der Formel I erhalten werden, in denen $R_5$ für verestertes oder amidiertes Carboxy, wie Niederalkoxycarbonyl oder Carbamoyl steht, können die Ausgangsmaterialien der Formel II und die Verfahrensbedingungen so gewählt werden, dass im letzten Schritt zwar $Z_1$, $Z_2$ und $Z_6$ freigesetzt werden, dass hingegen $Z_5$, welches für die gewünschte Gruppe $R_5$ steht, in diesem Verfahrensschritt unverändert bleibt.

Eine besonders bevorzugte Ausführungsform ist dementsprechend auf die Herstellung von Verbindungen der Formel I gerichtet, worin $R_5$ $C_1$-$C_4$-Alkoxycarbonyl und $R_6$ Amino bedeutet. Dieser Ausführungsform zufolge geht man vorzugsweise von Verbindungen der Formel II aus, worin $Z_1$ und $Z_2$ Niederalkoxy oder in höherer als der α-Stellung durch Halogen, wie Chlor substituiertes Niederalkoxy bedeuten, $Z_5$ für $C_1$-$C_4$-Alkoxycarbonyl steht und $Z_6$ Niederalkanoylamino, wie Formylamino, oder Niederalkoxycarbonylamino, wie Tertiärbutyloxycarbonylamino, bedeutet. Ausgehend von derartigen Verbindungen der Formel II lässt sich die Freisetzung der geschützten Gruppen in einem inerten Lösungsmittel, wie einem halogenierten Kohlenwasserstoff, z.B. Dichlormethan, bei Temperaturen im Bereich von Raumtemperatur, mit einem Reagens wie Trimethylbromsilan, durch nachfolgendes Behandeln mit einem Niederalkanol, wie Ethanol, und einem Halogenwasserstoff-Abfänger, wie einem aliphatischen Epoxid, insbesondere einem Epoxyniederalkan, z.B. Propylenoxid, so steuern, dass unmittelbar Verbindungen der Formel I erhalten werden, worin $R_1$ für Hydroxy steht, $R_5$ für $C_1$-$C_4$-Alkoxycarbonyl steht, $R_6$ Amino bedeutet und die Variablen $R_2$, $R_3$ und $R_4$ die für Formel I angegebenen Bedeutungen aufweisen.

Dieses Verfahren ist insbesondere bevorzugt zur Herstellung von Verbindungen der Formel I, in denen A Methylen oder 1,3-Propylen und B eine Bindung bedeutet, $R_3$ und $R_4$ Wasserstoff bedeuten und $R_2$ für Alkyl mit bis zu 4 Kohlenstoffatomen, wie Methyl, steht.

Eine andere, ebenso bevorzugte Ausführungsform betrifft die Herstellung von Verbindungen der Formel I, worin $R_5$ Carboxy bedeutet. In diesem Falle geht man vorzugsweise von Verbindungen der Formel II aus, worin $Z_1$ und $Z_2$ Niederalkoxy oder in höherer als der α-Stellung durch Halogen, wie Chlor substituiertes Niederalkoxy bedeuten, $Z_5$ für gegebenenfalls geschütztes, z.B. durch Behandlung mit N,O-bis-Trimethylsilylacetamid silyliertes, Carboxy steht und $Z_6$ Niederalkoxycarbonylamino, insbesondere α-verzweigtes Niederakoxycarbonylamino, wie Tertiärbutyloxycarbonylamino, dar stellt. In diesem Falle wird die Carboxygruppe vorzugs-, jedoch nicht notwendigerweise intermediär geschützt, bespielsweise durch Behandeln mit einem Silylierungsmittel, wie einem N,O-Silylniederalkansäureamid, z.B. mit N,O-Trimethylsilylacetamid.

Zwischenprodukte der Formel II werden vorzugsweise hergestellt, indem man eine Verbindung der Formel

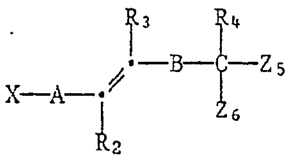

(III),

worin $R_2$, $R_3$, $R_4$, A und B wie für Formel I definiert sind, $Z_5$ die Bedeutung von $R_5$ hat oder für geschütztes Carboxy steht, $Z_6$ für eine geschützte Gruppe $R_6$ steht und X für reaktionsfähiges verestertes Hydroxy steht, mit einer Verbindung der Formel

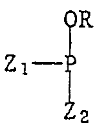

(IV),

worin $Z_1$ für gegebenenfalls geschütztes Hydroxy steht, $Z_2$ die Bedeutung von $R_1$ hat oder für geschütztes

Hydroxy steht und R eine verethernde Gruppe bedeutet, umsetzt, und können ohne Isolierung oder spezielle Reinigung eingesetzt werden.

Verbindungen der Formel III lassen sich z.B. herstellen, indem ein N-geschützter Aminomalonsäureester der Formel

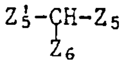 (V) ,

worin $Z_5$ und $Z_5'$ gleiche oder verschiedene veresterte Carboxygruppen, z.B. Niederalkoxycarbonylgruppen, bedeuten, mit Verbindungen der Formel

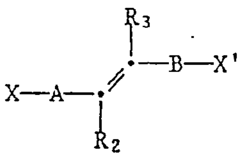 (VI)

auf an sich bekannte Weise umgesetzt werden, worin X und X′ unabhängig voneinander reaktionsfähiges verestertes Hydroxy, wie Halogen bedeuten. Die so erhaltenen Verbindungen der Formel

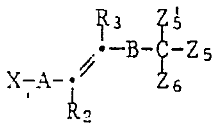 (VII)

lassen sich in Verbindungen der Formel III überführen, worin $R_4$ Wasserstoff bedeutet, indem sie, z.B. unter hydrolytischen Bedingungen, wie solche einer sauren Hydrolyse, z.B. mit Halogenwasserstoffsäuren, wie mit Salzsäure, vorzugsweise unter Erwärmen verseift und decarboxyliert oder ohne vorhergehende Verseifung durch Erhitzen in einem wässrigen aprotischen Lösungsmittel, wie Dimethylsulfoxid, in Anwesenheit eines Alkalihalogenids, wie Natriumchlorid, dealkoxycarbonyliert werden.

Diese Variante ist dementsprechend besonders geeignet zur Herstellung von Verbindungen III, worin $R_4$ Wasserstoff, $Z_5$ freies oder verestertes Carboxy und $Z_6$ geschütztes Amino, wie Niederalkanoylamino, bedeutet.

Zwischenprodukte III, worin A gegebenenfalls durch Alkyl substituiertes Methylen, B eine Bindung, X Halogen und $Z_6$ Formylamino bedeuten, lassen sich ferner herstellen, indem eine Verbindung der Formel

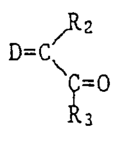 (VIII),

worin D für gegebenenfalls durch Alkyl substituiertes Methyliden steht, wie ein entsprechender α,β-ungesättigter Aldehyd, z.B. Acrolein oder Methacrolein, mit einem α-Isocyanessigsäurederivat, wie einem α-Isocyanessigsäureniederalkylester, umgesetzt wird. Unter geeigneter Katalyse, wie mit niederwertigen, d.h. mit von Metallen der Gruppen I und II des periodischen Systems der Elemente abgeleiteten, Metallsalzen, z.B. entsprechenden Metalloxiden oder Metallhalogeniden, wie Zinkchlorid, Cadmiumchlorid, Silberoxid oder, bevorzugt, Kupferoxid oder Komplexe von Gold-I-tetrafluoroborat mit aliphatischen oder cycloaliphatischen Isocyaniden, z.B. Bis(cyclohexylisocyanid)gold(I)tetrafluoroborat,, werden so auf an sich bekannte Weise 5-Vinyl-2-oxazolin-4-carbonsäurederivate, z.B. -ester der Formel

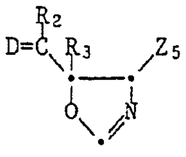

(IX)

erhalten, die sich in die offenkettigen Verbindungen der Formel

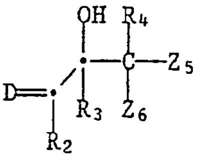

(X)

überführen lassen, worin D für gegebenenfalls durch Alkyl substituiertes Methyliden steht. Diese Verbindungen wiederum können durch selektive Halogenierung, wie Bromierung oder Chlorierung, vorzugsweise unter Kühlen, und unter Verlagerung der Doppelbindung im Sinne einer Allylumlagerung in Verbindungen der Formel III übergeführt werden.

Ein anderes Verfahren zur Herstellung von Verbindungen II, worin $R_4$ für Wasserstoff, A für Methylen oder 1,3-Propylen und $R_5$ für Carboxy steht, beruht auf dem Prinzip, dass man eine Verbindung der Formel

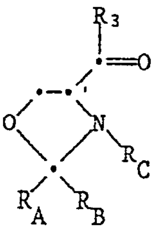

(XI),

worin $R_A$ und $R_B$ Wasserstoff oder vorzugsweise Niederalkyl, wie Methyl, bedeuten und $R_C$ für eine Aminoschutzgruppe steht, mit einem 2-$R_2$-Essigsäureester oder zunächst mit einer 1-$R_2$-Ethenmetallverbindung, z.B. mit Isopropenylmagnesiumbromid, und anschliessend mit einem Essigsäureester kondensiert, in der erhaltenen Verbindung der Formel

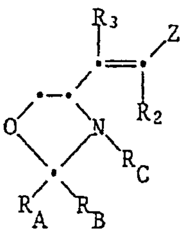

(XIIa) bzw.

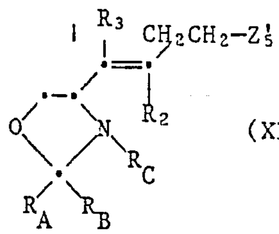

(XIIb)

worin $Z_5'$ verestertes Carboxy, z.B. Niederalkoxycarbonyl ist, diese Gruppe z.B. mittels Diisobutylaluminiumhydrid, zu Hydroxymethyl reduziert, die Hydroxymethylgruppe halogeniert, z.B. mittels Tetrabrommethan/Triphenylphosphin bromiert, die erhaltene Verbindung der Formel

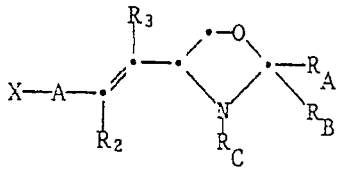 (XIII),

worin A Methylen bzw. 1,3-Propylen, X Halogen, z.B. Brom, bedeutet mit einer Verbindung der Formel IV weiterumsetzt, den Oxazolidinring, z.B. mittels eines Ionenaustauschers, wie Amberlyst 15®, aufspaltet und in der erhaltenen Verbindung der Formel

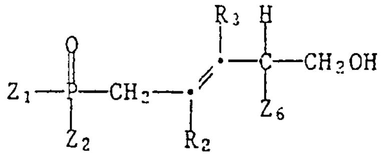 (XIV)

worin $R_6$ eine geschützte Aminogruppe der Formel $R_C$-NH- (II') bedeutet, die Hydroxymethylgruppe in üblicher Weise zur Carboxy oxidiert.

Bei der Durchführung der vorstehend beschriebenen Verfahren zur Herstellung von Zwischenprodukten III und Weiterumsetzung derselben zu Zwischenprodukten II ist es nicht erforderlich, sämtliche Zwischenstufen zu isolieren. So kann insbesondere die Ueberführung von Verbindungen X in Verbindungen III sowie deren Weiterumsetzung mit Verbindungen IV in Zwischenprodukten II vorteilhaft in situ durchgeführt werden.

Eine bevorzugte Ausführungsform des erfindungsgemässen Verfahrens besteht dementsprechend darin, dass man eine Verbindung der Formel

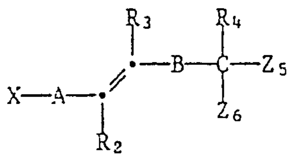 (III),

worin $R_2$, $R_3$, $R_4$, A und B wie für Formel I definiert sind, $Z_5$ die Bedeutung von $R_5$ hat oder für geschütztes Carboxy steht, $Z_6$ für eine geschützte Gruppe $R_6$, beispielsweise geschütztes Amino, steht und X für reaktionsfähiges verestertes Hydroxy steht, mit einer Verbindung der Formel

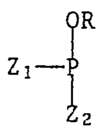 (IV),

worin $Z_1$ für gegebenenfalls geschütztes Hydroxy steht, $Z_2$ die Bedeutung von $R_1$ hat oder für geschütztes Hydroxy steht und R eine verethernde Gruppe bedeutet, umsetzt, die geschützten Gruppen $Z_6$ und gegebenenfalls $Z_1$, $Z_2$ und/oder $Z_5$ durch Behandeln mit einem Triniederalkylhalogensilan, beispielsweise mit Trimethylbromsilan, freisetzt, und gewünschtenfalls eine erhaltene Verbindung der Formel I in eine andere Verbindung der Formel I überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

Eine reaktionsfähige veresterte Hydroxygruppe, wie X, ist eine mit einer starken organischen Säure veresterte Hydroxygruppe, z.B. eine mit einer aliphatischen oder aromatischen Sulfonsäure (wie einer Niederalkansulfonsäure, insbesondere Methansulfonsäure, Trifluormethansulfonsäure, insbesondere Benzolsulfonsäure, p-Toluolsulfonsäure, p-Brombenzolsulfonsäure und p-Nitrobenzolsulfonsäure) oder mit einer starken anorganischen Säure, wie insbesondere Schwefelsäure, oder einer Halogenwasserstoffsäure, wie Chlorwasserstoffsäure oder am meisten bevorzugt Iodwasserstoffsäure oder Bromwasserstoffsäure, veresterte Hydroxygruppe.

Hinzuweisen ist in diesem Zusammenhang auf die überraschende Feststellung, dass die Herstellung der

Zwischenprodukte II und ihre erfindungsgemässe Weiterumsetzung zu den Endstoffen I stereoselektiv geführt werden kann. D.h. weder in der Reaktionsfolge III + IV → II bzw. IIa → I, noch in den Reaktionsfolgen X → III und XI → XII → XIII → XIV → II erfolgt Konfigurationsumkehr oder nennenswerte Racemisierung. Das erfindungsge mässe Verfahren ist deshalb vorzüglich geeignet zur Direktherstellung von Verbindungen der Formel I mit der bevorzugten R-Konfiguration am die Aminogruppe $R_6$ tragenden C-Atom. Die Herstellung sterisch einheitlicher Verbindungen der Formel I sowie sterisch einheitlicher Zwischenprodukte der Formeln II, III, X, XI, XII, XIII und/oder XIV ist ein weiterer Gegenstand der vorliegenden Erfindung.

Einer anderen bevorzugten Ausführungsform zufolge unterwirft man eine Verbindung der Formel

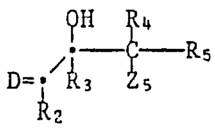

(X)

der selektiven Halogenierung, z.b. mittels Thionylchlorid, zum entsprechenen Zwischenprodukt III und setzt dieser in situ, d.h. ohne Isolierung mit der Komponente IV um.

Wie erwähnt, können erfindungsgemäss erhältliche Verbindungen in andere Verbindungen der Formel I überführt werden. Insbesondere kann eine freie Aminogruppe $R_6$ substituiert, z.B. in eine gegebenenfalls phenylierte Alkylaminogruppe überführt, freies Carboxy $R_5$ verestert bzw. verestertes oder amidiertes Carboxy $R_5$ in freies Carboxy überführt und/oder freies oder verestertes Carboxy $R_5$ in amidiertes Carboxy umgewandelt werden.

Zur Ueberführung einer Aminogruppe in eine gegebenenfalls phenylierte Alkylaminogruppe kann die Aminogruppe substitutiv, z.B. mit einem reaktionsfähigen veresterten gegebenenfalls phenylierten Alkanol, wie einem Alkylhalogenid, oder reduktiv, wie mit einem Aldehyd oder Keton, sowie katalytisch aktiviertem Wasserstoff, oder im Fall von Formaldehyd, vorteilhaft mit Ameisensäure als Reduktionsmittel, alkyliert werden.

Freie Carbonsäuren der Formel I oder deren Salze können mit den geeigneten Alkoholen oder entsprechenden Derivaten davon nach bekannten Verfahren in die entsprechenden Ester übergeführt werden, d.h. in Verbindungen der Formel I, die z.B. als Niederalkyl-, Arylniederalkyl-, Niederalkanoyloxymethyl- oder Niederalkoxycarbonylniederalkylester vorliegen.

Zur Veresterung kann eine Carbonsäure direkt mit einem Diazoalkan, insbesondere Diazomethan, oder mit einem entsprechenden Alkohol in Gegenwart eines stark sauren Katalysators (z.B. eine Halogenwasserstoffsäure, Schwefelsäure oder eine organische Sulfonsäure) und/oder eines dehydratisierenden Mittels (z.B. Dicyclohexylcarbodiimid) umgesetzt werden. Alternativ kann die Carbonsäure in ein reaktionsfähiges Derivat übergeführt werden, wie in einen reaktionsfähigen Ester, oder in ein gemischtes Anhydrid, z.B. mit einem Säurehalogenid (z.B. insbesondere ein Säurechlorid), und dieser aktivierte Zwischenstoff wird mit dem gewünschten Alkohol umgesetzt.

Verbindungen der Formel I, worin $R_5$ für verestertes Carboxy, wie insbesondere Niederalkoxycarbonyl, z.B. Ethoxycarbonyl, steht, können in Verbindungen der Formel I übergeführt werden, worin $R_5$ für Carboxy steht, z.B. durch Hydrolyse insbesondere in Gegenwart von anorganischen Säuren, wie Halogenwasserstoffsäuren oder Schwefelsäure, oder in zweiter Linie von wässrigen Alkalien, wie Alkalimetallhydroxiden, z.B. von Lithium-oder Natriumhydroxid. In diesem Zusammenhang sei darauf hingewiesen, dass auch die Freisetzung aus Carboxy aus verestertem Carboxy so geführt werden kann, dass keine nennenswerte Racemisierung erfolgt. Dies kann insbesondere dadurch erreicht werden, dass man mit etwa 0,2- bis etwa 4-normaler, z.B. etwa 1-normaler, d.h. etwa 0,5- bis etwa 2-normaler, wässriger Mineralsäure behandelt, erforderlichenfalls unter Erwärmen, z.B. auf etwa 60°C bis etwa Siedetemperatur, d.h. ungefähr 100°C. In überraschender Weise verläuft die Verseifung z.B. von Phosphonsäurecarbonsäureniederalkyestern der Formel I auch ohne Zusatz von sauren oder basischen Reagenzien mit hoher Ausbeute. Ein bevorzugtes Verfahren zur Herstellung von Carbonsäuren der Formel I aus den entsprechenden Niederalkylestern, wie den jeweiligen Ethylestern, besteht daher in der sauren Hydrolyse durch Behandeln mit etwa 0,2- bis etwa 4-normaler wässriger Mineralsäure, z.B. Salzsäure, Schwefelsäure, Phosphorsäure oder dergleichen, sowie in der - möglicherweise autokatalytischen - Verseifung in Wasser, vorzugsweise bei erhöhten Temperaturen, wie bei Erhitzen unter Rückfluss.

Die vorstehenden Reaktionen werden nach Standard-Methoden in Gegenwart oder Abwesenheit von Verdünnungsmitteln, vorzugsweise solchen, die gegenüber den Reagentien inert sind und deren Lösungsmittel darstellen, von Katalysatoren, Kondensationsmitteln bzw. den anderen Mitteln und/oder in inerter Atmosphäre, bei niedrigen Temperaturen, Raumtemperatur oder erhöhten Temperaturen, vorzugsweise beim Siedepunkt der verwendeten Lösungsmittel, bei atmosphärischem oder überatmosphärischem Druck, durchgeführt.

Die Erfindung umfasst weiterhin jegliche Variante der vorliegenden Verfahren, bei der ein bei irgendeiner Stufe derselben erhältliches Zwischenprodukt als Ausgangsmaterial verwendet wird und die verbliebenen Stufen ausgeführt werden, oder das Verfahren bei irgendeiner Stufe desselben unterbrochen wird oder bei der die Ausgangsmaterialien unter Reaktionsbedingungen gebildet werden oder bei der die Reaktionskomponenten in Form ihrer Salze oder optisch reinen Antipoden verwendet werden. Hauptsächlich diejenigen Ausgangsmaterialien sollten bei diesen Reaktionen verwendet werden, die zur Bildung der vorstehend als besonders wertvoll angegebenen Verbindungen führen.

Die Erfindung betrifft auch neue Ausgangsmaterialien und Verfahren zu deren Herstellung.

In Abhängigkeit von der Wahl der Ausgangsmaterialien und Methoden können die neuen Verbindungen in Form eines der möglichen optischen Isomeren oder von deren Gemischen vorliegen, beispielsweise in Abhängigkeit von der Anzahl der asymmetrischen Kohlenstoffatome, als reine optische Isomere, wie als Antipoden, oder als Gemische von optischen Isomeren, wie als Racemate, oder als Gemische von Diastereoisomeren, aus denen ein Antipode, wenn erwünscht, isoliert werden kann.

Erhaltene Gemische von Diastereoisomeren und Gemische der Racemate können wegen der physiko-chemischen Unterschiede der Bestandteile in bekannter Weise in die reinen Isomere, Diastereoisomere oder Racemate getrennt werden, z.B. durch Chromatographie und/oder fraktionierte Kristallisation.

Die erhaltenen Racemate (racemische Diastereoisomere) können weiterhin in die optischen Antipoden nach an sich bekannten Methoden, beispielsweise durch Umkristallisieren aus einem optisch aktiven Lösungsmittel, mit Hilfe von Mikroorganismen bzw. Enzymkatalysatoren in freier oder immobilisierter Form oder durch Umsetzung eines sauren Endprodukts mit einer optisch aktiven Base, die Salze mit der racemischen Säure bildet, und Trennung der auf diese Weise erhaltenen Salze, beispielsweise auf Basis ihrer unterschiedlichen Löslichkeiten, in die Diastereoisomere, aus denen die Antipoden durch Einwirken geeigneter Mittel freigesetzt werden können, aufgetrennt werden. Basische racemische Produkte können gleichfalls in die Antipoden, z.B. durch Trennung von deren diastereoisomeren Salzen, z.B. durch fraktionierte Kristallisation von deren d-oder $\ell$-Tartraten, getrennt werden. Irgendwelche racemischen Zwischenprodukte oder Ausgangsmaterialien können in ähnlicher Weise getrennt werden.

Schliesslich werden die erfindungsgemässen Verbindungen entweder in freier Form oder in Form von deren Salzen erhalten. Irgendeine erhaltene Base kann in ein entsprechendes Säureadditionssalz, vorzugsweise unter Verwendung einer pharmazeutisch verträglichen Säure oder eines Anionenaustausch-Präparates, übergeführt werden, oder erhaltene Salze können in die entsprechenden freien Basen, beispielsweise unter Verwendung einer stärkeren Base, wie eines Metall- oder Ammoniumhydroxids oder eines basischen Salzes, z.B. eines Alkalimetallhydroxids oder -carbonats, oder eines Kationenaustausch-Präparats, übergeführt werden. Eine Verbindung der Formel I kann auch in die entsprechenden Metall- oder Ammoniumsalze übergeführt werden. Diese oder andere Salze, beispielsweise die Pikrate, können auch für die Reinigung der erhaltenen Basen verwendet werden. Die Basen werden in die Salze übergeführt, die Salze werden getrennt, und die Basen werden aus den Salzen freigesetzt. Im Hinblick auf die enge Verwandtschaft zwischen den freien Verbindungen und den Verbindungen in Form ihrer Salze ist, wenn immer in dieser Anmeldung eine Verbindung erwähnt wird, auch ein entsprechendes Salz dieser Verbindung mit umfasst, vorausgesetzt, dass dies unter den gegebenen Umständen möglich oder sinnvoll ist.

Die Verbindungen einschliesslich ihrer Salze können auch in Form ihrer Hydrate erhalten werden oder andere für die Kristallisation verwendete Lösungsmittel enthalten.

Die erfindungsgemässen pharmazeutischen Präparate sind solche, die für die enterale, wie die orale oder rektale, und die parenterale Verabreichung an Säuger, einschliesslich des Menschen, zur Behandlung oder Verhinderung von Erkrankungen geeignet sind, die auf die Blockierung von NMDA-Rezeptoren ansprechen, wie z.B. cerebrale Ischämie, Muskelspasmen (Spastizität), Konvulsionen (Epilepsie), Angstzustände oder manische Zustände. Sie umfassen eine wirksame Menge einer pharmakologisch aktiven Verbindung der Formel I oder eines pharmazeutisch verträglichen Salzes derselben, allein oder in Kombination mit einem oder mehreren pharmazeutisch verträglichen Trägern.

Die pharmakologisch aktiven Verbindungen der Erfindung sind bei der Herstellung von pharmazeutischen Zusammensetzungen verwendbar, die eine wirksame Menge derselben allein oder in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalze und/oder Polyethylenglykol, für Tabletten auch c) Bindemitteln, z.B. Magnesiumaluminiumsilikat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvantien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 100 %, vorzugsweise etwa 1 bis 50 %, des aktiven Bestandteils. Eine Einheitsdosis für einen Säuger von etwa 50 bis 70 kg kann zwischen etwa 1 und 500 mg, bevorzugt zwischen etwa 10 und 500 mg, aktiven Bestandteil enthalten.

Die folgenden Beispiele sollen die Erfindung erläutern und keine Einschränkungen darstellen. Die Temperaturen sind in Celsiusgraden angegeben, und sämtliche Teile sind in Form von Gewichtsteilen

angegeben. Wenn nicht anders erwähnt, werden sämtliche Verdampfungen unter vermindertem Druck vorzugsweise zwischen etwa 2 und 13 Kilopascal (kPa) durchgeführt.

Beispiel 1: E-2-Amino-4-methyl-5-phosphono-3-pentensäureethylester

a) 5-(2-Propenyl)-oxazolin-4-carbonsäureethylester(1)

1,6 g rotes Kupfer(I)oxid werden in 200 ml Benzol vorgelegt. Unter intensivem Rühren wird zu dieser Suspension innerhalb von 10 Minuten eine Lösung von 140 g Isocyanessigsäureethylester und 105 g frisch destilliertem Methacrolein in 200 ml Benzol getropft. Die Reaktionstemperatur wird dabei durch Eiskühlung zwischen 30 und 32° gehalten. Nach beendigter Zugabe wird der Ansatz bis zum Abklingen der Exothermie bei 30-32° gehalten, und anschliessend 1 Stunde bei Raumtemperatur gerührt. Nach Abfiltrieren von überschüssigem Kupfer(I)oxid wird das Filtrat im Vakuum bei 30° eingedampft. Der Rückstand wird mit 600 ml Ether versetzt, über Celite filtriert und im Vakuum bis zur Trockne eingedampft. Man erhält so den 5-(2-Propenyl-2-oxazolin-4-carbonsäureethylester als farbloses Oel vom Kp. 110-130° (5,3 Pa).

b) E-2-Formylamino-3-hydroxy-4-methyl-4-pentensäureethylester(2).

139 g des 5-(2-Propenyl)-2-oxazolin-4-carbonsäureethylester werden in 70 ml Tetrahydrofuran gelöst und mit 27,4 g Wasser und 3,5 g Triethylamin versetzt. Das Reaktionsgemisch wird 62 Stunden bei 65-70° gerührt und nach dem Abkühlen in 200 ml Dichlormethan aufgenommen. Die Lösung wird über 200 g Magnesiumsulfat getrocknet, filtriert und im Vakuum eingedampft. Reinigung des verbliebenen viskosen Oels durch Säulen chromatographie (Silicagel; Hexan/Essigester 3:2) ergibt den 2-Formylamino-3-hydroxy-4-methyl-4-pentensäureethylester als Diastereomerengemisch, vom Fp. 67°.

c) E-2-Formylamino-4-methyl-5-diisopropylphosphono-3-pentensäureethylester(3).

Zu einer Lösung von 40,20 g rohem 2-Formylamino-3-hydroxy-4-methyl-4-pentensäureethylester in 600 ml 1,2-Dichlorethan werden bei 20° unter Argon 18,6 ml Thionylbromid innerhalb von 5 Minuten zugetropft (schwach kühlen). Nach zweistündigem Rühren bei Raumtemperatur werden 400 ml Wasser zugegeben, wobei die ersten 50 ml langsam zugetropft werden. Das Gemisch wird noch 15 Minuten gut ausgerührt. Die organische Phase wird abgetrennt und dreimal mit Eiswasser und einmal mit Eis/gesättigter Kaliumbicarbonatlösung (pH ca. 7,5) gewaschen. Nach Trocknen über Natriumsulfat und Abdestillieren des 1,2-Dichlorethans bei 35° im Vakuum erhält man das rohe Bromid als Zwischenprodukt, das bei Raumtemperatur mit 160 ml Triisopropylphosphit versetzt und dann bei 75° (Badtemperatur) unter Teilvakuum (ca. 13 kPa) 17 Stunden gerührt wird. Dann werden das überschüssige Triisopropylphosphit und andere flüchtige Nebenprodukte im Hochvakuum abdestilliert (Badtemperatur 90°). Durch Chromatographie des Rückstandes an der zehnfachen Gewichtsmenge Kieselgel (Korngrösse 0,04-0,06 mm) mit Essigester als Eluierungsmittel erhält man den E-2-Formylamino-4-methyl-5-diisopropylphosphono-3-pentensäureethylester als hellgelben Honig, IR ($CH_2Cl_2$): 3410 (NH); 1740 (CO-Ester); 1690 (CO-Amid); 1235 (P=O); 980-1015 (P-O-C).

d) E-2-Amino-4-methyl-5-phosphono-3-pentensäureethylester(4)

Zu einer Lösung von 25,42 g E-2-Formylamino-4-methyl-5-diisopropylphosphono-3-pentensäureethylester in 102 ml trockenem Dichlormethan werden bei 20° unter Argon 56,7 ml Trimethylbromsilan innerhalb von 15 Minuten zugetropft. Nach 20-stündigem Rühren bei Raumtemperatur werden innerhalb von 15 Minuten 102 ml Ethanol zugetropft, und das Ganze wird weitere 20 Stunden gerührt. Hierauf wird die klare Reaktionslösung im Vakuum vollständig eingedampft. Der Rückstand wird noch dreimal nach Zusatz von je 100 ml Toluol eingedampft. Der ölige Rückstand wird in 102 ml Ethanol gelöst und tropfenweise mit einer Lösung von 102 ml Propylenoxid in 102 ml Ethanol versetzt. Das kristallin anfallende Produkt wird nach 2 Stunden (Raumtemperatur) abfiltriert und mit Ethanol und Ether gewaschen. Nach dem Trocknen (80°, 4 Stunden) im Hochvakuum wird der E-2-Amino-4-methyl-5-phosphono-3-pentensäureethylester analysenrein erhalten, Fp. 212° (Zers.).

Beispiel 2: E-2-Amino-4-methyl-5-phosphono-3-pentensäureethylester

a) E-2-Formylamino-4-methyl-5-dimethylphosphono-3-pentensäureethylester (1)

100,5 g 2-Formylamino-3-hydroxy-4-methyl-4-pentensäureethylester werden in 1,5 l Dichlorethan gelöst, bei 20-25° werden 47 ml Thionylbromid zugetropft, und der Ansatz wird 1 Stunde bei Raumtemperatur gerührt. Das Reaktionsgemisch wird mit 750 ml Wasser versetzt und 10 Minuten intensiv gerührt. Die organische Phase wird abgetrennt, mit 1 Liter Eiswasser, 1 Liter 1 N Kaliumhydrogencarbonatlösung und nochmals 1 Liter Eiswasser extrahiert, über Magnesiumsulfat getrocknet und eingedampft. Der so erhaltene E-5-Brom-2-formylamino-4-methyl-3-pentensäureethylester, ein gelbes Oel, wird unmittelbar mit 50 ml Trimethylphosphit versetzt und 15 Stunden bei 70° Badtemperatur und ca. 15 kPa gerührt. Das Reaktionsgemisch wird 30 Minuten am Wasserstrahlvakuum und 1 Stunde am Hochvakuum bei 40-50° entgast. Das erhaltene Produkt wird in 600 ml Wasser aufgenommen und dreimal mit je 500 ml Essigester extrahiert. Die vereinigten organischen Phasen werden zweimal mit je 300 ml Wasser gewaschen. Alle Wasserphasen werden vereinigt, mit Natriumchlorid gesättigt und dreimal mit je 500 ml Dichlormethan extrahiert. Die vereinigten organischen Phasen werden über Magnesiumsulfat getrocknet und eingedampft. Das Produkt wird über Kieselgel (Essigester/Isopropanol 7:2) chromatographiert. Man erhält so den E-2-Formylamino-4-methyl-5-dimethylphosphono-3-pentensäureethylester als gelbes Oel, $^1$H-NMR (DMSO): 1,82 (d, 3H, 4-$CH_3$), 2,69 (d, 2H), 5,03 (m, 1H), 5,32 (m, 1H).

b) E-2-Amino-4-methyl-5-phosphono-3-pentensäureethylester(2)

16,9 g E-2-Formylamino-4-methyl-5-dimethylphosphono-3-pentensäureethylester werden in 80 ml Dichlormethan unter Stickstoff-Atmosphäre gelöst und bei ca. 25° werden 30 ml Trimethylbromsilan innerhalb von 30 Minuten zugetropft. Es wird 20 Stunden bei Raumtemperatur gerührt, und anschliessend werden bei ca. 25° 80 ml Ethanol innerhalb von 30 Minuten zugetropft, dann wird nochmals 22 Stunden bei Raumtemperatur gerührt und anschliessend eingedampft. Der Rückstand wird in 80 ml Ethanol gelöst, und unter leichter Kühlung werden 80 ml Propylenoxid in 80 ml Ethanol zugetropft. Es wird 1 Stunde bei Raumtemperatur ausgerührt, filtriert und mit Ethanol und Ether gewaschen. Man erhält so den E-2-Amino-4-methyl-5-phosphono-3-pentensäureethylester, Fp. 215 - 217° (Zers.).

Beispiel 3: E-2-Amino-4-methyl-5-phosphono-3-pentensäureethylester

a) E-2-Formylamino-4-methyl-5-di(2-chlorethyl)phosphono-3-pentensäureethylester(1)

8,2 g E-5-Brom-2-formylamino-4-methyl-3-pentensäureethylester und 19 ml Tris-(2-chlorethyl)phosphit werden 20 Stunden bei 70° Badtemperatur gerührt. Das erhaltene Gemisch wird an Kieselgel chromatographiert, wobei mit Essigester und Essigester/Isopropanol (7:1) eluiert wird, das Produkt wird aus Essigester/Diethylether kristallisiert. Man erhält so den E-2-Formylamino-4-methyl-5-di(2-chlorethyl)phosphono-3-pentensäureethylester, Fp. 47-49°.

b) E-2-Amino-4-methyl-5-phosphono-3-pentensäureethylester(2)

Unter Verwendung von 3 g E-2-Formylamino-4-methyl-5-di(chlorethyl)phosphono-3-pentensäureethylester wird auf analoge Weise wie in Beispiel 2 ebenfalls der E-2-Amino-4-methyl-5-phosphono-3-pentensäureethylester, Fp. 215° (Zers.), erhalten.

Beispiel 4: E-2-Amino-4-phosphonomethyl-3,6-heptadiensäure

a) 2-Methylen-4-pentenaldehyd(1)

Ein Gemisch aus 14,7 g Piperazin, 20,5 g Eisessig und 21,28 g Wasser wird mit 24,5 g einer 37 %igen wässrigen Formaldehydlösung versetzt und 10 Minuten bei Raumtemperatur gerührt. Unter fortgesetztem Rühren werden 25,35 g 4-Pentenaldehyd zugegeben, und das Reaktionsgemisch wird 3 Stunden bei 75° erhitzt. Nach Abkühlen auf Raumtemperatur wird die organische Phase abgetrennt, und die wässrige Phase wird dreimal mit je 50 ml Diethylether extrahiert. Die vereinigten organischen Phasen werden dreimal mit je 50 ml einer gesättigten Natriumbicarbonatlösung gewaschen, über Magnesiumsulfat getrocknet und im Vakuum eingedampft. Fraktionierte Destillation des Rückstandes liefert den 2-Methylen-4-pentenaldehyd als farbloses Oel, Kp. 65°/6,6 kPa.

b) 5-[2-(1,4-Pentadienyl)]-2-oxazolin-4-carbonsäuremethylester(2)

Durch Reaktion von Isocyanessigsäuremethylester mit 2-Methylen-4-pentenaldehyd in Toluol, auf analoge Weise wie in Beispiel 1 beschrieben und nach anschliessender säulenchromatographischer Reinigung (Silicagel, Essigester/Hexan 1:4) wird der 5-(2-(1,4-Pentadienyl))-2-oxazolin-4-carbonsäuremethylester als farbloses Oel erhalten, $^1$H-NMR ($CDCl_3$): 2,80 (d, 2H, $CH_2$); 4,45 (dd, 1H, C(4)-H); 5,82 (m, 1H, C=CH-); 7,00 (d, 1H, C(2)-H).

8,1 g 5-(2-(1,4-Pentadienyl))-2-oxazolin-4-carbonsäuremethylester werden in 20 ml Tetrahydrofuran gelöst und mit 10 ml Wasser versetzt. Das Reaktionsgemisch wird 1,5 Stunden bei 75° gerührt und nach dem Abkühlen im Vakuum eingedampft. Kristallisation des resultierenden Rückstandes aus Isopropanol/Hexan ergibt den 2-Formylamino-3-hydroxy-4-methylen-6-heptensäuremethylester als Diastereomerengemisch. Fp. 75-77°.

c) E-2-Formylamino-4-brommethyl-3,6-heptadiensäuremethylester(3)

5,0 g 2-Formylamino-3-hydroxy-4-methylen-6-heptensäuremethylester in 200 ml trockenem Tetrahydrofuran werden auf -78° abgekühlt und mit 20 ml 1,5-Hexadien versetzt. 9 ml Thionylbromid werden langsam so zugetropft, dass die Reaktionstemperatur -50° nicht übersteigt. Nach beendigter Zugabe wird die Reaktionslösung innerhalb von ca. 3 Stunden auf 0° erwärmt und 2 Stunden bei dieser Temperatur gerührt. Die Lösung wird darauf auf 300 ml einer kalten (5-10°) gesättigten Natriumbicarbonat-Lösung gegossen und mit Diethylether extrahiert. Die organischen Extrakte werden mit gesättigter Kochsalzlösung gewaschen, über Magnesiumsulfat getrocknet und im Vakuum eingedampft. Nach säulenchromatographischer Reinigung (Silicagel, Essigester/Hexan 1:1) wird der E-2-Formylamino-4-brommethyl-3,6-heptadiensäuremethylester als farbloses Oel erhalten, $^1$H-NMR ($CDCl_3$): 3,20 (d, 2H, C(5)-H); 4,00 (s, 2H, $CH_2Br$).

d) E-2-Formylamino-4-diethylphosphonomethyl-3,6-heptadiensäuremethylester(4)

3,7 g E-2-Formylamino-4-brommethyl-3,6-heptadiensäuremethylester werden in 37 ml Triethylphosphit gelöst und 8 Stunden bei 75° erhitzt. Ueberschüssiges Triethylphosphit wird darauf im Hochvakuum abdestilliert. Durch säulenchromatographische Reinigung (Silicagel, Methanol/Essigester 1:10) wird der E-2-Formylamino-4-diethylphosphonomethyl-3,6-heptadiensäuremethylester als farbloses Oel erhalten, $^1$H-NMR ($CDCl_3$): 2,54 (d, 2H, P-$CH_2$); 3,10 (m, 2H, C(5)-H); 5,10 (m, 2H, C(7)-H); 5,37 (d, 1H, C(2)-H); 5,74 (m, 1H, C(6)-H).

e) E-2-Amino-4-phosphonomethyl-3,6-heptadiensäure(5)

0,74 g E-2-Formylamino-4-diethylphosphonomethyl-3,6-heptadiensäuremethylester werden in 12 ml Dichlormethan gelöst und tropfenweise mit 0,7 ml Trimethyljodsilan versetzt. Nach vierstündigem Rühren bei Raumtemperatur wird soviel einer 1N Natriumthiosulfat Lösung zugefügt, bis sich die Reaktionslösung aufhellt. Danach wird das Reaktiongemisch mit 10 ml einer 4,5 N Salzsäurelösung versetzt und 30 Minuten bei Raumtemperatur gerührt. Die wässrige Phase wird abgetrennt, zweimal mit je 20 ml Dichlormethan gewaschen und im Vakuum eingedampft. Der Rückstand wird in 10 ml 4,5 N Salzsäure gelöst, 16 Stunden bei Raumtemperatur gerührt und anschliessend im Vakuum eingedampft. Der so erhaltene Rückstand wird in 40 ml Ethanol aufgenommen, klarfiltriert und tropfenweise mit 10 ml Propylenoxid/Ethanol (1:1) versetzt. Der entstandene weisse Niederschlag wird abfiltriert und durch Säulenchromatographie (Dowex 50 x 8/$H_2O$) gereinigt. Nach Einengen wird die E-2-Amino-4-phosphonomethyl-3,6-heptadiensäure als weisses Kristallisat erhalten, Fp. 154-157°, $^1$H-NMR ($D_2O$): 2,64 (d, 2H, P-$CH_2$); 3,15 (m, 2H, C(5)-H); 5,20 (m, 2H, C(7)-H); 5,50 (dd, 1H, C(3)-H); 5,90 (m, 1H, C(6)-H).

Beispiel 5: (2R)-2-Amino-4-methyl-5-phosphono-3-pentensäure

a) (L)-N-tert.Butoxycarbonyl-serin-N-methoxy-N-methylamid (1).

Zu einer Lösung von 1 kg (L)-N-tert.Butoxycarbonyl-serin in 1 l Tetrahydrofuran gibt man bei -20° bis -25° 541,6 ml N-Methylmorpholin innert 27 min. Nach 15 min. Rühren werden bei derselben Temperatur 699,6 ml Chlorameisensäure-isobutylester innert 42 min. und 445,8 ml N-Methoxy-N-methylamin innert 40 min. zugegeben. Man lässt auf Raumtemperatur aufwärmen und dampft das Lösungsmittel am Rotationsverdampfer ab. Der Rückstand wird in 3 l Ethylacetat gelöst, die Lösung mit 3,5 l 2n-Salzsäure und 3 l gesättigter $NaHCO_3$-Lösung extrahiert. Die Wasserphasen werden dreimal mit je 1 l Eethylacetat extrahiert, die organischen Phasen werden mit 2 l gesättigter NaCl-Lösung gewaschen, mit $MgSO_4$ getrocknet und bei 50° am Vakuum eingedampft. Der Rückstand wird 2 Std. unter Eiskühlung mit 3,5 l Hexan verrührt. Die weisse Suspension wird filtriert, das Filtergut wird mit 1 l Hexan gewaschen, Trocknen bei 40° am Vakuum 781 g 1, Smp. 116-117°. $C_{10}H_{20}N_2O_5$, berechnet: C 48,38 %, H 8,12 %, N 11,28 %; gefunden C 48,28 %, H 8,02 %, N 11,32 %.

b) (L)-3-tert.Butoxycarbonyl-2,2-dimethyl-oxazolidin-4-carbonsäure-N-methoxy-N-methylamid (2).

Eine Mischung von 781 g 1, 3,3 l Acetondimethylacetal und 42 g Pyridinium-(toluol-4-sulfonat) wird auf 72° erwärmt und anschliessend 17 Std. unter Rückfluss gekocht. Nach Zugabe von 20 g Pyridiniumtosylat wird weitere 9 Std. gekocht, wobei ca. 750 ml Lösungsmittel abdestilliert und durch 700 ml Aceton-dimethylacetal ersetzt werden. Das Lösungsmittel wird abgedampft und der Rückstand wird in 2 l Diethylether gelöst. Die organische Phase wird zweimal mit 1n-Salzsäure (1 l, 0,5 l) und je einmal mit gesättigter $NaHCO_3$- bzw. NaCl-Lösung (je 0,3 l) extrahiert. Die Wasserphasen werden zweimal mit je 0,5 l Diäthyläther extrahiert. Der Rückstand der mit $MgSO_4$ getrockneten organischen Phase wird heiss in 850 ml Hexan/Diäthyläther = 9:1 gelöst. Nach Zugabe von 600 ml Hexan und 50 ml Diäthyläther wird abgekühlt und während der Kristallisation mit 1,1 l Hexan verdünnt. Die Suspension wird filtriert. Das Filtergut wir dmit Hexan gewaschen. Trocknen bei

40° am Vakuum gibt 640 g 2, Smp. 67-68°. $C_{13}H_{24}N_2O_5$; berechnet: C 54,15 %, H 8,39 %, N 9,72 %, gefunden: C 53,96 %, H 8,37 %, N 9,91 %.

c) (4S)-2,2-Dimethyl-4-formyl-3-oxazolidin-carbonsäure-tert.butylester (3).

c.a) Reduktion mit $LiAlH_4$

Zu einer Lösung von 28,8 g 2 in 350 ml trockenem Diäthyläther werden unter Eiskühlung 2,53 g $LiAlH_4$ gegeben; Innentemperatur 5-15° C. Nach 1,5 Std. Rühren bei 5° wird eine Lösung von 5,77 g $KHSO_4$ in 60 ml Wasser so schnell zugetropft, dass die Temperatur nicht über 15° steigt (40 min.). Man filtriert die Suspension und wäscht das Filtergut mit Ether. Unter Zugabe von Eis wird das Filtrat zweimal mit je 200 ml 1n-Salzsäure, zweimal mit je 150 ml 5 %-ige $NaHCO_3$-Lösung und mit gesättigter NaCl-Lösung gewaschen. Die Wasserphasen werden einmal mit Diäthyläther extrahiert, die organischen Extrakte werden mit $Na_2SO_4$ getrocknet und eingeengt. Destillation des Rückstandes bei 0,4 mbar gibt 17,78 g 3 (Sdp. 85-90° C), $[\alpha]_D$ = -93° (c=1, $CHCl_3$); $C_{11}H_{19}NO_4$; berechnet: C 57,63 %, H 8,35 %, N 6,11 %, O 27,91 %; gefunden: C 57,59 %, H 8,54 %, N 6,17 %, O 27,74 %.

c.b) Reduktion mit Diisobutylaluminiumhydrid

Zu einer Lösung von 10 g 2 in 120 ml trockenem Diäthyläther werden unter Eiskühlung 66 ml einer 1M-Lösung von Diisobutylaluminiumhydrid in Hexan getropft. Man rührt 10 min. bei Raumtemperatur, kühlt wieder auf 0° und gibt eine Lösung von 38 g $KHSO_4$ in 160 ml Wasser zu (Temp. 0-15°). Es wird 30 min. bei Raumtemperatur gerührt und filtriert. Das Filtergut wird mit Diäthyläther und Wasser gewaschen, die organische Phase des Filtrates wird unter Eiskühlung zweimal mit je 100 ml 1n-Salzsäure, zweimal mit je 100 ml 10 % $NaHCO_3$-Lösung und zweimal mit 100 ml ges. NaCl-Lösung gewaschen. Die organischen Extrakte werden mit $Na_2SO_4$ getrocknet und eingedampft. Destillation des Rückstandes bei 0,2 mbar gibt 5,83 g 3 (Sdp. 78° C).

d), 3-((4′R)-N-tert.Butyloxycarbonyl-2′ ,2′-dimethyl-4′-oxazolidinyl)-2-methyl-propensäureäthylester(4)

Eine Lösung von 39,5 g (4S)-2,2-Dimethyl-4-formyl-oxazolidin-3-carbonsäure-tert.butylester 3 in 200 ml Dichloromethan wird innerhalb von 2 Stunden zu einer Lösung von 68,7 g 1-Ethoxycarbonylethyliden-triphenylphosphoran in 900 ml Dichlormethan getropft. Nach 6 stündigem Rühren bei Raumtemperatur wird auf 10° gekühlt und innerhalb von 15 Minuten 530 ml einer 10 %-igen wässrigen Natriumhydrogenphosphatlösung zugetropft. Nach 30 minütigem Rühren bei 15° wird die organische Phase abgetrennt und die Wasserphase mit 250 ml Dichlormethan extrahiert. Die organischen Phasen werden über Magnesiumsulfat getrocknet und eingedampft. Der Rückstand wird mit 70 ml Ether verrührt. Die Suspension wird filtriert, das Filtergut wird mit Ether gewaschen. Das Filtrat wird eingedampft, der Rückstand wird durch Chromatographie an Kieselgel aufgetrennt. Elution mit Hexan/Ethylacetat = 9:1 gibt neben 2,32 g cis-Isomer und 2,21 g Mischfraktion (cis/trans = 38:62) 45,4 g 4. $^1$H-NMR (60 MHz, $CDCl_3$, trans-Isomer): u.a. 4,7 ppm (m, H-C(4′)); 6,7 ppm (d, J=9, H-C(3)). $^1$H-NMR (60 MHz, $CDCl_3$, cis-Isomer): u.a. 5,2 ppm (m, H-C(4′)); 6,08 ppm (d, J=7, H-C(3)).

e) (4R)-2,2-Dimethyl-4-(3′-hydroxy-2′-methyl-prop-1′-enyl)-oxazolidin-3-carbonsäure-tert.butylester(5)

Zu einer auf 3° gekühlten Lösung von 48,7 g 4 in 1 l trockenem Diethylether werden innert 15 Minuten 389 ml einer 1-molaren Lösung von Diisobutylaluminiumhydrid in Hexan gegeben. Man lässt auf 11° aufwärmen und gibt unter Eiskühlung 100 ml Ethylacetat hinzu, gefolgt von 50 ml 2n-Natronlauge. Man lässt ohne Kühlung auf etwa 28° erwärmen und gibt weiter 7 ml 2n-Natronlauge zu. Man lässt 15 Stunden bei Raumtemperatur rühren, gibt Natriumsulfat hinzu und filtriert. Eindampfen des Filtrates ergibt 42,1 g rohes 5. Eine Probe (0,97 g) wird durch Chromatographie an 40 g Silicagel gereinigt. Elution mit Hexan/Ethylacetat = 3:1 gibt 0,74 g 2. $^1$H-NMR (300 MHz, DMSO-$d_6$): u.a. 3,52 (dxd, J=9 und 3) und 4,02 (dxd, J=9 und 6) (2H-C(5)); 3,78 (m, 2H-C(3′)); 4,54 (m, H-C(4)); 4,81 (t, J=6, OH); 5,33 (d, J=9, H-C(1′)).

f) (4R)-2,2-Dimethyl-4-(3′-brom-2′-methyl-prop-1′-enyl)-oxazolidin-3-carbonsäure-tert.butylester (6).

Zu einer Lösung von 41,0 g 5 und 60,2 g Tetrabrommethan in 1 Liter trockenem Diethylether werden bei 0° 47.6 g Triphenylphosphin gegeben. Nach 30 Minuten wird das Kühlbad entfernt und 17 Stunden bei Raumtemperatur gerührt. Man gibt 20 g Tetrabrommethan und 15,9 g Triphenylphosphin hinzu und rührt 2 Stunden bei Raumtemperatur. Man filtriert die weisse Suspension und wäscht das Filtergut mit Aether. Der Eindampfrückstand des Filtrates wird an 0,9 kg Kieselgel chromatographiert. Elution mit Hexan/Ethylacetat = 9:1 gibt 30,59 g 6, Smp. 62-65° C. $^1$H-NMR (300 MHz, DMSO-$d_6$): u.a. 3,55 (dxd, J=9 und 2) und 4,04 (dxd, J=9 und 6) (2H-C(5)); 4,15 (m, 2H-C(3′)); 4,49 (m, H-C(4)); 5,65 (d, J=9, H-C(1′)).

g) (4R)-2,2-Dimethyl-4-(3'-dimethylphosphono-2'-methyl-prop-1'-enyl)-oxazolidin-3-carbonsäure-tert.butylester (7).

Eine Lösung von 13,4 g 6 in 70 ml Trimethylphosphit wird 15 Stunden bei 80° gerührt. Das überschüssige Phosphit wird bei 24 mbar abgedampft. Trocknen des Rückstandes am Hochvakuum ergibt 14,3 g rohes 7. $^1$H-NMR (300 MHz, DMSO-$d_6$): u.a. 2,63 (d, J=23, 2H-C(3')); 3,59 (d, J=11, $(CH_3O)_2PO$).

h) N-((2R)-5-Dimethylphosphono-1-hydroxy-4-methyl-3-penten-2-yl)carbaminsäure-tert.butylester (8)

Zu einer Lösung von 14,0 g 7 in 250 ml Methanol werden 7 g Amberlyst®-15 ($H^+$-Form, 20-50 mesh) gegeben. Man rührt 17 Stunden bei Raumtemperatur, filtriert und dampft das Filtrat ein. Chromatographie des Rückstandes an 0,33 kg Kieselgel mit Ethylacetat/Methanol = 10:1 als Eluens gibt 10,6 g 8. $^1$H-NMR (300 MHz, DMSO-$d_6$): u.a. 4,60 (t, J=6, OH); 6,67 (d, J=7, NH).

i) (2R)-2-tert.Butoxycarbonylamino-5-dimethylphosphono-4-methyl-3-pentensäure (9).

i,a) Oxidation mit Chromschwefelsäure

Zu einer Lösung von 0,323 g 8 in 10 ml Aceton werden 0,77 ml einer Lösung, welche 3,25-molar an Chromtrioxid und 5,29-molar an Schwefelsäure ist, gegeben. Man rührt 40 Minuten bei Raumtemperatur, gibt 2 ml Isopropanol gefolgt von 50 ml Ethylacetat hinzu und versetzt die Mischung mit 0,1 g Aktivkohle. Nach 10 Minuten wird filtriert und mit 50 ml Ethylacetat gewaschen. Man extrahiert das Filtrat dreimal mit je 50 ml 10 %-iger Natriumhydrogencarbonatlösung. Die Wasserphase wird zweimal mit je 40 ml Ethylacetat extrahiert, mit 2n-Salzsäure auf pH 1 angesäuert und dreimal mit je 70 ml Ethylacetat extrahiert. Die organischen Extrakte werden mit gesättigter Kochsalzlösung gewaschen, über Magnesiumsulfat getrocknet und eingedampft. Chromatographie an 8 g Kieselgel mit Chloroform/Methanol/Essigsäure = 18:1:1 als Eluens gibt 65 mg 9. $^1$H-NMR (300 MHz, DMSO-$d_6$): 1,37 (s, $(CH_3)_3CO$); 1,82 (d, J=2, $CH_3$-C(4)); 2,63 (d, J=22, 2H-C(5)); 3,61 (d, J=11, $(CH_3O)_2PO$); 4,62 (t, J=8, H-C(2)); 5,25 (m, H-C(3)); 7,18 (d, J=8, NH); 11,7-12,5 ($CO_2H$).

i,b) Oxidation mit Sauerstoff/Platin

Zu einer Lösung von 0,66 g 8 und 0,2 g Natriumhydrogencarbonat in 20 ml Wasser und 2 ml Dioxan wird eine Suspension von Platin, hergestellt durch Hydrierung von 313 mg Platinoxid in 50 ml Wasser, gegeben. In einer zylinderischen Apparatur wird unter heftigem Rühren bei 55° Sauerstoff mittels einer Glasfritte von unten nach oben durchgeleitet. Man filtriert, wäscht mit Wasser und extrahiert das Filtrat fünfmal mit je 100-150 ml Ethylacetat. Eindampfen der Extrakte ergibt 220 mg Edukt 5. Die Wasserphase wird mit 1 g Amberlist® 15 (stark sauer) versetzt, filtriert und am Vakuum bei 40° eingedampft. Reinigung wie f,a) gibt 156 mg 9.

$^1$H-NMR (300 MHz, $CDCl_3$): 1,43 (s, $(CH_3)_3C$); 1,96 (d, J=3, $CH_3$-C(4)); 2,55 und 2,71 (2 dxd, J=22 und 15, 2H-C(5)); 3,75 und 3,76 (2 d, J=11, 2 $OCH_3$); 4,97 (m, H-C(2)); 5,25-5,45 (m, NH und H-C(3)).

j) (2R)-2-Amino-4-methyl-5-phosphono-3-pentensäure (10).

Zu einer Lösung von 123 mg 9 in 3 ml Dichlormethan werden bei 0° 0,71 ml Trimethylsilylbromid gegeben. Nach 4 Stunden Rühren bei 0° werden 20 ml Wasser zugegeben. Nach 30 Minuten wird die Dichlormethan-Phase abgetrennt, und dreimal mit je 15 ml Wasser gewaschen. Die Wasserphasen werden dreimal mit je 20 ml Dichlormethan extrahiert und am Vakuum eingedampft. Der Rückstand wird in 10 ml 5n-Salzsäure gelöst und 48 Stunden gerührt, mit 20 ml Wasser verdünnt und dreimal mit je 20 ml Dichlormethan extrahiert. Die Wasserphase wird am Vakuum eingedampft, der Rückstand am Hockvakuum getrocknet, in 3 ml Aethanol gelöst und tropfenweise mit ca. 1 ml Propylenoxid versetzt. Die Suspension wird filtriert. Waschen des Filtergutes mit Ethanol und Trocknen am Hockvakuum bei Raumtemperatur gibt 62 mg 10, Smp. 165°C (Zersetzung).

Eine Probe wird zur Analyse der Enantiomereneinheit mit (R)-(+)-Methoxy-trifluormethyl-phenylessigsäurechlorid zum Amid derivatisiert. $^1$H-NMR-Analyse (300 MHz) durch Integration der $OCH_3$-Signale ergibt $\geqq$ 95 % (2R)-Isomer (3,44 ppm) und $\leqq$ 5 % (2S)-Isomer (3,37 ppm).

Beispiel 6: (2R)-2-Amino-4-methyl-5-phosphono-3-pentensäureethylester

a) (4R)-2,2-Dimethyl-4-(3'-diisopyropylphosphono-2'-methyl-prop-1'-enyl)-oxazolidin-3-carbonsäure-tert.butylester (1).

Eine Lösung von 6,68 g Bromid gemäss Beispiel 5f) in 14,8 ml trockenem Triisopropylphosphit wird 17

Stunden bei einem Druck von 100 mbar auf 70°C erwärmt. Die Mischung wird bei 0,4 mbar/70° eingedampft. Chromatographie an 350 g Kieselgel (Eluens, Hexan/Ethylacetat = 1:1) gibt 8,28 g 1, $R_f$-Wert = 0,077.

b) N-((2R)-5-Diisopropylphosphono-1-hydroxy-4-methyl-3-penten-2-yl)carbaminsäure-tert.butylester (2).

Zu einer Lösung von 4,49 g Phosphonsäureester 1 gemäss a) in 100 ml Methanol werden 2,25 g Amberlyst® 15 ($H^+$-Form, 20-50 mesh) gegeben. Man rührt 2 Tage bei Raumtemperatur, filtriert und dampft das Filtrat ein. Chromatographie des Rückstandes an 125 g Kieselgel (Eluens Ethylacetat/Methanol = 20:1) gibt 2,44 g 2.

c) (2R)-2-tert.Butoxycarbonylamino-5-diisopropylphosphono-4-methyl-3-pentensäure (3).

Zu einer Lösung von 1,6 g Alkohol 2 gemäss b) in 60 ml Aceton werden bei 0-5° 3,3 ml einer Lösung, welche 3,25-molar an Chrom(VI)oxid und 5,29-molar an Schwefelsäure ist, gegeben. Man rührt 6 Stunden bei 0° und 12 Stunden bei Raumtemperatur. Nach Zugabe von 5 ml Isopropanol und 40 ml 20 %-iger Kochsalzlösung wird 10 Minuten gerührt, anschliessend 15 Stunden in einer Kutscher-Steudel Apparatur kontinuierlich mit Methylacetat extrahiert. Die organische Phase wird über Natriumsulfat getrocknet, eingedampft und der Rückstand an 75 g Hexan/Ethylacetat/Essigsäure = 16:10:1 chromatographiert. Dies ergibt 0,92 g 3, $[\alpha]_D$ = -94,5° (c=1,2, $CHCl_3$). $^1H$-NMR (300 MHz, $CDCl_3$): 1,2-1,3 (4d, $(2\text{-PropO})_2$); 1,4 (s, $(CH_3)_3CO$). 1,95 (d, J=3, $CH_3$-C(4)); 3,5 und 3,62 (2 dxd, J=23 und 15, 2H-C(5)); 4,66 (m, $(2\text{-PropO})_2$); 4,92 (m, H-C(2)); 5,30 (m, H-C(3); 5,42 (d, J=7, NH), 9,0-10,0 (breit, $CO_2H$).

d) (2R)-2-tert.Butoxycarbonylamino-5-diisopropylphosphono-4-methyl-3-pentensäure-ethylester (4).

Zu einer Lösung von 0,2 g Säure 3 gemäss c) in 15 ml trockenem Dichlormethan werden bei 0-5° 0,09 g 1-Amino-1-chlor-N,N,2-trimethylpropen gegeben. Nach 30 Minuten Rühren bei 0° werden 0,4 g Pyridin in 5 ml Ethanol zugegeben. Man rührt 90 Minuten bei 0° und 15 Stunden bei Raumtemperatur nach, verdünnt mit 20 ml Dichlormethan und wäscht zweimal mit je 20 ml Wasser. Die organische Phase wird mit Natriumsulfat getrocknet, eingedampft und an 25 g Kieselgel chromatographiert. Elution mit Ethylacetat/Methanol = 10:1 ergibt 0,12 g 4. $^1H$-NMR (300 MHz, $CDCl_3$): 1,2-1.4 (m, 2 $(CH_3)_2CHO$, $CH_3CH_2O$); 1,45 (s, $(CH_3)_3CO$); 1,98 (d, J=3, $CH_3$-C(4)); 2,55 (d, J=23, 2H-C(5)); 4,2 (m, $CH_3CH_2O$); 4,69 (m, 2 $(CH_3)_2CHO$); 5,0 (m, H-C(2)); 5,16 (m, H-C(3), NH).

e) (2R)-2-Amino-4-methyl-5-phosphono-3-pentensäureethylester (5).

Zu einer Lösung von 0,1 g Ester 4 gemäss d) in 10 ml Dichlormethan werden bei 0° 0.11 ml Trimethylsilylbromid gegeben. Man rührt 4 Stunden bei 0° und 15 Stunden bei Raumtemperatur. Man gibt 20 ml Wasser zu, rührt 15 Minuten, trennt die Wasserphase ab und dampft das Wasser am Hochvakuum ab. Der Rückstand wird zweimal in je 5 ml Ethanol gelöst, eingedampft und erneut in 5 ml Aethanol gelöst. Es werden 0,5 ml Propylenoxid zugegeben. Der Niederschlag wird abfiltriert, mit Ethanol gewaschen und 15 Stunden am Hochvakuum getrocknet (15 Stunden); 48 mg 5, $[\alpha]_D$ = -75° (c=0,5, $H_2O$).

Eine Probe wird zur Analyse der Enantiomerenreinheit mit (R)-(+)-Methoxy-trifluormethyl-phenylessigsäure-chlorid gefolgt von Diazomethan zum Amid-dimethylester derivatisiert. $^1H$-NMR-Analyse (300 MHz) durch Integration der $OCH_3$-Signale ergibt ≧ 97 % (2R)-Isomer (3,5 ppm) und ≦ 3 % (2S)-Isomer (3,37 ppm).

Beispiel 7: (2R)-2-Amino-4-methyl-7-phosphono-3-heptensäure

a) (4R)-2,2-Dimethyl-4-(1′-hydroxy-2′-methylprop-2′-enyl)-oxazolidin-3-carbonsäure-tert.butylester (1).

Zu einer Lösung von 6,9 g (4S)-2,2-dimethyl-4-formyl-oxazolidin-3-carbonsäure-tert.butylester (gemäss Beispiel 5c)) in 60 ml trockenem Tetrahydrofuran gibt man innerhalb von 25 Minuten bei 0-5° 45 ml einer 1,1-molaren Lösung von Isopropenylmagnesiumbromid tropfenweise hinzu. Man rührt 45 Minuten bei 0°, lässt auf Raumtemperatur aufwärmen, kühlt wieder auf 10° und gibt 90 ml Pufferlösung (1-m, Phosphat, PH-7) zu. Man filtriert und extrahiert das Filtrat zweimal mit je 100 ml Ethylacetat. Die organische Phase wird zweimal mit je 50 ml Wasser und mit gesättigter Kochsalzlösung gewaschen, mit Natriumsulfat getrock net. Abdampfen des Lösungsmittels ergibt 8 g 1, ein Diasteromerengemisch. Die Auftrennung kann durch Chromatographie an Kieselgel mit Hexan/Ethylacetat = 4:1 erfolgen und ergibt kristallines (1′S)-threo-Epimer ($R_f$-Wert:0,2) und (1′R)-erythro-Epimer ($R_f$-Wert: 0,16) im Verhältnis von ca. 1:2.

b) (4R)-4-(1′-Acetoxy-2′-methyl-prop-2′-enyl)-2,2-dimethyl-oxazolidin-3-carbonsäure-tert,butylester (2)

Zu einer Lösung von 15,6 g Epimerengemisch gemäss a) in 60 ml Pyridin werden bei 0-5° innerhalb von 10 Minuten 60 ml Essigsäureanhydrid getropft. Man rührt 15 Stunden bei Raumtemperatur, verdünnt mit 0,5 Liter Diethylether und gibt unter Eiskühlung 200 ml 2n-Salzsäure hinzu. Die organische Phase wird mit 250 ml 2n-Salzsäure und zweimal mit je 200 ml 10 %-iger Natriumcarbonatlösung gewaschen. Trocknen über

Natriumsulfat und Abdampfen des Lösungsmittels ergibt 15,4 g 2.

c) (4R)-4-(4'-Carboxy-2'-methyl-butenyl)-2,2-dimethyl-oxazolidin-3-carbonsäure-tert.butylester (3).

Eine Lösung von 5,25 g Diisopropylamin in 200 ml trockenem Tetrahydrofuran wird bei 0° mit 34,5 ml einer 1,6-molaren Lösung von Butyllithium in Hexan versetzt. Man kühlt auf -75°C und tropft innerhalb von 10 Minuten eine Lösung von 15 g Acetat 2 gemäss b) in 100 ml Tetrahydrofuran ein und gibt nach 5 Minuten eine Lösung von 8 g tert.-Butyldimethylsilylchlorid in 30 ml 1,3-Dimethyl-3,4,5,6-tetrahydro-2-(1H)-pyrimidinon zu. Man lässt auf Raumtemperatur erwärmen, erhitzt 2 Stunden zum Rückfluss, kühlt auf Raumtemperatur ab, gibt 230 ml 45 %-ige Ammoniumfluoridlösung hinzu, rührt 20 Stunden bei Raumtemperatur und dampft ein. Der ölige Eindampfrückstand der organischen Phase wird unter Eiskühlung mit 150 ml n-Natronlauge versetzt und die Mischung zweimal mit je 200 ml Dichlormethan extrahiert. Die Wasserphase wird mit 300 ml 20 %-iger Zitronensäure-Lösung angesäuert und dreimal mit je 300 ml Dichlormethan extrahiert. Die organischen Phasen werden mit 20 %-iger Kochsalzlösung gewaschen, über Natriumsulfat getrocknet und eingedampft. Chromatographie des Rückstandes an 50 g Kieselgel mit Hexan/Ethylacetat = 1:1 als Eluens gibt 11 g festes 3.

d) (4R)-4-(4'-Carbaethoxy-2'-methyl-butenyl)-2,2-dimethyl-oxazolidin-3-carbonsäure-tert.-butylester (4).

d,a) Ausgehend von der Säure 3

Zu einer eisgekühlten Lösung von 7,8 g Carbonsäure 3 gemäss c) in 100 ml trockenem Dichlormethan tropft man innert 10 Minuten 3,9 ml 1-Amino-1- chlor-N,N,2-trimethylpropen hinzu. Nach 30 Minuten bei 0° wird innert 20 Minuten eine Lösung von 2,2 g Pyridin in 80 ml Ethanol zugegeben. Nach 12 Stunden Rühren bei Raumtemperatur wird mit 100 ml Dichlormethan verdünnt und zweimal mit je 100 ml Wasser gewaschen. Die organische Phase wird über Natriumsulfat getrocknet und eingedampft. Chromatographie des Rückstandes an Kieselgel mit Hexan/Ethylacetat = 10:1 ergibt 6,5 g Ester 4, $[\alpha]_D = +6,17°$ (c=1, $CHCl_3$). $C_{18}H_{31}NO_5$, berechnet: C 63,32 %, H 9,15 %, N 4,10 %; gefunden: C 63,4 %, H 9,2 %, N 4,5 %.

d,b) Ausgehend vom Epimerengemisch 1

Eine Lösung von 8 g Alkohol 1 gemäss a) und 0,05 ml Propionsäure in 10,5 ml Orthoessigsäure-triethylester wird unter langsamer Abdestillation von Ethanol 14 Stunden auf 135 - 140° erwärmt. Man dampft am Hochvakuum bei 40° ein. Chromatographie des Rückstandes an Kieselgel mit Hexan/Ethylacetat = 10:1 ergibt 8 g 4.

e) (4R)-2,2-Dimethyl-4-(5'-hydroxy-2'-methyl-pent-1'-enyl)-oxazolidin-3-carbonsäure-tert.butylester (5).

Zu einer Lösung von 14,5 g 4 gemäss d) in 250 ml absolutem Diethylether werden bei 0° portionsweise 1,61 g Lithiumaluminiumhydrid hinzugegeben. Man rührt 18 Stunden bei 0-2° und gibt unter Kühlen mit Aceton/Trockeneis eine Lösung von 5 g Kaliumhydrogensulfat in 60 ml Wasser hinzu. Man filtriert und wäscht viermal mit je 200 ml Diethylether. Die organische Phase wird dreimal mit je 80 ml n-Salzsäure, dreimal mit je 80 ml gesättigter Natriumbicarbonatlösung, zweimal mit je 200 ml gesättigter Kochsalzlösung gewaschen, über Natriumsulfat getrocknet und eingedampft. Chromatographie an Kieselgel mit Hexan/Ethylacetat = 1,1 gibt 11,3 g 5, $C_{16}H_{29}NO_4$, berechnet, C 64,19 %, H 9,77 %, N 4,68 %; gefunden: C 63,6 %, H 9,8 %, N 4,7 %.

f) (4R)-4-(5'-Brom-2'-methyl-pent-1'-enyl)-2,2-dimethyl-oxazolidin-3-carbonsäure-tert.butylester (6).

Bei 0-2° werden zu einer Lösung von 4,4 g Alkohol 5 gemäss e) in 200 ml Dichlormethan 4,87 g Tetrabrommethan, 3.85 g Triphenylphosphin und 0,6 ml Pyridin gegeben. Nach 12 Stunden bei 0-2° werden weitere je 1 g Tetrabrommethan und Triphenylphosphin zugegeben und 6 Stunden bei 0° gerührt. Man dampft ein, nimmt in Ethylacetat auf, filtriert und engt ein. Chromatographie an Kieselgel mit Hexan/Ethylacetat = 10:1 gibt 4,5 g 6, $C_{16}H_{28}NO_3Br$; berechnet: C 53,04 %, H 7,79 %, N 3,87 %, Br 22,06 %; gefunden C 53,1 %, H 7,7 %, N 3,9 %, Br 21,6 %.

g) (4R)-2,2-Dimethyl-4-(2'-methyl-5'-diisopropylphosphono-pent-1'-enyl)-oxazolidin-3-carbonsäure-tert.butylester (7).

Eine Lösung von 10,9 g Bromid 6 in 22 ml Triisopropylphosphit wird bei 100 mbar 24 Stunden auf 135 - 140° erwärmt. Das überschüssige Reagens wird bei 0,1 mbar/60° abgedampft. Chromatographie des Rückstandes an Kieselgel mit Hexan/Ethylacetat = 1:1 gibt 10,7 g 7.

h) N-((2R)-1-Hydroxy-4-methyl-7-diisopropylphosphono-hept-3-en-2-yl)-carbaminsäure-tert.butylester (8).

Zu einer Lösung von 3 g 7 gemäss g) in 100 ml Aethanol werden 3 g Amberlyst® 15 ($H^+$-Form) gegeben. Man rührt 20 Stunden bei Raumtemperatur, filtriert, dampft ein und chromatographiert an 50 g Kieselgel. Elution mit Ethylacetat/Methanol = 10:1 gibt 2,3 g 8, $[\alpha]_D$ = -5,9° (c=1), $CHCl_3$.

$C_{19}H_{38}NO_6P$; berechnet: C 56,0 %, H 9,4 %, N 3,44 %, P 7,6 %; gefunden C 55,4%, H 9,3 %, N 3,4 %, P 7,3 %.

i) (2R)-2-tert.Butoxycarbonylamino-4-methyl-7-diisopropylphosphono-3-heptensäure (9).

i,a) Oxidation mit Chromsäure

Bei 0 - 5° tropft man 0,84 ml einer Lösung, welche 3,25-molar an Chromtrioxid und 5,29-molar an Schwefelsäure ist, zu einer Lösung von 0,5 g Alkohol 8 gemäss h) in 15 ml Aceton. Man rührt 30 Minuten bei 0° und 35 Minuten bei Raumtemperatur, gibt 4 ml Isopropanol, 80 ml Ethylacetat und 30 ml 20 %-ige Kochsalzlösung hinzu und filtriert. Die Wasserphase wird dreimal mit je 20 ml Ethylacetat extrahiert. Die organische Phase wird über Natriumsulfat getrocknet und eingedampft. Chromatographie an 20 g Kieselgel mit Hexan/Ethylacetat/Essigsäure = 16:10:1 gibt 0,34 g 9, $[\alpha]_D$ = -35,25° (c=1,39, $CHCl_3$), $^{13}C$-NMR (75 MHz, $CDCl_3$): 173,8 ($CO_2H$); 155,0 (OCON); 140,4 (C(4)); 121,1 (C(3)); 79,5 ($OC(CH_3)_3$); 70,3 (OCH); 52,1 (C(2)); 39,7 (d, J=18, C(5)); 28,3 ($(CH_3)_3C$); 25,7 (d, J=142, C(7)); 24,0 ($(CH_3)_2CH$); 20,2 (d, J=5, C(6)).

i,b) Oxidation mit Platin-Sauerstoff

Zu einer Lösung von 3 g Alkohol 8 gemäss h) in 105 ml Dioxan wird bei 55° eine Suspension von Platin in 45 ml Wasser, hergestellt durch Hydrierung und Entgasung von 1 g Platinoxid in 45 ml Wasser, gegeben. Bei 55 - 60° wird unter heftigem Rühren (ca. 1900 rpm) Sauerstoff durchgeleitet. Man filtriert durch Celite®, wäscht zweimal mit je 80 ml Wasser und dampft das Filtrat bei 40° am Hochvakuum ein. Man löst in 200 ml Wasser, gibt 1 g Natriumbicarbonat und 50 ml 20 %-ige Kochsalzlösung zu und extrahiert dreimal mit je 100 ml Ethylacetat. Dir organischen Phasen werden über Natriumsulfat getrocknet. Filtration und Eindampfen ergibt 1,8 g Edukt 8. Die Wasserphase wird mit ca. 20 ml n-Schwefelsäure angesäuert und fünfmal mit je 120 ml Ethylacetat extrahiert. Trocknen über Natriumsulfat, Eindampfen und Chromatographie des Rückstandes gemäss i,a) gibt 0,8 g Säure 9.

j) (2R)-2-Amino-4-methyl-7-phosphono-3-heptensäure (10).

Eine Lösung von 3,3 g Säure 9 gemäss i) und 2,6 g N,O-bis-Trimethylsilylacetamid wird 1 Stunden unter Argon bei Raumtemperatur gerührt. Nach Zugabe von 4,4 g Trimethylbromsilan wird 24 Stunden gerührt. Man tropft das Reaktionsgemisch bei 0° zu 400 ml Wasser und rührt 30 Minuten. Die organische Phase wird abgetrennt und dreimal mit je 50 ml Wasser gewaschen. Die Wasserphasen werden dreimal mit je 30 ml Dichlormethan extrahiert und bei 40° am Hockvakuum auf 10 ml eingeengt. Chromatographie an 20 ml Dowex® 50 Wx8 mit Wasser als Eluens und Lyophilsierung des Eluates gibt 0,4 g 10 als amorphes weisses Pulver vom Smp. 252° (Zersetzung); $[\alpha]_D$ = -86,5° (c=1, $H_2O$); $C_8H_{16}NO_5P \cdot 1\ H_2O$; berechnet C 37,05 %, H 6,9 %, N 5,5 %, gefunden: C 36,3 %, H 6,5 %, N 5,6 %.

Eine Probe wird zur Analyse der Enantiomerenreinheit mit (R)-(+)-Methoxy-trifluormethyl-phenylessigsäurechlorid zum Amid derivatisiert. $^1H$-NMR-Analyse (300 MHz) durch Integration der $OCH_3$-Signale ergibt ≧ 94 % (2R)-Isomer (3,24 ppm) und ≦ 6 % (2S)-Isomer (3,17 ppm).

Beispiel 8: (2R)-2-Amino-7-phosphono-3-heptensäure

a) (4R)-2,2-Dimethyl-4-(1′-hydroxy-prop-2′-enyl)-oxazolidin-3-carbonsäure-tert.butylester (1).

Zu einer Lösung von 25 g (4S)-2,2-Dimethyl-4-formyl-oxazolidin-3-carbonsäure-tert.-butylester (gemäss Beispiel 5c) in 300 ml trockenem Tetrahydrofuran gibt man innerhalb von 30 Minuten bei 0 - 5° 60 ml einer 2,4 M Lösung von Vinylmagnesiumbromid in Tetrahydrofuran. Man rührt 1 Stunde bei 0°, lässt auf Raumtemperatur erwärmen und rührt 1 Stunde bei Raumtemperatur nach. Unter Kühlen auf 10° werden 300 ml Pufferlösung (1-molar, Phosphat, pH-7) zugegeben. Nach 10 Minuten wird filtriert, zweimal mit je 150 ml Ethylacetat extrahiert und zweimal mit je 100 ml Wasser gewaschen. Die Wasserphase wird zweimal mit je 100 ml Ethylacetat extrahiert. Die organischen Extrakte werden über Natriumsulfat getrocknet und eingedampft. Chromatographie des Rückstandes an Kieselgel mit Hexan/Ethylacetat = 4:1 gibt 24,2 g Epimerengemisch 1; $C_{13}H_{23}NO_4$; berechnet: C 60,68%, H 9,01 %, N 5.44 %; gefunden: C 60,7 %, H 9,1 %, N 5,6 %.

b) (4R)-4-(4'-Ethoxycarbonylbutenyl)-2,2-dimethyl-oxazolidin-3-carbonsäure-tert.butylester (2).

Eine Lösung von 22,5 g Alkohol 1 gemäss a) und 0,3 ml Propionsäure in 38,5 ml Orthoameisensäure-triäthylester wird unter langsamer Abdestillation von Ethanol 4 Stunden auf 135 - 140 C erwärmt. Man dampft am Hochvakuum bei 50° ein und chromatographiert an 300 g Kieselgel. Elution mit Hexan/Ethylacetat = 4:1 gibt 23,9 g 2, $[\alpha]_D$ = -10,0° (c=1,5, $CHCl_3$); $C_{17}H_{29}NO_5$; berechnet: C 62,36 %, H 8,93 %, N 4,28 %; gefunden: C 62,2 %, H 8,9 %, N 4,4 %.

c) (4R)-2,2-Dimethyl-4-(5'-hydroxy-pentenyl)-oxazolidin-3-carbonsaure-tert.butylester (3).

Bei 0 - 2° gibt man zu einer Lösung von 23,5 g 2 gemäss b) in 550 ml absolutem Diäthyläther portionenweise 2,7 g Lithiumaluminiumhydrid. Nach 3 Stunden Rühren bei 0 - 2° wird unter Kühlen eine Lösung von 25 g Kaliumhydrogensulfat in 250 ml Wasser zugetropft. Man filtriert durch Celite® und wäscht gut mit Diethylether. Die organische Phase wird zweimal mit je 200 ml n-Salzsäure und zweimal mit je 250 ml 10 %-iger Natriumbicarbonatlösung gewaschen. Die Wasserphasen werden zweimal mit je 100 ml Ether extrahiert. Die mit 20 %-iger Kochsalzlösung gewaschenen organischen Phasen werden über Natriumsulfat getrocknet und eingedampft. Chromatographie an Kieselgel mit Hexan/Ethylacetat als Eluens gibt 18,6 g 3, $[\alpha]_D$ = -10,1° (c=1,4, $CHCl_3$); $C_{15}H_{27}NO_4$; berechnet: C 63,13 %, H 9,54 %, N 4,91 %; gefunden: C 63,0 %, H 9,5 %, N 5,0 %.

d) (4R)-4-(5'-Brom-pentenyl)-2,2-dimethyl-oxazolidin-3-carbonsäure-tert.butylester (4).

Zu einer Lösung von 18,5 g Alkohol 3 gemäss c), 28,2 g Tetrabrommethan und 22,3 g Triphenylphosphin in 600 ml Dichlormethan werden bei 0° 7 ml Pyridin getropft. Man rührt 12 Stunden bei 0 - 2°, dampft ein, nimmt in 150 ml Ethylacetat auf, filtriert, engt auf 50 ml ein und chromatographiert an 200 g Kieselgel. Elution mit Hexan/Ethylacetat 10:1 gibt 19,9 g Bromid 4, $[\alpha]_D$ = -18,9° (c = 1, $CHCl_3$); $C_{15}H_{26}NO_3Br$; berechnet: C 51,73 %, H 7,53 %, N 4,02 %, Br 22,94 %; gefunden: C 51,7 %, H 7,7 %, N 4,2 %, Br. 23,0 %.

e) (4R)-2,2-Dimethyl-4-(5'-di-2-propylphosphono-pentenyl)-oxazolidin-3-carbonsäure-tert.butylester (5).

Eine Lösung von 19,9 g Bromid 4 gemäss d) in 60 ml Triisopropylphosphit wird unter 100 mbar 20 Stunden auf 130 - 135° erwärmt. Das überschüssige Reagens wird bei 60°/0,1 mbar abdestilliert, der Rückstand an 250 g Kieselgel chromatographiert. Elution mit Hexan/Ethylacetat = 1:1 gibt 20,6 g Phosphonsäureester 5, $[\alpha]_D$ = -8,6° (c=0,8, $CHCl_3$); $C_{21}H_{40}NO_6P$; berechnet: C 58,18 %, H 9,30 %, N 3,23 %, P 7,15 %; gefunden: C 57,4 %, H 9,3 %, N 3,2 %, P 7,5 %.

f) N-((2R)-1-Hydroxy-7-(diisopropylphosphono-hept-3-en-2-yl)-carbaminsäure-tert.butylester (6).

Eine Lösung von 20,6 g 5 gemäss e) in 800 ml Methanol wird 20 Stunden mit 30 g Amberlyst® 15 ($H^+$-Form) bei Raumtemperatur gerührt. Man filtriert, wäscht mit Methanol, dampft ein und chromatographiert an 100 g Kieselgel. Elution mit Ethylacetat gibt 14,8 g 6, $[\alpha]_D$ = -3,6° (c=1,5, $CHCl_3$); $C_{18}H_{36}NO_6P$; berechnet: C 54,95 %, H 9,22 %, N 3,56 %, P 7,87 %;
gefunden: C 53,6 %, H 9,0 %, N 3,4 %, P 9,0 %.

g) (2R)-2-tert.Butoxycarbonylamino-7-diisopropylphosphono-3-heptensäure (7)

Zu einer Lösung von 7,4 g Alkohol 6 gemäss f) in 300 ml Aceton werden bei 0 - 2° C innerhalb von 20 Minuten 15 ml einer Lösung, welche 3,25-molar an Chromtrioxid und 5,29-molar an Schwefelsäure ist, zugetropft. Man rührt 2 Stunden bei 0 - 2°, 4 Stunden bei Raumtemperatur und gibt dann 30 ml Isopropanol, 300 ml Ethylacetat und 200 ml 20 %-ige Kochsalzlösung zu. Die Wasserphase wird dreimal mit je 250 ml Ethylacetat extrahiert, die organischen Phasen werden über Natriumsulfat getrocknet und eingedampft. Chromatographie an 200 g Kieselgel mit Hexan/Ethylacetat/Essigsäure = 16:10:1 gibt 4,55 g Säure 7, $[\alpha]_D$ = -24,9° (c = 0,8, $CHCl_3$).

h) (2R)-2-Amino-7-phosphono-3-heptensäure (8).

Eine Lösung von 3,1 g Säure 7 und 3 ml N,O-Bis-trimethylsilylacetamid in 200 ml trockenem Dichlormethan wird 1 Stunde bei Raumtemperatur gerührt. Man gibt 3,5 ml Trimethylbromsilan zu und rührt 30 Stunden bei Raumtemperatur. Die flüchtigen Anteile werden abgedampft, der Rückstand wird in 50 ml Dichlormethan aufgenommen und bei 0 - 2° mit 250 ml Wasser versetzt. Die Wasserphase wird abgetrennt und am Hochvakuum auf 10 ml eingeengt. Chromatographie an 20 ml Dowex® 50 Wx8 mit Wasser als Eluens und Lyophilisierung des Eluates gibt 1,04 g 8 als amorphes Pulver, $[\alpha]_D$ = -65,2° (c = 1, $H_2O$); $^{13}C$-NMR (75 MHz, $D_2O$: 172,8 ($CO_2H$); 140,3 (C(4)); 122,2 (C(3)); 56,4 (C(2)); 33,3 (d, J = 17, C(5)), 27,3 (d, J=134, C(7)); 22,6 (d, J=4, C(6)). Eine Probe wird zur Analyse der Enantiomerenreinheit mit (R)-(+)-Methoxytrifluormethyl-phenylessigsäurechlorid gefolgt von Diazomethan zum Amid-Trimethylester derivatisiert. $^1H$-NMR-Analyse (300 MHz) durch Integration der $OCH_3$-Signale ergibt ≧ 95 % (2R)-Isomer (3,54 ppm) und ≦ 5 % (2S)-Isomer

(3,37 ppm).

Beispiel 9: (2R)-2-Amino-4-fluor-5-phosphono-3-pentensäure (7).

a) (4R)-4-2'-Carbomethoxy-2'-fluor-vinyl)-2,2-dimethyl-oxazolidin-3-carbonsäure-tert.butylester (1).

Zu einer Suspension von 13 g Zinkpulver und 2 g Kupfer(I)chlorid in einer Lösung von 8,2 g (4S)-2,2-Dimethyl-4-formyl-oxazolidin-3-carbonsäure-tert.butylester (gemäss Beispiel 5c) in 200 ml trockenem Tetrahydrofuran und 4,1 g Essigsäureanhydrid werden unter Rühren und Erwärmen 8,1 g Dichlorfluoressigsäure-methylester zugegeben (Argon-Atmosphäre). Man kocht 2 Std. unter Rückfluss, lässt abkühlen, verdünnt mit Diethylether, filtriert durch Celite® und dampft das Filtrat ein. Chromatographie des Rückstandes an Kieselgel mit Hexan/Ethylacetat = 19,1 als Eluens gibt 5,07 g 1, Massenspektrum (field-desorption): 303 ($M^+$); $^1$H-NMR (60 MHz, $CDCl_3$): u.a. 5,93 (dxd, J=31 und 8,5, H-C(1')).

b) (4R)-2,2-Dimethyl-4-(2'-fluoro-3'-hydroxy-propenyl)-oxazolidin-3-carbonsäure-tert-butylester (2).

Zu einer Lösung von 4,9 g Ester 1 gemäss a) in 160 ml absolutem Diethylether werden bei 0-5° 39 ml einer 1M-Lösung von Diisobutylaluminiumhydrid in Hexan getropft. Man rührt 1 Std. bei 0-5°, gibt 80 ml Ethylacetat gefolgt von 25 ml 2N-Natronlauge zu, wobei die Temperatur unter 25° gehalten wird. Man lässt 30 min. bei Raumtemperatur rühren, gibt 50 g Natriumsulfat zu, filtriert und wäscht mit Ethylacetat. Trocknen des Filtrates mit Natriumsulfat, Eindampfen und Chromatographie an 100 g Kieselgel mit Hexan/Ethylacetat = 3:1 gibt 3,7 g 2, $[\alpha]_D$ = +2,5 (c=2,5, $CHCl_3$); $C_{13}C_{22}NO_4F$: berechnet: C56,72 %, H 8,06 %, N 5,09 %; gefunden: C 56,2 %, H 8, 2 %, N 5,1 %.

c) (4R)-4-(3'-Brom-2'-fluor-propenyl)-2,2-dimethyl-oxazolidin-3-carbonsäure-tert.butylester (3).

Zu einer Lösung von 3,7 g Alkohol 2 gemäss b) in 200 ml trockenem Dichlormethan werden bei 0-2° innert 10 min. 6,63 g Tetrabrommethan und 5,24 g Triphenylphosphin gegeben. Nach 15 Std. Rühren bei 0-2° wird eingedampft, in 80 ml Ethylacetat aufgenommen, filtriert, das Filtrat eingedampft. Chromatographie des Rückstandes an 50 g Kieselgel mit Hexan/Ethylacetat = 4:1 gibt 3,7 g Bromid 3, $[\alpha°]_D$ = +12,7° (c=1, $CHCl_3$); $C_{13}H_{21}NBrFO_3$; berechnet ; C 46,17 %, H 6,26 %, N 4,14 %; gefunden: C 46,4 %, H 6,3 %, N 4,2 %.

d) (4R)-2,2-Dimethyl-4-(2'-fluoro-3'-di-2-propylphosphono-propenyl)-oxazolidin-3-carbonsäure-tert.butylester (4).

Ein Lösung von 3,7 g Bromid 3 gemäss c) in 30 ml Tri-2-propylphosphit wird bei 100 mbar während 7 Std. uf 130-135° erwärmt.. Das überschüssige Reagens wird bei 90°/0,1 mbar abgedampft. Chromatographie des Rückstandes an 100 g Kieselgel mit Hexan/Ethylacetat = 3:1 gibt 2,8 g Phosphonat 4; $[\alpha]_D$ = -8,6 (c=3, $CHCl_3$); $C_{19}H_{35}NFO_6P$; berechnet: C 53.89 %, H 8,33 %, N 3,31 %; gefunden: C 53, 7 %, H 8,4 %, N 3,4 %.

e) N-((2R)-4-Fluor-1-hydroxy-5-di-2-propyl-phosphono-pent-3-en-2-yl)-carbaminsäure-tert.butylester (5).

Eine Lösung von 2,3 g Phosphonat 4 gemäss d) in 250 ml Methanol wird mit 5 g Amberlyst-15 ($H^+$-Form) 20 Std. bei Raumtemperatur gerührt. Man filtriert, dampft ein und chromatographiert an 80 g Kieselgel. Elution mit Ethylacetat gibt 1,31 g Alkohol 5, $[\alpha]_D$ = -21,1° (c=1, $CHCl_3$); $C_{13}H_{31}NFO_5P$; berechnet: C 52,31 %, H 8,51 %, N 3,81 %; gefunden: C 50,2 %, H 8,2 %, N 3,7 %.

f) (2R)-2-tert.Butoxycarbonylamino-4-fluor-5-di-2-propylphosphono-3-pentensäure (6).

Zu einer Lösung von 1,3 g Alkohol 5 gemäss e) in 80 ml Aceton werden bei 0-2° 2,5 ml einer Lösung, welche 3,25 M an Chromtrioxid und 5,29 M an Schwefelsäure ist, gegeben. Man rührt 2 Std. bei 0-2° und lässt innert 4 Std. auf Raumtemperatur aufwärmen. Nach Zugabe von 10 ml 2-Propanol, 100 ml Ethylacetat und 50 ml 20 % NaCl-Lösung wird die Wasserphase abgetrennt und viermal mit je 100 ml Ethylacetat extrahiert. Trocknen der organischen Phasen mit Natriumsulfat, Eindampfen, und Chromatographie an 70 g Kieselgel mit Hexan/Ethylacetat/Essigsäure = 10:16:1 als Eluens gibt 0,87 g Säure 6, $[\alpha]_D$ = -72,9° (c=0,8, $CHCl_3$).

g) (2R)-2-Amino-4-fluor-5-phosphono-3-pentensäure (7).

Eine Lösung von 0,8 g 6 gemäss f) und 0,84 ml N,O-Bis-trimethylsilylacetamid in 80 ml trockenem Dichlormethan wird 1 Std. bei Raumtemperatur gerührt. Nach Zugabe von 0.98 ml Trimethylbromsilan wird 30 Std. bei Raumtemperatur gerührt. Die flüchtigen Anteile werden abgedampft, der Rückstand wird in 50 ml Dichlormethan gelöst und bei 0-5° mit 200 ml Wasser versetzt. Die organische Phase wird abgetrennt und zweimal mit je 50 ml Wasser gewaschen. Die Wasserphasen werden am Hochvakuum auf 10 ml eingeengt.

Chromatographie an 30 ml Dowex 50 Wx8 mit Wasser als Eluens und Lyophilisierung des Eluates gibt 0,22 g 7, $[\alpha]_D$ = -44,8 (c=0,5, $H_2O$); Massenspektrum (fast-atom bombardment): 214, $(M+H)^+$. $^{13}C$-NMR (75 MHz, $D_2O$): 172,7 ($CO_2H$); 161,5 (dxd, J=263 und 12, C(4)); 100,9 (t, J ca. 11, C(3)); 49,5 (C(2)); 33,5 (dxd, J=128 und 26, C(5)).
Eine Probe wird zur Analyse der Enantiomerenreinheit mit (R)-(+)-Methoxy-trifluormethylphenylessigsäure-chlorid zum Amid derivatisiert. $^1H$-NMR-Analyse (300 MHz) durch Integration der $OCH_3$-Sinale ergibt ≧ 90 % (2R)-Isomer (3,49 ppm) und ≦ 10 % (2S)-Isomer (3,38 ppm).

Beispiel 10: (2R)-2-Amino-4-methyl-5-phosphono-3-pentensäureethylester

a) (2R, 3S)-2-Formylamino-3-hydroxy-4-methyl-4-pentensäureethylester (1).

aa) Ausgehend von 1,1,3,3-Tetramethyl-1,3-disila-2-azolidin-N-essigsäureethylester.

Zu einer Lösung von 2,26 ml N-Cyclohexyl-N-isopropylamin in 60 ml absolutem Tetrahydrofuran werden bei -20° 7,6 ml einer 1,6-molaren Lösung von Butyllithium in Hexan gegeben. Nach 20 Minuten wird auf -78° gekühlt und eine Lösung von 3 g 1,1,3,3-Tetramethyl-1,3-disila-2-azolidin-N-essigsäureethylester in 60 ml Tetrahydrofuran zugetropft. Man rührt 1 Stunde bie -78° und gibt anschliessend 142 ml einer ca. 0,035-molaren Lösung von Cyclopentadienyl-bis-0-1,2:5,6-diisopropyliden-D-glucofuranosyl-titan(IV)chlorid in Ether hinzu und rührt 17 Stunden bei -78°. Die Reaktionslösung wird mittels einer Stahlkanüle durch Argon-Druck in ein Gefäss mit einer auf -78° gekühlten Lösung von 1,1 ml Methacrolein in 15 ml Tetrahydrofuran überführt. Man lässt langsam auf Raumtemperatur aufwärmen, rührt 2 Stunden, gibt 1,5 ml Wasser hinzu und filtriert. Das Filtrat wird mit 250 ml Diethylether verdünnt, dreimal mit je 250 ml ca. 10 %-iger Kochsalzlösung und mit 250 ml gesättigter Kochsalzlösung gewaschen. Die Wasserphasen werden zweimal mit je 250 ml Diethylether extrahiert. Man trocknet die organischen Phasen über Natriumsulfat, dampft ein und löst heiss in 150 ml Cyclohexan. Beim Abkühlen kristallisiert 1,2:5,6-Di-O-isopropyliden-D-glucofuranose. Die Mutterlauge wird eingedampft, der Rückstand in 120 ml Tetrahydrofuran, 24 ml Wasser und 4,5 ml Essigsäure aufgenommen und 2 Stunden bei Raumtemperatur gerührt. Eindampfen am Hochvakuum bei Raumtemperatur gibt 10,6 g Rückstand, enthaltend (2R, 3S)-2-Amino-3-hydroxy-4-methyl-4-pentensäureethylester. Eine Probe (5 mg) wird 2 Stunden mit 0,2 ml Trifluoressigsäureanhydrid in 0,3 ml Dichlormethan derivatisiert. Kapillargaschromatographie (Chirasil®-L-Val, 90 - 180°/2° pro Minuten) ergibt 99,25 % (2R,3S)-Enantiomer (Retentionszeit $T_{ret}$ = 10,28 Minuten) und 0,75 % (2S,3R)-Enantiomer (Retentionszeit $T_{ret}$ = 11,48 Minuten). Man erhitzt anschliessend 5 1/2 Stunden in 80 ml Ameisensäureethylester zum Rückfluss. Eindampfen und Chromatographie an Kieselgel mit Hexan/Ethylacetat = 1,1 ergibt 1,64 g Formamid 1. Analyse einer Probe (5 mg) als Acetat durch Gaschromatographie (Chirasil®-L-Val, 160 - 180°, 1° pro Minuten): 99,2 % (2R,3S)-Enantiomer ($T_{ret}$ = 13,64 Minuten). 0,8 % (2S,3R)-Enantiomer ($T_{ret}$ = 13,94 Minuten).

a,b) Ausgehend von Isocyanessigsäureethylester

Zu einer Lösung von 24,88 g Isocyanessigsäureethylester und 18,51 g Methacrolein in 220 ml 1,2-Dichlormethan werden bei 60° 1,65 g (S)-N-Methyl-N[2-(dimethylamino)-äthyl]-1-[(R)-1',2-bis-(diphenylphosphino)-ferrocenyl]-äthylamin und 1,105 g Bis(Cyclohexylisocyanid)gold(I)tetrafluoroborat gegeben. Man rührt 5 Stunden bei 50° unter Argon, dampft ein, nimmt in 400 ml Diethylether auf, filtriert und dampft das Filtrat ein. Destillation des Eindampfrückstandes am Hochvakuum (0,04 mbar) ergibt 33,62 g eines Stereoisomerengemisches von 5-(2-Propenyl)-oxazolin-4-carbonsäureäthylester vom Sdp. 42 -52°; MS.: m/e = 183 (2 %, $M^+$), 110 (80 %), 85 (100 %). Eine Lösung von 33 g dieses Gemisches in 74 ml Wasser und 33 ml Tetrahydrofuran wird 3 Stunden zum Rückfluss erhitzt. Eindampfen am Vakuum ergibt 36.1 g eines Gemisches stereoisomerer 2-Formylamino-3-hydroxy-4-methyl-4-pentensäure-ethylester 2. Eine Probe (35 mg) wird in 2 ml Dichlormethan mit 0,05 ml N,O-Bis-trimethylsilylacetamid derivatisiert und mittels Kapillar-Gaschromatographie (Chirasil®-L-Val, 150°) analysiert: 89,1 % (2R,3S)-Isomer ($T_{ret}$ = 22,1 Minuten), 5,8 % (2S,3R)-Isomer ($T_{ret}$ = 23,1 Minuten, 2,8 % (2R,3R)-Isomer ($T_{ret}$ = 24,3 Minuten, 2,3 % (2S,3S)-Isomer ($T_{ret}$ = 25,4 Minuten).

Zu einer Lösung von 20,2 g 2, 16,83 ml Triethylamin und 0,62 g 4-(Dimethylamino)pyridin in 300 ml Dichlormethan wird innerhalb von 25 Minuten bei 0 - 3° eine Lösung von 11,42 ml Essigsäureanhydrid in 50 ml Dichlormethan getropft. Nach 30 Minuten wäscht man zweimal mit eiskalter 2n-Salzsäure und zweimal mit 10 %-iger Kochsalzlösung. Die Wasserphasen werden mit 100 ml Dichlormethan extrahiert. Der Eindampfrückstand der über Natriumsulfat getrockneten organischen Phasen wird heiss in Hexan/Ethylacetat = 4:1 gelöst. Beim langsamen Abkühlen auf ca. 30° kristallisieren 1,82 g racemischer (2R*,2S*)-2-Formylamino-3-acetoxy-4-methyl-4-pentensäureethylester vom Smp. 98-106°. Die Mutterlauge wird langsam auf -12°C gekühlt und 1 Stunde bei dieser Temperatur gehalten. Filtration ergibt 15,04 g Acetat 3, Smp. 73-75°, $[\alpha]_D$ = -75,6° (c=1, $CHCl_3$), gaschromatographische Analyse (Chirasil®-L-Val, 160 -180°, 1° pro Minuten): (2R,3S)-Isomer 93,5 % ($T_{ret}$ = 14,5 Minuten), (2S,3R)-Isomer 2,2 % ($T_{ret}$ = 14,8 Minuten), (2R,3R)-Isomer 2,2 % ($T_{ret}$ = 16,8 Minuten), (2S,3S)-Isomer 2,1 % ($T_{ret}$ = 17,1 Minuten.

Zu einer Lösung von (2R,3S)-2-Formylamino-3-acetoxy-4-methyl-4-pentensäureethylester (3) in 400 ml

absolutem Ethanol werden bei -16° 20,64 g wasserfreies Kaliumcarbonat gegeben. Nach 4 Stunden Rühren bei -18° bis -11°C werden 500 ml Pufferlösung (1-molar, Phosphat, pH=7) zugetropft. Man rührt 30 Minuten bei Raumtemperatur und extrahiert dreimal mit je 350 ml Dichlormethan. Chromatographie des Eindampfrückstandes der über Natriumsulfat getrockneten organischen Phasen an 1 kg Kieselgel mit Hexan/Ethylacetat = 1:2 als Eluens gibt 9,8 g Alkohol 1.

b) (2R)-2-Formylamino-4-methyl-5-diisopropylphosphono-3-pentensäureäthylester (4).

Zu einer Lösung von 14,19 g 1 gemäss a) in 210 ml 1,2-Dichlorethan werden bei 18 - 20° 6,57 ml Thionylbromid und nach 2 Stunden Rühren 135 ml Wasser gegeben. Nach 15 Minuten wird die organische Phase abgetrennt und dreimal mit je 150 ml Eiswasser und einmal mit 100 ml eisgekühlter gesättigter Natriumhydrogencarbonat-Lösung gewaschen. Der Eindampfrückstand der mit Natriumsulfat getrockneten organischen Phase wird in 60 ml Triisopropylphosphit gelöst 17 Stunden bei 75°/100 mbar gerührt. Das überschüssige Reagens wird am Hochvakuum bei 90° abdestilliert. Chromatographie des Rückstandes an 650 g Kieselgel mit Ethylacetat/Methanol = 20:1 gibt 10,88 g Phosphonsäureester 4, $[\alpha]_D$ = -123,5° (c=1, $CHCl_3$). $^1H$-NMR-Analyse (300 MHz) unter Zugabe von (1R)-1-(9'-Anthracenyl)-2,2,2-trifluorethanol zeigt eine Enantiomerenreinheit von ≧ 90 %.

c) (2R)-2-Amino-4-methyl-5-phosphono-3-pentensäureethylester (5).

Zu einer Lösung von 10,3 g 4 gemäss b) in 42 ml Dichlormethan werden bei Raumtemperatur innert 15 Minuten 23 ml Trimethylbromsilan getropft. Nach 21 1/2 Stunden werden unter Eiskühlung 42 ml Ethanol zugegeben und 20 stunden weitergerührt. Man dampft ein, löst den Rückstand dreimal in je 70 ml Toluol und dampft jeweils wieder ein. Der Rückstand wird in 42 ml Ethanol gelöst und mit 42 ml Propylenoxid versetzt. Nach 1 1/2 Stunden wird filtriert. Trocknen des Filtergutes im Vakuumexsiccator über $P_2O_5$/KOH (3 Stunden/80°) gibt 6,17 g 5; $[\alpha]_D$ = -78° (c = 0,6, $H_2O$); Smp. 194-197° (Zers.), $C_8H_{16}NO_5P$; berechnet: C 40,51 %, H 6,80 %, N 5,91 %, P 13,05 %; gefunden: C 39,4 %, H 7,09 %, N 5,73 %, P 12,98 %.

Eine Probe wird zur Analyse der Enantiomerenreinheit mit (R)-(+)-Methoxytrifluormethyl-phenylessigsäure-chlorid und Diazomethan zum Amid-dimethylphosphonat derivatisiert und durch $^1H$-NMR (300 MHz) anhand der Integration der $OCH_3$-Signale analysiert: (2R)-Isomer ≧ 93 % (3,51 ppm), (2S)-Isomer ≦ 7 % (3,37 ppm).

Beispiel 11: (2R)-2-Amino-4-methyl-5-phosphono-3-pentensäure.

Eine Lösung von 100 mg (2R)-2-Amino-4-methyl-5-phosphono-3-pentensäureethylester gemäss Beispiel 10 in 3 ml n-Salzsäure wird 4 1/2 Stunden in einem Bad von 100°C erwärmt. Man dampft ein und trocknet den Rückstand 30 Minuten am Hockvakuum bei 60°. Der Rückstand wird in 15 ml Ethanol gelöst und mit 4 ml Propylenoxid versetzt. Filtration und Trocknen des Filtergutes im Vakuumexsiccator über $P_2O_5$/KOH gibt 62 mg Säure.

Eine Probe wird zur Ermittlung der Enantiomerenreinheit mit (R)-(+)-Methoxy-trifluormethyl-phenylessigsäurechlorid zum Amid derivatisiert und durch $^1H$-NMR (300 MHz), Integration der $OCH_3$-Signal, analysiert: ≧ 95 % (2R)-Enantiomer (3,25 ppm) und ≦ 5 % (2S)-Enantiomer (3,18 ppm).

Beispiel 12: E-2-Amino-4-methyl-5-phosphono-3-pentensäure (1)

12 g E-2-Amino-4-methyl-5-phosphono-3-pentensäureethylester werden in 70 ml Wasser 19 Stunden unter Rückfluss gerührt. das Reaktionsgemisch wird langsam auf Raumtemperatur gekühlt, 1 Stunde im Eisbad gerührt, filtriert und mit kaltem Wasser gewaschen. Man erhält so die E-2-Amino-4-methyl-5-phosphono-3-pentensäure als Monohydrat, Fp. 163° (Zers.).

Beispiel 13 : E-2-Dimethylamino-4-methyl-5-phosphono-3-pentensäure

Ein Gemisch von 3,56 g E-2-Amino-4-methyl-5-phosphono-3-pentensäureethylester, 45 ml 98 %-iger Ameisensäure und 30 ml 37 %-iger wässriger Formaldehydlösung wird 30 Minuten bei einer Badtemperatur von 105° gerührt. Hierauf wird im Vakuum zur Trockne eingedampft. Der Rückstand wird in wenig Wasser aufgenommen und das Ganze wiederum im Vakuum eingedampft. Diese Vorgehensweise wird noch zweimal wiederholt. Der feste Rückstand wird mit 80 ml Wasser verrührt. Nach einer Stunde wird das Ungelöste über ein Hartfilter abgetrennt und mit Wasser nachgewaschen. Das Filtrat und das Waschwasser werden im Vakuum zur Trockne eingedampft. Der Rückstand wird in 100 ml Wasser suspendiert und nach Zusatz von 30 ml 1 N Natronlauge 2 Tage bei Raumtemperatur stehengelassen. Das Reaktionsgemisch wird bis auf ein Restvolumen von ca. 25 ml im Vakuum eingedampft und durch Ionenaustauscher-Chromatographie (Dowex 50 W x 8 $H_2O$) gereinigt. Die Fraktionen, welche das gewünschte Produkt enthalten, werden vereinigt, im Vakuum eingedampft und aus Wasser/Ethanol umkristallisiert. Man erhält so die E-2-Dimethylamino-4-methyl-5-phosphono-3-pentensäure, Fp. 239° (Zers.).

Beispiel 14: E-2-Dimethylamino-4-methyl-5-phosphono-3-pentensäureethylester

1,19 g E-2-Dimethylamino-4-methyl-5-phosphono-3-pentensäure werden mit 30 ml 8-n ethanolischer Chlorwasserstofflösung versetzt und 24 Stunden bei 40° gerührt. Das Reaktionsgemisch wird im Vakuum zur Trockne eingedampft. Nochmaliges Eindampfen nach Zusatz von 30 ml reinem Ethanol ergibt 1,90 g rötliches Oel, das in heissem Isopropanol gelöst wird. Nach Abkühlen wird das kristalline E-2-Dimethylamino-4-methyl-5-phosphono-3-pentensäureethylester-hydrochlorid erhalten, Fp. 203° (Zers.).

Beispiel 15: E-2-Benzylamino-4-methyl-5-phosphono-3-pentensäureethylester

Eine Lösung von 5,69 g E-2-Amino-4-methyl-5-phosphono-3-pentensäureethylester in 48 ml Wasser und 48 ml Ethanol wird mit 16 ml Eisessig, 5,90 g Natriumacetat (wasserfrei) und 12, 2 ml Benzaldehyd versetzt. Unter intensivem Kühlen mit Eis/Kochsalz werden innerhalb einer Stunde in ca. 70 Portionen 15,70 g Natriumborhydrid zugegeben, wobei nach der Hälfte der Zugabe, nach ca. 30 Minuten, 4 ml Benzaldehyd zugesetzt werden. Die Temperatur des Reaktonsgemisches wird zwischen 0 und 10° gehalten. Nach beendeter Zugabe wird 1 Stunde bei 0° ausgerührt und dann 1 N Salzsäure bis zur kongosauren Reaktion zugetropft. Die ungelösten Salze werden abfiltriert und mit Wasser nachgewaschen. das Filtrat wird im Vakuum zur Trockne eingedampft, der Rückstand wird noch zweimal nach Zusatz von Ethanol eingedampft. Nun wird der Rückstand mit 150 ml Ethanol verrührt, das Ungelöste abgenutscht und mit Ethanol nachgewaschen. Das Filtrat wird mit 25 ml Propylenoxid versetzt und 2 Stunden gerührt. Das auskristallisierte Material (hauptsächlich Edukt) wird abfiltriert. Die Mutterlauge wird zur Trockne eingedampft und mit 350 ml Ethylacetat verrührt. Das nach Abnutschen erhaltene kristalline Rohprodukt wird aus Ethanol umkristallisiert. Man erhält so E-2-Benzylamino-4-methyl-5-phosphono-3-pentensäureethylester, Fp. 192° (Zers.).

Beispiel 16: E-2-Benzylamino-4-methyl-5-phosphono-3-pentensäureethylester

Eine Lösung von 1,00 g E-2-Benzylamino-4-methyl-5-phosphono-3-pentensäureethylester in 6 ml Wasser wird 20 Stunden unter Rückfluss gerührt. Das Reaktionsgemisch wird im Vakuum zur Trockne eingedampft. Der Rückstand wird mit Ethanol versetzt und wiederum eingedampft. Dieser Vorgang wird noch zweimal wiederholt. Der Rückstand wird in siedendem Methanol gelöst. Nach Abkühlen wird die kristalline E-2-Benzylamino-4-methyl-5-phosphono-3-pentensäure erhalten, Fp. 150° (Zers.).

Beispiel 17: E-2-Isopropylamino-4-methyl-5-phosphono-3-pentensäureethylester

Eine Lösung von 6,40 g E-2-Amino-4-methyl-5-phosphono-3-pentensäureethylester in 54 ml Wasser und 18 ml Eisessig wird mit 6,64 g Natriumacetat (wasserfrei) und 18 ml Aceton versetzt. Unter intensivem Kühlen mit Eis/Kochsalz werden innerhalb von 90 Minuten in ca. 70 Portionen 17,67 g Natriumborhydrid zugegeben, wobei nach 20 Minuten und 50 Minuten je 18 ml Aceton zugesetzt werden. Nach beendeter Zugabe wird die dicke, weisse Suspension noch 30 Minuten bei 0° ausgerührt und dann 1 N Salzsäure bis zur kongosauren Reaktion zugetropft. Die entstandene klare Lösung wird im Vakuum eingedampft, der Rückstand wird noch zweimal nach Zusatz von Ethanol eingedampft. Der Rückstand wird mit 200 ml Ethanol bei Raumtemperatur verrührt, das Ungelöste abfiltriert und mit Ethanol nachgewaschen. Das Filtrat wird im Vakuum eingedampft, und der Rückstand wird aus Isopropanol umkristallisiert. Man erhält so das E-2-Isopropylamino4-methyl-5-phosphono-3-pentensäureethylester-hydrochlorid, Fp. 203-205° (Zers.).

Beispiel 18: E-2-Isopropylamino-4-methyl-5-phosphono-3-pentensäure

Eine Lösung von 1,20 g E-2-Isopropylamino-4-methyl-5-phosphono-3-pentensäureethylester-hydrochlorid in 7 ml Wasser wird 20 Stunden unter Rückfluss gerührt. Das Reaktionsgemisch wird im Vakuum zur Trockne eingedampft. Der Rückstand wird mit Ethanol versetzt und wiederum eingedampft. Dieser Vorgang wird.noch zweimal wiederholt. Der Rückstand wird in 25 ml Ethanol gelöst, unter Rühren tropfenweise mit total 5 ml Propylenoxid versetzt und das Ganze zur Trockne eingedampft. Der Rückstand wird in wenig Wasser gelöst und mit Ethanol bis zur Trübung versetzt. Beim vierstündigen Ausrühren bei Raumtemperatur erfolgt langsame Kristallisation. Das Produkt wird abfiltriert, mit Ethanol und Diethylether gewaschen und bei 100° im Hochvakuum getrocknet. Man erhält so die E-2-Isopropylamino-4-methyl-5-phosphono-3-pentensäure, Fp. 225-227° (Zers.).

Beispiel 19: E-2-Methylamino-4-methyl-5-phosphono-3-pentensäure

a) 2-(N-Methyl-N-formyl-amino)-3-hydroxy-4-methyl-4-pentensäureethylester(1)

20,60 g 5-(2-Propenyl)-2-oxazolin-4-carbonsäureethylester, hergestellt gemäss Beispiel 1, werden in 200 ml trockenem Dichlormethan unter Argon gelöst. Bei 15° wird nun eine Suspension von 16,60 g Trimethyloxoniumtetrafluorborat in 200 ml trockenem Dichlormethan zugetropft. Das Reaktionsgemisch wird

17 Stunden bei Raumtemperatur gerührt und danach langsam mit 150 ml Wasser und 45 ml gesättigter Kaliumbicarbonatlösung versetzt, so dass ein pH von 7 resultiert. Die abgetrennte organische Phase wird zweimal mit Wasser und einmal mit gesättigter Kochsalzlösung gewaschen und über Natriumsulfat getrocknet. Nach Abdestillieren des Dichlormethans wird der ölige Rückstand im Kugelrohr destilliert, Kp. 120-130°/13 Pa. Man erhält so den 2-(N-Methyl-formylamino)-3-hydroxy-4-methyl-4-pentensäureethylester als hellgelben Honig, IR ($CH_2Cl_2$): 3550 (HO); 1740 (CO-Ester); 1675 (CO-Amid).

b) E-2-(N-Methyl-N-formyl-amino)-4-methyl-5-diisopropylphosphono-3-penten säureethylester(2)

Zu einer Lösung von 17,80 g 2-(N-Methylformylamino)-3-hydroxy-4-methyl-4-pentensäureethylester in 248 ml 1,2-Dichlorethan werden bei 20° unter Argon 7,70 ml Thionylbromid zugetropft. Das Gemisch wird 2 Stunden bei Raumtemperatur gerührt. Hierauf werden unter schwacher Kühlung (20°) 150 ml Wasser zugetropft. Das zweiphasige Gemisch wird noch 20 Minuten gut ausgerührt. Die organische Phase wird abgetrennt und dreimal mit Wasser/Eis, einmal mit Eis/gesättigter Kaliumbicarbonatlösung und einmal mit gesättigter Kochsalzlösung gewaschen. Das nach Trocknen über Natriumsulfat und Abdestillieren des 1,2-Dichlorethans bei 35° im Vakuum erhaltene Zwischenprodukt wird bei Raumtemperatur mit 66 ml Triisopropylphosphit versetzt und dann bei 75° unter vermindertem Druck (ca. 13 kPa) 17 Stunden gerührt. Dann werden das überschüssige Triisopropylphosphit und andere flüchtige Nebenprodukte im Hochvakuum abdestilliert. Durch säulenchromatographische Reinigung (Kieselgel, Essigester) erhält man den E-2-(N-Methyl-formylamino)-4-methyl-5-diisopropylphosphono-3-pentensäureethylester als gelblichen Honig; IR ($CH_2Cl_2$): 1740 (CO-Ester); 1670 (CO-Amid); 1235 (P=O), 980-1010 (P-O-C). Die Verbindung liegt gemäss $^1$H-NMR-Spektrum als Gemisch zweier Rotamere vor.

c) E-2-Methylamino-4-methyl-5-phosphono-3-pentensäure(3)

Zu einer Lösung von 9,50 g E-2-(N-Methyl-formylamino)-4-methyl-5-diisopropylphosphono-3-pentensäureethylester in 40 ml trockenem Dichlormethan werden bei 20° unter Argon 20 ml Trimethylbromsilan innerhalb von 10 Minuten zugetropft. Nach 20-stündigem Rühren bei Raumtemperatur werden innerhalb von 15 Minuten 37 ml Ethanol zugetropft, und das Ganze wird wiederum 20 Stunden gerührt. Hierauf wird die klare Reaktionslösung im Vakuum zur Trockne eingedampft. Der Rückstand wird noch zweimal nach Zusatz von je 30 ml Toluol eingedampft. Das resultierende Oel wird mit 128 ml 2 N Salzsäure versetzt und 16 Stun den bei einer Badtemperatur von 85° gerührt. Das Reaktionsgemisch wird im Vakuum eingedampft. Zweimaliges Eindampfen nach Zusatz von Ethanol/Toluol 1:1 ergibt einen öligen Rückstand, der in 51 ml Ethanol gelöst und tropfenweise mit einer Lösung von 51 ml Propylenoxid in 51 ml Ethanol versetzt wird. Das kristallin anfallende Produkt wird nach 2 Stunden abfiltriert und aus Wasser/Ethanol umkristallisiert. Man erhält so die E-2-Methylamino-4-methyl-5-phosphono-3-pentensäure, Fp. 239° (Zers.).

Beispiel 20:

In analoger Weise wie in den Beispielen 1 - 3 oder 6 und 9 beschrieben kann man ferner herstellen:
E-2-Amino-5-phosphino-3-pentensäureethylester, Fp. 172-173°.
E-2-Amino-5-phosphino-3-pentensäurebutylester, Fp. 160-161°.
E-2-Amino-5-phosphono-3-pentensäureethylester, Fp. 167-168°.
E-2-Amino-5-phosphono-3-pentensäurebutylester, Fp. 160-161°.
E-2-Amino-5-phosphono-3-pentensäureoctylester, Fp. 161-162°.
E-2-Amino-5-phosphono-3-pentensäurepropylester, Fp. 161-162°.
E-2-Amino-5-phosphono-3-pentensäurepentylester, Fp. 160-161°.
E-2-Amino-5-phosphono-3-pentensäureisobutylester, Fp. 163-164°.
E-2-Amino-5-phosphono-3-pentensäure-sekundär-butylester, Fp. 169-170°.
E-2-Amino-4-methyl-5-phosphono-3-pentensäuremethylester, Fp. 193-194°, [Wasser.Aceton (9:1)];
E-2-Amino-4-methyl-5-phosphono-3-pentensäurepropylester, Fp. 184-185°, (Wasser);
E-2-Amino-4-methyl-5-phosphono-3-pentensäure-n-butylester, Fp. 186-187°, [Wasser/Aceton (2:1)];
E-2-Amino-4-methyl-5-phosphono-3-pentensäure-isobutylester, Fp. 181-182°, [Wasser/Aceton (9:1)];
E-2-Amino-4-methyl-5-phosphono-3-pentensäurepentylester, Fp. 207-208°;
E-2-Amino-4-methyl-5-phosphono-3-pentensäurehexylester, Fp. 207-208°.
E-2-Amino-7-phosphono-4-heptensäurebutylester, Fp. 186°.
E-2-Amino-5-phosphono-4-pentensäuremethylester, Fp. 219-220°.
E-2-Amino-5-phosphono-4-pentensäureethylester, Fp. 234°.
E-2-Amino-5-phosphono-4-pentensäurebutylester, Fp. 239°.
E-2-Amino-5-phosphono-4-pentensäureoctylester, Fp. 236°.
E-2-Amino-5-phosphono-4-pentensäure-2-hydroxyethylester, Fp. 197°.

Beispiel 21:

In analoger Weise wie in den Beispielen 4, 12, 13, 18 und 19 oder 5, 7, 8, 9 und 11 kann man ferner herstellen:

E-2-Amino-5-phosphono-4-pentensäure, Fp. 219-220°;
E-2-Amino-5-methylphosphonyl-4-pentensäure, Fp. 222°;
E-2-Amino-5-butylphosphonyl-4-pentensäure, Fp. 232-233°;
E-2-Amino-5-octylphosphonyl-4-pentensäure, Fp. 221-223°;
E-2-Amino-5-dodecylphosphonyl-4-pentensäure, Fp. 211°;
E-2-Amino-6-phosphono-4-hexensäure, Fp. 244-246°;
E-2-Amino-6-methylphosphonyl-4-hexensäure, Fp. 145-150°;
E-2-Amino-6-butylphosphonyl-4-hexensäure, Fp. 216°;
E-2-Amino-6-octylphosphonyl-4-hexensäure, Fp. 209-210°;
E-2-Amino-6-dodecylphosphonyl-4-hexensäure, Fp. 197-200°;
E-2-Amino-7-phosphono-4-heptensäure, Fp. 125° (Zersetzung)
E-2-Amino-5-phosphono-3-pentensäure, weisses amorphes Pulver, $^1$H-NMR ($D_2O$): 2,39 (dd, 2H, C(5)-H); 4,27(d, 1H, C(2)-H); 5,53 (m, 1H, C(3)-H); 5,87 (m, 1H, C(4)-H), Fp. nach Umkristallisieren aus Ethanol/Wasser 191-192°;
E-2-Amino-4-methyl-5-phosphono-3-pentensäure, $^1$H-NMR ($D_2O$); 1,73 (s, 3H, $CH_3$); 4,55 (s, 1H, C(2)-H);
E-2-Amino-4-methylphosphonyl-3-pentensäure, amorphes weisses Pulver, $^1$H-NMR($D_2O$): 2,55 (dd, 2H, C(5)-H); 4,38 (d, 1H, C(2)-H); 5,64 (m, 1H, C(3)-H); 5,91 (m, 1H, C(4)-H);
E-2-Amino-5-phosphino-3-pentensäure, Fp. 139-140°;
E-2-Amino-4-methyl-5-phosphino-3-pentensäure, Fp. 176-177°; (2S)-E-2-Amino-4-methyl-5-phosphono-3-pentensäure, Fp 196°, $[\alpha]_D^{20} = +97,1 \pm 1,9°$ (c = 0,5; Wasser);
E-2-Amino-4-methyl-5-methylphosphonyl-3-pentensäure, $^1$H-NMR($D_2O$): 1,20 (d, 3H, $Ch_3$-P); 1,75 (d, 3H, $CH_3$); 2,45 (d, 2H, C(5)-H); 4,50 (d, 1H, C(2)-H); 5,15 (m, 1H, C(3)-H);
E-2-Amino-2-methyl-5-phosphono-3-pentensäure, Fp. 225-226° (aus Wasser);
E-2-Amino-4-methyl-5-phosphono-3-pentensäure, weisses Pulver, Fp. 168°, $^1$H-NMR($D_2O$): 1,50 (d, 3H, $CH_3$); 2,4 (m, 2H, $CH_2$); 4,30 (s, 1H, C(2)-H); 5,60 (m, 1H, C(4)-H;
E-2-Amino-4-methyl-5-phosphono-3-pentensäure, weisser amorpher Festkörper. $^1$H-NMR ($D_2O$): 1,05 (dd, 3H, $CH_3$); 2,45 (m, 1H, C(5)-H); 4,33 (d, 2H, C(2)-H); 5,5 und 5,9 (2m, 2H, C(3)-H und C(4)-H);
E-2-Amino-4-ethyl-5-phosphono-3-pentensäure, Fp. 176°;
E-2-Amino-4-propyl-5-phosphono-3-pentensäure, Fp. 193°;
E-2-Amino-4-butyl-5-phosphono-3-pentensäure, Fp. 186-187°;
E-2-Amino-4-isopropyl-5-phosphono-3-pentensäure, Fp. 201°;
E-2-Amino-4-tert.butyl-5-phosphono-3-pentensäure; Fp. 252-253°; $^1$H-NMR ($D_2O$): 0,95 (s, 9H, $(CH_3)_3C$);
Z-2-Amino-4-tert.butyl-5-phosphono-3-pentensäure; $^1$H-NMR ($D_2O$): 1,08 (s, 9H, $(CH_3)_3C$); 2,45 (m, 2H, $CH_2$); 4,95 (d, 1H, C(2)-H); 5.20 (m, 1H, C(3)-H).
E-2-Amino-4-benzyl-phosphono-3-pentensäure, farblose Nadeln, Fp. 196-198°;
E-2-Amino-4-phenyl-5-phosphono-3-pentensäure, farblose Nadeln, Fp. 230-233°;
E-2-Amino-4-methyl-5-methylphosphono-3-pentensäure als Nebenprodukt, Fp. 149-150°.

Beispiel 22: E-2-Methylamino-4-methyl-3-pentensäureäthylester

1,20 g E-2-Methylamino-4-methyl-5-phosphono-3-pentensäure werden mit 50 ml 8 N ethanolischer Chlorwasserstofflösung versetzt und 25 Stunden bei 40° gerührt. Das Reaktionsgemisch wird im Vakuum zur Trockne eingedampft. Zweimaliges Eindampfen nach Zusatz von Ethanol/Toluol 1:1 ergibt einen öligen Rückstand, der in 5 ml Ethanol gelöst und tropfenweise mit einer Lösung von 5 ml Propylenoxid in 5 ml Ethanol versetzt wird. Das kristallin anfallende Produkt wird nach 2 Stunden abfiltriert und mit Ethanol und Ether gewaschen. Nach dem Trocknen (80°, 4 Stunden) im Hochvakuum wird der E-2-Methylamino-4-methyl-5-phosphono-3-pentensäureethylester erhalten, Fp. 235° - 240° (unter Zersetzung).

Beispiel 23:

Herstellung von 1000 Kapseln mit einem Gehalt von jeweils 10 mg der aktiven Substanz des Beispiels 6 mit folgender Zusammensetzung:
E-2-Amino-6-phosphono-4-hexensäure 10,0 g
Milchzucker 207,0 g
Modifizierte Stärke 80,0 g
Magnesiumstearat 3,0 g

Verfahren:

Sämtliche pulvrigen Bestandteile werden mit einem Sieb von 0,6 mm Maschenweite gesiebt. Dann wird der Wirkstoff in einen geeigneten gegeben und bis zur Homogenität zuerst mit Magnesiumstearat, dann mit Milchzucker und Stärke vermischt. No. 2 Gelatinekapseln werden mit je 300 mg dieser Mischung gefüllt, wobei man eine Kapselabfüllmaschine verwendet.

Auf analoge Weise werden Kapseln hergestellt, die 10-200 mg einer anderen offenbarten und in den Beispielen 1 - 21 erwähnten Verbindungen enthalten.

Beispiel 24:

Herstellung von 10'000 Tabletten mit einem Gehalt von je 10 mg der aktiven Substanz des Beispiels 6 mit folgender Zusammensetzung:

E-2-Amino-6-phosphono-4-hexensäure 100,00 g
Milchzucker 2,535,00 g
Maisstärke 125,00 g
Polyethylenglykol 6000 150,00 g
Magnesiumstearat 40,00 g
gereinigtes Wasser q.s.

Verfahren:

Sämtliche pulvrigen Bestandteile werden mit einem Sieb von 0,6 mm Maschenweite gesiebt. Dann wird der Wirkstoff mit Milchzucker, Magnesiumstearat und mit der Hälfte der Stärke in einem geeigneten Mischer vermischt. Die andere Hälfte der Stärke wird in 65 ml Wasser suspendiert, und die Suspension wird zur siedenden Lösung von Polyethylenglykol in 260 ml Wasser gegeben. Die erhaltene Paste wird zu den Pulvern gegeben und, gegebenenfalls unter Zugabe einer weiteren Wasser menge, granuliert. Das Granulat wird über Nacht bei 35° getrocknet, durch ein Sieb von 1,2 mm Maschenweite getrieben und zu Tabletten gepresst, die eine Bruchrille aufweisen.

Auf analoge Weise werden Tabletten hergestellt, die 10-200 mg einer der anderen offenbarten und in den Beispielen 1 - 21 erwähnten Verbindungen enthalten.

**Patentansprüche**

1. Verfahren zur Herstellung ungesättigter Aminosäureverbindungen der Formel

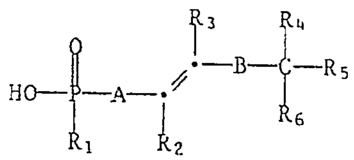

(I),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für gegebenenfalls verestertes oder amidiertes Carboxy steht, $R_6$ eine unsubstituierte durch Niederalkyl oder Arylniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, und ihrer Salze, dadurch gekennzeichnet, dass man in einer Verbindung der Formel

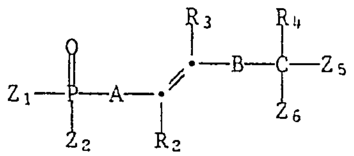

(II),

worin $Z_1$ gegebenenfalls geschütztes Hydroxy bedeutet, $Z_2$ eine Gruppe $R_1$ oder geschütztes Hydroxy bedeutet, $Z_5$ eine Gruppe $R_5$ oder geschütztes Carboxy bedeutet. $Z_6$ eine geschützte Gruppe $R_6$ darstellt und $R_1$, $R_2$, $R_3$, $R_4$, $R_5$, $R_6$, A und B die vorstehend angegebenen Bedeutungen haben, die geschützten Gruppen $Z_6$ und gegebenenfalls $Z_1$, $Z_2$ und/oder $Z_5$ durch Behandeln mit einem Triniederalkylhalogensilan freisetzt und gewünschtenfalls die erhaltene Verbindung in eine andere Verbindung der Formel I umwandelt, ein erhaltenes Gemisch optischer Isomeren in die Komponenten auftrennt und das gewünschte Isomer abtrennt und/oder eine erhaltene freie Verbindung in ein Salz oder ein erhaltenes

Salz in die freie Verbindung oder in ein anderes Salz überführt.

2. Verfahren gemäss Anspruch 1, dadurch gekennzeichnet, dass man eine Verbindung der Formel

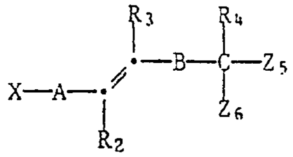

(III)

worin $R_2$, $R_3$, $R_4$, A und B wie für Formel I definiert sind, $Z_5$ die Bedeutung von $R_5$ hat oder für geschütztes Carboxy steht, $Z_6$ eine geschützte Gruppe $R_6$ bedeutet und X für reaktionsfähiges verestertes Hydroxy steht, mit einer Verbindung der Formel

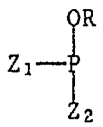

(IV),

worin $Z_1$ für gegebenenfalls geschütztes Hydroxy steht, $Z_2$ die Bedeutung von $R_1$ hat oder für geschütztes Hydroxy steht und R eine verethernde Gruppe bedeutet, umsetzt, in dem erhaltenen Zwischenprodukt der Formel

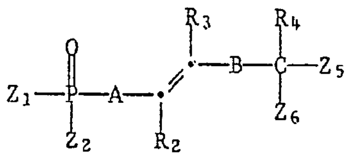

(II),

die geschützten Gruppen $Z_6$ und gegebenenfalls $Z_1$, $Z_2$ und/oder $Z_5$ durch Behandeln mit einem Triniederalkylhalogensilan freisetzt und gewünschtenfalls in einer verfahrensgemäss erhältlichen Verbindung, worin $R_5$ verestertes Carboxy ist, die veresterte Carboxygruppe in Carboxy oder in einer verfahrensgemäss erhältlichen Verbindung, worin $R_5$ Carboxy ist, die Carboxygruppe in eine veresterte Carboxygruppe überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

3. Verfahren gemäss Anspruch 1 zur Herstellung von Verbindungen der Formel I, worin $R_1$, $R_2$, $R_3$, $R_4$, $R_6$, A und B die in Anspruch 1 angegebenen Bedeutungen haben und $R_5$ verestertes oder amidiertes Carboxy bedeutet, und ihrer Salze, dadurch gekennzeichnet, dass man eine Verbindung der Formel

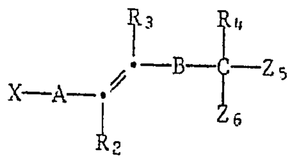

(III)

worin $R_2$, $R_3$, $R_4$, A und B wie für Formel I definiert sind, $Z_5$ die Bedeutung von $R_5$ hat, $Z_6$ für geschütztes Amino steht und X für reaktionsfähiges verestertes Hydroxy steht, mit einer Verbindung der Formel III,

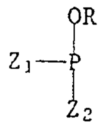

(IV)

worin $Z_1$ für gegebenenfalls geschütztes Hydroxy steht, $z_2$ die Bedeutung von $R_1$ hat oder für geschütztes Hydroxy steht und R eine verethernde Gruppe bedeutet, umsetzt, in dem erhaltenen Zwischenprodukt die geschützten Gruppen $Z_6$ und gegebenenfalls $Z_1$, $Z_2$ und/oder $Z_5$ unter Erhalt der Gruppe $R_5$ durch Behandeln mit Trimethylbromsilan freisetzt und gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

4. Verfahren gemäss Anspruch 1 zur Herstellung von Verbindungen der Formel I, worin $R_1$, $R_2$, $R_3$, $R_4$, $R_6$, A und B die in Anspruch 1 angegebenen Bedeutungen haben und $R_5$ Carboxy bedeutet, und ihrer Salze, dadurch gekennzeichnet, dass man in einer Verbindung der Formel II, worin $Z_1$ gegebenenfalls geschütztes Hydroxy bedeutet, $Z_2$ eine Gruppe $R_1$ oder geschütztes Hydroxy bedeutet, $Z_5$ Carboxy oder verestertes, amidiertes oder geschütztes Carboxy bedeutet und $Z_6$ eine geschützte Gruppe $R_6$ bedeutet und $R_1$, $R_2$, $R_3$, $R_4$, $R_6$, A und B obige Bedeutungen haben, die geschützten Gruppen $Z_6$ und gegebenenfalls $Z_1$, $Z_2$ und/oder $Z_5$ durch Behandeln mit einem Triniederalkylhalogensilan freisetzt und in einer verfahrensgemäss erhältlichen Verbindung, worin, $R_5$ verestertes oder amidiertes Carboxy ist, die veresterte oder amidierte carboxygruppe in Carboxy überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

5. Verfahren gemäss einem der Ansprüche 1-4, dadurch gekennzeichnet, dass man von einer Verbindung der Formeln II oder IV ausgeht, worin geschütztes Hydroxy $Z_1$ und/oder $Z_2$ mit einem aliphatischen Alkohol verethertes Hydroxy, wie mit einem gegebenenfalls durch Halogen oder in höherer als der α-Stellung durch Hydroxy, Oxo, Niederalkoxy, Niederalkanoyloxy und/oder Mono- oder Diniederalkylamino substituierten Niederalkanol, Niederalkenol oder Niederalkinol verethertes Hydroxy, darstellt.

6. Verfahren gemäss einem der Ansprüche 1-4, dadurch gekennzeichnet, dass man von einer Verbindung der Formel II oder IV ausgeht, worin geschütztes Hydroxy $Z_1$ und/oder $Z_2$ Niederalkoxy bedeutet.

7. Verfahren gemäss einem der Ansprüche 1-6, dadurch gekennzeichnet, dass man von einer Verbindung der Formeln II oder III und IV ausgeht, worin die geschützte Gruppe $Z_6$ eine gegebenenfalls durch Niederalkyl oder Arylniederalkyl N-substituierte Acylaminogruppe darstellt.

8. Verfahren gemäss einem der Ansprüche 1-6, dadurch gekennzeichnet, dass man von einer Verbindung der Formeln II oder III ausgeht, worin die geschützte Gruppe $Z_6$ eine Acylaminogruppe darstellt.

9. Verfahren gemäss einem der Ansprüche 1-8, dadurch gekennzeichnet, dass man von einer Verbindung der Formeln II oder III und IV ausgeht, worin die geschützte Gruppe $Z_6$ eine gegebenenfalls durch Niederalkyl oder Arylniederalkyl N-substituierte Acylaminogruppe, worin Acyl die Acylgruppe eines Kohlensäurehalbesters bedeutet, darstellt.

10. Verfahren gemäss einem der Ansprüche 1-8, dadurch gekennzeichnet, dass man von einer Verbindung der Formeln II oder III und IV ausgeht, worin die geschützte Gruppe $Z_6$ eine gegebenenfalls in 1- oder 2-Stellung substituierte Niederalkoxycarbonylaminogruppe, beispielsweise Tertiärbutyloxycarbonylamino, bedeutet.

11. Verfahren gemäss einem der Ansprüche 1-8, dadurch gekennzeichnet, dass man von einer Verbindung der Formeln II oder III und IV ausgeht, worin die geschützte Gruppe $Z_6$ eine gegebenenfalls durch Niederalkyl oder Arylniederalkyl N-substituierte Niederalkanoylaminogruppe, beispielsweise Formylamino, darstellt.

12. Verfahren gemäss einem der Ansprüche 1-8, dadurch gekennzeichnet, dass man von einer Verbindung der Formeln II oder III ausgeht, worin geschütztes Amino $Z_6$ eine Niederalkanoylaminogruppe, beispielsweise Formylamino, darstellt.

13. Verfahren gemäss einem der Ansprüche 1-12, dadurch gekennzeichnet, dass man als Triniederalkylhalogensilan ein Triniederalkylchlorsilan, Triniederalkyljodsilan oder Triniederalkylbromsilan verwendet.

14. Verfahren gemäss einem der Ansprüche 1-12, dadurch gekennzeichnet, dass man als Triniederalkylhalogensilan ein Triniederalkylbromsilan verwendet.

15. Verfahren gemäss einem der Ansprüche 1-12, dadurch gekennzeichnet, dass man als Triniederalkylhalogensilan Trimethylbromsilan verwendet.

16. Verfahren gemäss einem der Ansprüche 1-14, dadurch gekennzeichnet, dass man die Freisetzung der geschützten Gruppen $Z_1$, $Z_2$, $Z_5$ und/oder $Z_6$ in einem halogenierten Kohlenwasserstoff vornimmt.

17. Verfahren gemäss einem der Ansprüche 1-16, dadurch gekennzeichnet, dass man die Freisetzung der geschützten Gruppen $Z_1$, $Z_2$, $Z_5$ und/oder $Z_6$ in einem halogenierten aliphatischen Kohlenwasserstoff vornimmt.

18. Verfahren gemäss einem der Ansprüche 1-16, dadurch gekennzeichnet, dass man in Dichlormethan als Lösungs-oder Verdünnungsmittel arbeitet.

19. Verfahren gemäss einem der Ansprüche 1-18, dadurch gekennzeichnet, dass man einen Halogenwasserstoffabfänger hinzufügt.

20. Verfahren gemäss einem der Ansprüche 1-18, dadurch gekennzeichnet, dass man bei der Verwendung von Trimethylbromsilan als Triniederalkylhalogensilan einen Bromwasserstoffabfänger hinzufügt.

21. Verfahren gemäss einem der Ansprüche 1-20, dadurch gekennzeichnet, dass man ein Epoxyniederalkan im Gemisch mit einem Niederalkanol als Halogenwasserstoffabfänger hinzufügt.

22. Verfahren gemäss einem der Ansprüche 1-20, dadurch gekennzeichnet, dass man Propylenoxid im Gemisch mit Ethanol als Halogenwasserstoffabfänger hinzufügt.

23. Verfahren gemäss einem der Ansprüche 1-20, dadurch gekennzeichnet, dass man bei der Verwendung von Trimethylbromsilan als Triniederalkylhalogensilan Propylenoxid im Gemisch mit Ethanol als Bromwasserstoffabfänger hinzufügt.

24. Verfahren gemäss einem der Ansprüche 1-23 zur Herstellung ungesättigter Aminosäureverbindungen der Formel

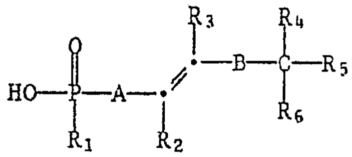

(I),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für gegebenenfalls verestertes oder amidiertes Carboxy steht, $R_6$ eine unsubstituierte durch Niederalkyl oder Aryniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, in Form derjenigen Enantiomeren, in denen das C-Atom, welches die Aminogruppe $R_6$ trägt, R-Konfiguration besitzt, und ihrer Salze, dadurch gekennzeichnet, dass man von einer Verbindung der Formel II bzw. von Verbindungen der Formeln III und IV ausgeht, worin das C-Atom, welches die Gruppe $Z_6$ trägt, R-Konfiguration aufweist.

25. Verfahren zur Herstellung ungesättigter Aminosäureverbindungen der Formel

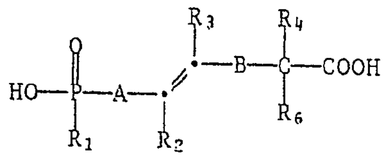

(Ia),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für Carboxy steht, $R_6$ eine unsubstituierte durch Niederalkyl oder Arylniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, und ihrer Salze, dadurch gekennzeichnet, dass man eine Verbindung der Formel,

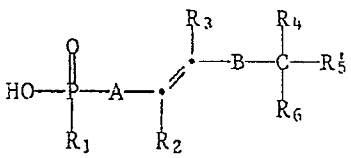

(Ib),

worin $R_5^!$ eine veresterte Carboxygruppe bedeutet, ohne Zusatz von Säure oder Base in Wasser hydrolysiert.

26. Verfahren gemäss Anspruch 25, dadurch gekennzeichnet, dass man von einer Verbindung der Formel Ib ausgeht, in der $R_5^!$ eine Niederalkoxycarbonylgruppe bedeutet.

27. Verfahren gemäss einem der Ansprüche 1-26, dadurch gekennzeichnet, dass man Verbindungen der Formel I, worin $R_1$ Wasserstoff, Alkyl mit bis und mit 12 Kohlenstoffatomen oder Hydroxy bedeutet, $R_2$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R_3$ Wasserstoff, Niederalkyl oder unsubstituiertes oder substituiertes Phenyl bedeutet, $R_4$ für Wasserstoff oder Niederalkyl steht, $R_5$ freies oder pharmazeutisch annehmbares verestertes oder amidiertes Carboxy bedeutet, $R_6$ für Amino, Mono- oder Diniederalkylamino steht, A unsubstituiertes oder durch Niederalkyl substituiertes α,ω-Alkylen mit 1 bis 3 Kohlenstoffatomen oder eine Bindung bedeutet, und B Methylen oder eine Bindung bedeutet, mit der Massgabe, dass A von einer Bindung verschieden ist, wenn B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, oder ein pharmazeutisch verwendbares Salz davon herstellt.

28. Verfahren gemäss einem der Ansprüche 3, 5, 6, 8, 12, 15, 16, 18, 20 und 23, dadurch gekennzeichnet, dass man Verbindungen der Formel I, worin $R_1$ Wasserstoff, Alkyl mit bis und mit 12 Kohlenstoffatomen oder Hydroxy -bedeutet, $R_2$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R_3$ Wasserstoff, Niederalkyl oder unsubstituiertes oder substituiertes Phenyl bedeutet, $R_4$ für Wasserstoff oder Niederalkyl steht, $R_5$ pharmazeutisch annehmbares verestertes oder amidiertes Carboxy bedeutet, $R_6$ für Amino, Mono- oder Diniederalkylamino steht, A unsubstituiertes oder durch Niederalkyl substituiertes α,ω-Alkylen mit 1 bis 3 Kohlenstoffatomen oder eine Bindung bedeutet, und B Methylen oder eine Bindung bedeutet, mit der Massgabe, dass A von einer Bindung verschieden ist, wenn B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, oder ein pharmazeutisch verwendbares Salz davon herstellt.

29. Verfahren gemäss Anspruch 28, dadurch gekennzeichnet, dass man Verbindungen der Formel I herstellt, worin $R_1$ bis $R_4$, A und B die in Anspruch 27 angegebenen Bedeutungen haben, $R_5$ Niederalkoxycarbonyl oder durch Amino, Mono- oder Diniederalkylamino, Hydroxy oder Niederalkanoyloxy substituiertes Niederalkoxycarbonyl bedeutet und $R_6$ für Amino oder Mononiederalkylamino steht, oder ein pharmazeutisch verwendbares Salz davon herstellt.

30. Verfahren gemäss einem der Ansprüche 1-26, dadurch gekennzeichnet, dass man Verbindungen der Formel I, worin $R_1$ für Wasserstoff, Alkyl mit bis und mit 12 Kohlenstoffatomen oder Hydroxy steht, $R_2$ für Wasserstoff, Niederalkyl, Phenyl, Halogenphenyl oder Phenylniederalkyl steht, $R_3$ und $R_4$ Wasserstoff oder Niederalkyl bedeuten, $R_5$ Carboxy ist und $R_6$ Amino bedeutet, wobei A für unsubstituiertes oder durch Niederalkyl substituiertes α,ω-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein pharmazeutisch verwendbares Salz davon herstellt.

31. Verfahren gemäss einem der Ansprüche 3, 5, 6, 8, 12, 15, 16, 18, 20 und 23, dadurch gekennzeichnet, dass man Verbindungen der Formel I, worin $R_1$ für Wasserstoff, Alkyl mit bis und mit 12 Kohlenstoffatomen oder Hydroxy steht, $R_2$ für Wasserstoff, Niederalkyl, Phenyl, Halogenphenyl oder Phenylniederalkyl steht, $R_3$ und $R_4$ Wasserstoff oder Niederalkyl bedeuten, $R_5$ für Alkoxycarbonyl oder Hydroxyniederalkoxycarbonyl steht und $R_6$ Amino oder Mononiederalkylamino bedeutet, wobei A für unsubstituiertes oder durch Niederalkyl substituiertes α,ω-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein pharmazeutisch verwendbares Salz davon herstellt.

32. Verfahren gemäss einem der Ansprüche 1-26, dadurch gekennzeichnet, dass man Verbindung der Formel I, worin $R_1$ worin $R_1$ für Wasserstoff, Alkyl mit bis und mit 12 C-Atomen oder Hydroxy steht, $R_2$ Wasserstoff, Niederalkyl oder Halogenphenyl bedeutet, $R_3$ für Wasserstoff oder Halogenphenyl steht, $R_4$ Wasserstoff bedeutet, $R_5$ Carboxy ist und $R_6$ Amino bedeutet, wobei A für α,ω-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein

pharmazeutisch verwendbares Salz davon herstellt.

33. Verfahren gemäss einem der Ansprüche 3, 5, 6, 8, 12, 15, 16, 18, 20 und 23, dadurch gekennzeichnet, das man Verbindungen der Formel I, worin $R_1$ für Wasserstoff, Alkyl mit bis und mit 12 C-Atomen oder Hydroxy steht, $R_2$ Wasserstoff, Niederalkyl oder Halogenphenyl bedeutet, $R_3$ für Wasserstoff oder Halogenphenyl steht, $R_4$ Wasserstoff bedeutet, $R_5$ Niederalkoxycarbonyl oder Hydroxyniederalkoxycarbonyl und $R_6$ Amino oder Mononiederalkylamino bedeutet, A für α,ω-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein pharmazeutisch verwendbares Salz davon herstellt.

34. Verfahren gemäss einem der Ansprüche 1-26, dadurch gekennzeichnet, dass man Verbindungen der Formel I, worin $R_1$ für Wasserstoff, Niederalkyl oder Hydroxy steht, $R_2$ Wasserstoff oder Niederalkyl bedeutet, $R_3$ und $R_4$ für Wasserstoff stehen, $R_5$ für Carboxy oder Niederalkoxycarbonyl steht und $R_6$ Amino bedeutet, wobei A für α,ω-Alkylen mit 1 bis 3 Kohlenstoffatomen steht, B eine Bindung bedeutet, oder ein pharmazeutisch verwendbares Salz davon herstellt.

35. Verfahren gemäss einem der Ansprüche 3, 5, 6, 8, 12, 15, 16, 18, 20 und 23, dadurch gekennzeichnet, dass man Verbindungen der Formel I, worin $R_1$ für Wasserstoff, Niederalkyl oder Hydroxy steht, $R_2$ Wasserstoff oder Niederalkyl bedeutet, $R_3$ und $R_4$ fur Wasserstoff stehen, $R_5$ für Niederalkoxycarbonyl steht und $R_6$ Amino bedeutet, wobei A für α,ω-Alkylen mit 1 bis 3 Kohlenstoffatomen steht, B eine Bindung bedeutet, oder ein pharmazeutisch verwendbares Salz davon herstellt.

36. Verfahren gemäss einem der Ansprüche 1, 2 und 4-25, dadurch gekennzeichnet, dass man von Verbindungen der Formel I, worin $R_1$ Hydroxy bedeuten, $R_2$ für Wasserstoff oder Niederalkyl steht, $R_3$ und $R_4$ für Wasserstoff stehen, $R_5$ Niederalkoxycarbonyl oder Carboxy bedeutet, $R_7$ für Amino steht, A Methylen und B eine Bindung bedeutet, oder ein pharmazeutisch verwendbares Salz davon herstellt.

37. Verfahren gemäss einem der Ansprüche 3, 5, 6, 8, 12, 15, 16, 18, 20, 23 und 26, dadurch gekennzeichnet, dass man Verbindungen der Formel I, worin $R_1$ Hydroxy bedeuten, $R_2$ für Wasserstoff oder Niederalkyl steht, $R_3$ und $R_4$ für Wasserstoff stehen, $R_5$ Niederalkoxycarbonyl oder Carboxy bedeutet, $R_7$ für Amino steht, A Methylen und B eine Bindung bedeutet, oder ein pharmazeutisch verwendbares Salz davon herstellt.

38. Verfahren gemäss einem der Ansprüche 1, 2 und 24, dadurch gekennzeichnet, dass man E-2-Amino-4-methyl-5-phosphono-3-pentensäureethylester oder ein pharmazeutisch verwendbares Salz davon herstellt.

39. Verfahren gemäss einem der Ansprüche 3, 5, 6, 8, 15, 16, 18, 20 und 23, dadurch gekennzeichnet, dass man E-2-Amino-4-methyl-5-phosphono-3-pentensäureethylester herstellt.

40. Verfahren gemäss einem der Ansprüche 1, 2 und 4-24, dadurch gekennzeichnet, dass man E-2-Amino-4-methyl-5-phosphono-3-pentensäure oder ein pharmazeutisch verwendbares Salz davon herstellt.

41. Verfahren gemäss Anspruch 26, dadurch gekennzeichnet, dass man E-2-Amino-4-methyl-5-phosphono-3-pentensäure oder ein pharmazeutisch verwendbares Salz davon herstellt.

42. Verfahren gemäss der Ansprüche 1, 2 und 4-24, dadurch gekennzeichnet, dass man E-2-Amino-4-methyl-5-phosphono-3-pentensäuremethylester, E-2-Amino-4-methyl-5-phosphono-3-pentensäure-n-propylester, E-2-Amino-4-methyl-5-phosphono-3-pentensäure-n-butylester, E-2-Amino-4-methyl-5-phosphono-3-pentensäure-iso-butylester, E-2-Amino-4-methyl- 5-phosphono-3-pentensäure-n-pentylester, E-2-Amino-4-methyl-5-phosphono-3-pentensäure-n-hexylester oder jeweils ein pharmazeutisch verwendbares Salz davon herstellt.

43. Verfahren gemäss einem der Ansprüche 3, 5, 6 8, 15, 16, 18, 20 und 23, dadurch gekennzeichnet, dass man E-2-Amino-4-methyl-5-phosphono-3-pentensäuremethylester herstellt, E-2-Amino-4-methyl-5-phosphono-3-pentensäure-n-propylester, E-2-Amino-4-methyl-5-phosphono-3-pentensäure-n-butylester, E-2-Amino-4-methyl-5-phosphono-3-pentensäure-iso-butylester, E-2-Amino-4-methyl-5-phosphono-3-pentensäure-n-pentylester oder E-2-Amino-4-methyl-5-phosphono-3-pentensäure-n-hexylester oder jeweils ein pharmazeutisch verwendbares Salz davon herstellt.

44. Verfahren gemäss Anspruch 1 zur Herstellung von E-2-Amino-4-phosphonomethyl-3,6-heptadiensäure und ihrer pharmazeutisch verwendbaren Salze, dadurch gekennzeichnet, dass man E-2-Formylamino-4-diethylphosphonomethyl-3,6-heptadiensäuremethylester in Dichlormethan mit Trimethyljodsilan umsetzt und das Primärprodukt mit Salzsäure behandelt.

45. Verfahren gemäss einem der Ansprüche 1-44, dadurch gekennzeichnet, dass man dasjenige Enantiomere herstellt, in dem das C-Atom, welches die Aminogruppe $R_6$ trägt, R-Konfiguration besitzt.

46. Verfahren gemäss einem der Ansprüche 1, 2 und 4-24, dadurch gekennzeichnet, dass man (2R)-E-2-Amino-4-methyl-5-phosphono-3-pentensäure oder ein pharmazeutisch verwendbares Salz davon herstellt.

47. Verfahren gemäss Anspruch 26, dadurch gekennzeichnet, dass man (2R)-E-2-Amino-4-methyl-5-phosphono-3-pentensäure oder ein pharmazeutisch verwendbares Salz davon herstellt.

48. Verfahren gemäss einem der Ansprüche 1, 2 und 4-24, dadurhc gekennzeichnet, dass man (2R)-E-2-Amino-4-methyl-5-phosphono-3-pentensäureethylester oder ein pharmazeutisch verwendbares Salz davon herstellt.

49. Verfaren gemäss einem der Ansprüche 3, 5, 6, 8, 12, 15, 18, 20 und 23, dadurch gekennzeichnet, dass

man (2R)-E-2-Amino-4-methyl-5-phosphono-3-pentensäureethylester oder ein pharmazeutisch verwendbares Salz davon herstellt.

50. (2R)-E-Amino-4-methyl-5-phosphono-3-pentensäureethylester oder ein pharmazeutisch verwendbares Salz davon sowie pharmazeutisch Präparate, enthaltend (2R)-E-2-Amino-4-methyl-5-phosphono-3-pentensäureethylester oder ein pharmazeutisch verwendbares Salz davon neben üblichen pharmazeutischen Hilfsstoffen.

**Patentansprüche für die folgenden Vertragsstaaten: AT, ES und GR**

1. Verfahren zur Herstellung ungesättigter Aminosäureverbindungen der Formel

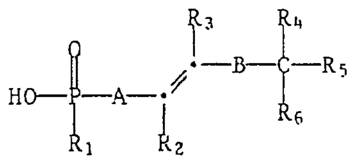

(I),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für gegebenenfalls verestertes oder amidiertes Carboxy steht, $R_6$ eine unsubstituierte durch Niederalkyl oder Arylniederalkyl substituierte Aminogruppe darstellt, A unsubstituiertes oder durch Alkyl substituiertes $\alpha,\omega$-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes $\alpha,\omega$-Alkylen mit 1 bis 3 C-Atomen darstellt, wenn B für eine direkte Bindung steht, und ihrer Salze, dadurch gekennzeichnet, dass man in einer Verbindung der Formel

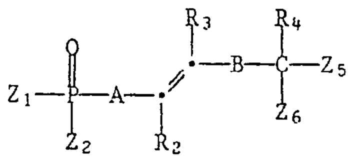

(II),

worin $Z_1$ gegebenenfalls geschütztes Hydroxy bedeutet, $Z_2$ eine Gruppe $R_1$ oder geschütztes Hydroxy bedeutet, $Z_5$ eine Gruppe $R_5$ oder geschütztes Carboxy bedeutet, $Z_6$ eine geschützte Gruppe $R_6$ darstellt und $R_1$, $R_2$, $R_3$, $R_4$, $R_5$, $R_6$, A und B die vorstehend angegebenen Bedeutungen haben, die geschützten Gruppen $Z_6$ und gegebenenfalls $Z_1$, $Z_2$ und/oder $Z_5$ durch Behandeln mit einem Triniederalkylhalogensilan freisetzt und gewünschtenfalls die erhaltene Verbindung in eine andere Verbindung der Formel I umwandelt, ein erhaltenes Gemisch optischer Isomeren in die Komponenten auftrennt und das gewünschte Isomer abtrennt und/oder eine erhaltene freie Verbindung in ein Salz oder ein erhaltenes Salz in die freie Verbindung oder in ein anderes Salz überführt.

2. Verfahren gemäss Anspruch 1, dadurch gekennzeichnet, dass man eine Verbindung der Formel

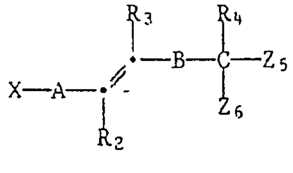

(III)

worin $R_2$, $R_3$, $R_4$, A und B wie für Formel I definiert sind, $Z_5$ die Bedeutung von $R_5$ hat oder für geschütztes Carboxy steht, $Z_6$ eine geschützte Gruppe $R_6$ bedeutet und X für reaktionsfähiges verestertes Hydroxy steht, mit einer Verbindung der Formel

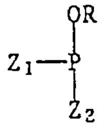 (IV),

worin $Z_1$ für gegebenenfalls geschütztes Hydroxy steht, $Z_2$ die Bedeutung von $R_1$ hat oder für geschütztes Hydroxy steht und R eine verethernde Gruppe bedeutet, umsetzt, in dem erhaltenen Zwischenprodukt der Formel

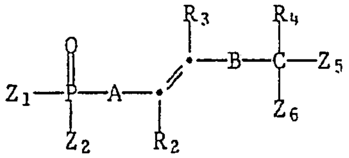 (II),

die geschützten Gruppen $Z_6$ und gegebenenfalls $Z_1$, $Z_2$ und/oder $Z_5$ durch Behandeln mit einem Triniederalkylhalogensilan freisetzt und gewünschtenfalls in einer verfahrensgemäss erhältlichen Verbindung, worin $R_5$ verestertes Carboxy ist, die veresterte Carboxygruppe in Carboxy oder in einer verfahrensgemäss erhältlichen Verbindung, worin $R_5$ Carboxy ist, die Carboxygruppe in eine veresterte Carboxygruppe überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

3. Verfahren gemäss Anspruch 1 zur Herstellung von Verbindungen der Formel I, worin $R_1$, $R_2$, $R_3$, $R_4$, $R_6$, A und B die in Anspruch 1 angegebenen Bedeutungen haben und $R_5$ verestertes oder amidiertes carboxy bedeutet, und ihrer Salze, dadurch gekennzeichnet, dass man eine Verbindung der Formel

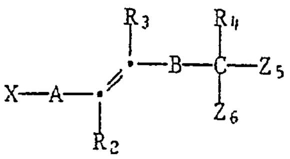 (III)

worin $R_2$, $R_3$, $R_4$, A und B wie für Formel I definiert sind, $Z_5$ die Bedeutung von $R_5$ hat, $Z_6$ für geschütztes Amino steht und X für reaktionsfähiges verestertes Hydroxy steht, mit einer Verbindung der Formel III,

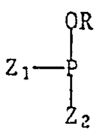 (IV)

worin $Z_1$ für gegebenenfalls geschütztes Hydroxy steht, $z_2$ die Bedeutung von $R_1$ hat oder für geschütztes Hydroxy steht und R eine verethernde Gruppe bedeutet, umsetzt, in dem erhaltenen Zwischenprodukt die geschützten Gruppen $Z_6$ und gegebenenfalls $Z_1$, $Z_2$ und/oder $Z_5$ unter Erhalt der Gruppe $R_5$ durch Behandeln mit Trimethylbromsilan freisetzt und gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

4. Verfahren gemäss Anspruch 1 zur Herstellung von Verbindungen der Formel I, worin $R_1$, $R_2$, $R_3$, $R_4$, $R_6$, A und B die in Anspruch 1 angegebenen Bedeutungen haben und $R_5$ Carboxy bedeutet, und ihrer Salze, dadurch gekennzeichnet, dass man in einer Verbindung der Formel II, worin $Z_1$ gegebenenfalls geschütztes Hydroxy bedeutet, $Z_2$ eine Gruppe $R_1$ oder geschütztes Hydroxy bedeutet, $Z_5$ Carboxy oder verestertes, amidiertes oder geschütztes Carboxy bedeutet und $Z_6$ eine geschützte Gruppe $R_6$ bedeutet und $R_1$, $R_2$, $R_3$, $R_4$, $R_6$, A und B obige Bedeutungen haben, die geschützten Gruppen $Z_6$ und gegebenenfalls $Z_1$, $Z_2$ und/oder $Z_5$ durch Behandeln mit einem Triniederalkylhalogensilan freisetzt und in einer verfahrensgemäss erhältlichen Verbindung, worin, $R_5$ verestertes oder amidiertes Carboxy ist, die

veresterte oder amidierte Carboxygruppe in Carboxy überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I eines Salzes davon isoliert.

5. Verfahren gemäss einem der Ansprüche 1-4, dadurch gekennzeichnet, dass man von einer Verbindung der Formeln II oder IV ausgeht, worin geschütztes Hydroxy $Z_1$ und/oder $Z_2$ mit einem aliphatischen Alkohol verethertes Hydroxy, wie mit einem gegebenenfalls durch Halogen oder in höherer als der α-Stellung durch Hydroxy, Oxo, Niederalkoxy, Niederalkanoyloxy und/oder Mono- oder Diniederalkylamino substituierten Niederalkanol, Niederalkenol oder Niederalkinol verethertes Hydroxy, darstellt.

6. Verfahren gemäss einem der Ansprüche 1-4, dadurch gekennzeichnet, dass man von einer Verbindung der Formel II oder IV ausgeht, worin geschütztes Hydroxy $Z_1$ und/oder $Z_2$ Niederalkoxy bedeutet.

7. Verfahren gemäss einem der Ansprüche 1-6, dadurch gekenn:ij, dass man von einer Verbindung der Formeln II oder III und IV ausgeht, worin die geschützte Gruppe $Z_6$ eine gegebenenfalls durch Niederalkyl oder Arylniederalkyl N-substituierte Acylaminogruppe darstellt.

8. Verfahren gemäss einem der Ansprüche 1-6, dadurch gekennzeichnet, dass man von einer Verbindung der Formeln II oder III ausgeht, worin die geschützte Gruppe $Z_6$ eine Acylaminogruppe darstellt.

9. Verfahren gemäss einem der Ansprüche 1-8, dadurch gekennzeichnet, dass man von einer Verbindung der Formeln II oder III und IV ausgeht, worin die geschützte Gruppe $Z_6$ eine gegebenenfalls durch Niederalkyl oder Arylniederalkyl N-substituierte Acylaminogruppe, worin Acyl die Acylgruppe eines Kohlensäurehalbesters bedeutet, darstellt.

10. Verfahren gemäss einem der Ansprüche 1-8, dadurch gekennzeichnet, dass man von einer Verbindung der Formeln II oder III und IV ausgeht, worin die geschützte Gruppe $Z_6$ eine gegebenenfalls in 1- oder 2-Stellung substituierte Niederalkoxycarbonylaminogruppe, beispielsweise Tertiärbutyloxycarbonylamino, bedeutet.

11. Verfahren gemäss einem der Ansprüche 1-8, dadurch gekennzeichnet, dass man von einer Verbindung der Formeln II oder III und IV ausgeht, worin die geschützte Gruppe $Z_6$ eine gegebenenfalls durch Niederalkyl oder Arylniederalkyl N-substituierte Niederalkanoylaminogruppe, beispielsweise Formylamino, darstellt.

12. Verfahren gemäss einem der Ansprüche 1-8, dadurch gekennzeichnet, dass man von einer Verbindung der Formeln II oder III ausgeht, worin geschütztes Amino $Z_6$ eine Niederalkanoylaminogruppe, beispielsweise Formylamino, darstellt.

13. Verfahren gemäss einem der Ansprüche 1-12, dadurch gekennzeichnet, dass man als Triniederalkylhalogensilan ein Triniederalkylchlorsilan, Triniederalkyljodsilan oder Triniederalkylbromsilan verwendet.

14. Verfahren gemäss einem der Ansprüche 1-12, dadurch gekennzeichnet, dass man als Triniederalkylhalogensilan ein Triniederalkylbromsilan verwendet.

15. Verfahren gemäss einem der Ansprüche 1-12, dadurch gekennzeichnet, dass man als Triniederalkylhalogensilan Trimethylbromsilan verwendet.

16. Verfahren gemäss einem der Ansprüche 1-14, dadurch gekennzeichnet, dass man die Freisetzung der geschützten Gruppen $Z_1$, $Z_2$, $Z_5$ und/oder $Z_6$ in einem halogenierten Kohlenwasserstoff vornimmt.

17. Verfahren gemäss einem der Ansprüche 1-16, dadurch gekennzeichnet, dass man die Freisetzung der geschützten Gruppen $Z_1$, $Z_2$, $Z_5$ und/oder $Z_6$ in einem halogenierten aliphatischen Kohlenwasserstoff vornimmt.

18. Verfahren gemäss einem der Ansprüche 1-16, dadurch gekennzeichnet, dass man in Dichlormethan als Lösungs-oder Verdünnungsmittel arbeitet.

19. Verfahren gemäss einem der Ansprüche 1-18, dadurch gekennzeichnet, dass man einen Halogenwasserstoffabfänger hinzufügt.

20. Verfahren gemäss einem der Ansprüche 1-18, dadurch gekennzeichnet, dass man bei der Verwendung von Trimethylbromsilan als Triniederalkylhalogensilan einen Bromwasserstoffabfänger hinzufügt.

21. Verfahren gemäss einem der Ansprüche 1-20, dadurch gekennzeichnet, dass man ein Epoxyniederalkan im Gemisch mit einem Niederalkanol als Halogenwasserstoffabfänger hinzufügt.

22. Verfahren gemäss einem der Ansprüche 1-20, dadurch gekennzeichnet, dass man Propylenoxid im Gemisch mit Ethanol als Halogenwasserstoffabfänger hinzufügt.

23. Verfahren gemäss einem der Ansprüche 1-20, dadurch gekennzeichnet, dass man bei der Verwendung von Trimethylbromsilan als Triniederalkylhalogensilan Propylenoxid im Gemisch mit Ethanol als Bromwasserstoffabfänger hinzufügt.

24. Verfahren gemäss einem der Ansprüche 1-23 zur Herstellung ungesättigter Aminosäureverbindungen der Formel

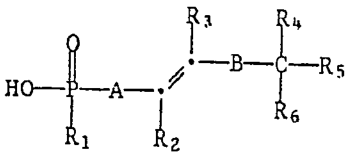

(I),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für gegebenenfalls verestertes oder amidiertes Carboxy steht, $R_6$ eine unsubstituierte oder durch Niederalkyl oder Aryniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, in Form derjenigen Enantiomeren, in denen das C-Atom, welches die Aminogruppe $R_6$ trägt, R-Konfiguration besitzt, und ihrer Salze, dadurch gekennzeichnet, dass man von einer Verbindung der Formel II bzw. von Verbindungen der Formeln III und IV ausgeht, worin das C-Atom, welches die Gruppe $Z_6$ trägt, R-Konfiguration aufweist.

25. Verfahren zur Herstellung ungesättigter Aminosäureverbindungen der Formel

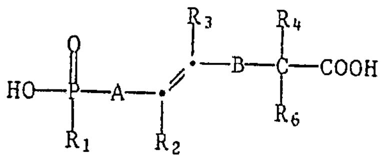

(Ia),

worin $R_1$ Wasserstoff, Alkyl oder Hydroxy bedeutet, $R_2$ Wasserstoff, Alkyl, Halogenniederalkyl, Hydroxyniederalkyl, Niederalkoxyniederalkyl, Arylniederalkyl, Niederalkenyl, Halogen oder Aryl bedeutet, $R_3$ für Wasserstoff, Alkyl oder Aryl steht, $R_4$ Wasserstoff oder Alkyl bedeutet, $R_5$ für Carboxy steht, $R_6$ eine unsubstituierte durch Niederalkyl oder Arylniederalkyl substituierte Aminogruppe darstellt, A unsubstituiertes oder durch Alkyl substituiertes α,ω-Alkylen mit 1 bis 3 Kohlenstoffatomen (C-Atomen) oder eine direkte Bindung darstellt und B Methylen oder eine Bindung bedeutet, mit der Massgabe, dass A unsubstituiertes oder durch Alkyl substituiertes α,ω-Alkylen mit 1 bis 3 C-Atomen darstellt, wenn B für eine direkte Bindung steht, und ihrer Salze, dadurch gekennzeichnet, dass man eine Verbindung der Formel,

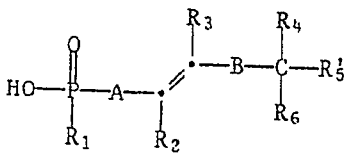

(Ib),

worin $R_5'$ eine veresterte Carboxygruppe bedeutet, ohne Zusatz von Säure oder Base in Wasser hydrolysiert.

26. Verfahren gemäss Anspruch 25, dadurch gekennzeichnet, dass man von einer Verbindung der Formel Ib ausgeht, in der $R_5'$ eine Niederalkoxycarbonylgruppe bedeutet.

27. Verfahren gemäss einem der Ansprüche 1-26, dadurch gekennzeichnet, dass man Verbindungen der Formel I, worin $R_1$ Wasserstoff, Alkyl mit bis und mit 12 Kohlenstoffatomen oder Hydroxy bedeutet, $R_2$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R_3$ Wasserstoff, Niederalkyl oder unsubstituiertes oder substituiertes Phenyl bedeutet, $R_4$ für Wasserstoff oder Niederalkyl steht, $R_5$ freies oder pharmazeutisch annehmbares verestertes oder amidiertes Carboxy bedeutet, $R_6$ für Amino, Mono- oder Diniederalkylamino steht, A unsubstituiertes oder durch Niederalkyl substituiertes α,ω-Alkylen mit 1 bis 3 Kohlenstoffatomen oder eine Bindung bedeutet, und B Methylen oder eine Bindung bedeutet, mit der Massgabe, dass A von einer Bindung verschieden ist, wenn B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, oder ein pharmazeutisch verwendbares Salz davon

herstellt.

28. Verfahren gemäss einem der Ansprüche 3, 5, 6, 8, 12, 15, 16, 18, 20 und 23, dadurch gekennzeichnet, dass man Verbindungen der Formel I, worin $R_1$ Wasserstoff, Alkyl mit bis und mit 12 Kohlenstoffatomen oder Hydroxy -bedeutet, $R_2$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl. Halogen oder unsubstituiertes oder substituiertes Phenyl bedeutet, $R_3$ Wasserstoff, Niederalkyl oder unsubstituiertes oder substituiertes Phenyl bedeutet, $R_4$ für Wasserstoff oder Niederalkyl steht, $R_5$ pharmazeutisch annehmbares verestertes oder amidiertes Carboxy bedeutet, $R_6$ für Amino, Mono- oder Diniederalkylamino steht, A unsubstituiertes oder durch Niederalkyl substituiertes $\alpha,\omega$-Alkylen mit 1 bis 3 Kohlenstoffatomen oder eine Bindung bedeutet, und B Methylen oder eine Bindung bedeutet, mit der Massgabe, dass A von einer Bindung verschieden ist, wenn B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, oder ein pharmazeutisch verwendbares Salz davon herstellt.

29. Verfahren gemäss Anspruch 28, dadurch gekennzeichnet, dass man Verbindungen der Formel I herstellt, worin $R_1$ bis $R_4$, A und B die in Anspruch 27 angegebenen Bedeutungen haben, $R_5$ Niederalkoxycarbonyl oder durch Amino, Mono- oder Diniederalkylamino, Hydroxy oder Niederalkanoyloxy substituiertes Niederalkoxycarbonyl bedeutet und $R_6$ für Amino oder Mononiederalkylamino steht, oder ein pharmazeutisch verwendbares Salz davon herstellt.

30. Verfahren gemäss einem der Ansprüche 1-26, dadurch gekennzeichnet, dass man Verbindungen der Formel I, worin R für Wasserstoff, Alkyl mit bis und mit 12 Kohlenstoffatomen oder Hydroxy steht, $R_2$ für Wasserstoff, Niederalkyl, Phenyl, Halogenphenyl oder Phenylniederalkyl steht, $R_3$ und $R_4$ Wasserstoff oder Niederalkyl bedeuten, $R_5$ Carboxy ist und $R_6$ Amino bedeutet, wobei A für unsubstituiertes oder durch Niederalkyl substituiertes $\alpha,\omega$-Alkylen mit 1 bis 3 Kohlenstoffatomen oder eine Bindung steht und B Methylen oder eine Bindung bedeutet, mit der Massgabe, dass A von einer Bindung verschieden ist, wenn b eine Bindung bedeutet, oder ein pharmazeutisch verwendbares Salz davon herstellt.

31. Verfahren gemäss einem der Ansprüche 3, 5, 6, 8, 12, 15, 16, 18, 20 und 23, dadurch gekennzeichnet, dass man Verbindungen der Formel I, worin $R_1$ für Wasserstoff, Alkyl mit bis und mit 12 Kohlenstoffatomen oder Hydroxy steht, $R_2$ für Wasserstoff, Niederalkyl, Phenyl, Halogenphenyl oder Phenylniederalkyl steht, $R_3$ und $R_4$ Wasserstoff oder Niederalkyl bedeuten, $R_5$ für Alkoxycarbonyl oder Hydroxyniederalkoxycarbonyl steht und $R_6$ Amino oder Mononiederalkylamino bedeutet, wobei A für unsubstituiertes oder durch Niederalkyl substituiertes $\alpha,\omega$-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein pharmazeutisch verwendbares Salz davon herstellt.

32. Verfahren gemäss einem der Ansprüche 1-26, dadurch gekennzeichnet, dass man Verbindung der Formel I, worin $R_1$ für Wasserstoff, Alkyl mit bis und mit 12 C-Atomen oder Hydroxy steht, $R_2$ Wasserstoff, Niederalkyl oder Halogenphenyl bedeutet, $R_3$ für Wasserstoff oder Halogenphenyl steht, $R_4$ Wasserstoff bedeutet, $R_5$ Carboxy ist und $R_6$ Amino bedeutet, wobei A für $\alpha,\omega$-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein pharmazeutisch verwendbares Salz davon herstellt.

33. Verfahren gemäss einem der Ansprüche 3, 5, 6, 8, 12, 15, 16, 18, 20 und 23, dadurch gekennzeichnet, das man Verbindungen der Formel I, worin $R_1$ für Wasserstoff, Alkyl mit bis und mit 12 C-Atomen oder Hydroxy steht, $R_2$ Wasserstoff, Niederalkyl oder Halogenphenyl bedeutet, $R_3$ für Wasserstoff oder Halogenphenyl steht, $R_4$ Wasserstoff bedeutet, $R_5$ Niederalkoxycarbonyl oder Hydroxyniederalkoxycarbonyl und $R_6$ Amino oder Mononiederalkylamino bedeutet, A für $\alpha,\omega$-Alkylen mit 1 bis 3 Kohlenstoffatomen oder eine Bindung steht und B Methylen oder eine Bindung bedeutet, mit der Massgabe, dass A von einer Bindung verschieden ist, wenn B eine Bindung bedeutet, oder ein pharmazeutisch verwendbares Salz davon herstellt.

34. Verfahren gemäss einem der Ansprüche 1-26, dadurch gekennzeichnet, dass man Verbindungen der Formel I, worin $R_1$ für Wasserstoff, Niederalkyl oder Hydroxy steht, $R_2$ Wasserstoff oder Niederalkyl bedeutet, $R_3$ und $R_4$ für Wasserstoff stehen, $R_5$ für Carboxy oder Niederalkoxycarbonyl steht und $R_6$ Amino bedeutet, wobei A für $\alpha,\omega$-Alkylen mit 1 bis 3 Kohlenstoffatomen steht, B eine Bindung bedeutet, oder ein pharmazeutisch verwendbares Salz davon herstellt.

35. Verfahren gemäss einem der Ansprüche 3, 5, 6, 8, 12, 15, 16, 18, 20 und 23, dadurch gekennzeichnet, dass man Verbindungen der Formel I, worin $R_1$ für Wasserstoff, Niederalkyl oder Hydroxy steht, $R_2$ Wasserstoff oder Niederalkyl bedeutet, $R_3$ und $R_4$ fur Wasserstoff stehen, $R_5$ für Niederalkoxycarbonyl steht und $R_6$ Amino bedeutet, wobei A für $\alpha,\omega$-Alkylen mit 1 bis 3 Kohlenstoffatomen steht, B eine Bindung bedeutet, oder ein pharmazeutisch verwendbares Salz davon herstellt.

36. Verfahren gemäss einem der Ansprüche 1, 2 und 4-25, dadurch gekennzeichnet, dass man von Verbindungen der Formel I, worin $R_1$ Hydroxy bedeuten, $R_2$ für Wasserstoff oder Niederalkyl steht, $R_3$ und $R_4$ für Wasserstoff stehen, $R_5$ Niederalkoxycarbonyl oder Carboxy bedeutet, $R_7$ für Amino steht, A Methylen und B eine Bindung bedeutet, oder ein pharmazeutisch verwendbares Salz davon herstellt.

37. Verfahren gemäss einem der Ansprüche 3, 5, 6, 8, 12, 15, 16, 18, 20, 23 und 26, dadurch

gekennzeichnet, dass man Verbindungen der Formel I, worin $R_1$ Hydroxy bedeuten, $R_2$ für Wasserstoff oder Niederalkyl steht, $R_3$ und $R_4$ für Wasserstoff stehen, $R_5$ Niederalkoxycarbonyl oder Carboxy bedeutet, $R_7$ für Amino steht, A Methylen und B eine Bindung bedeutet, oder ein pharmazeutisch verwendbares Salz davon herstellt.

38. Verfahren gemäss einem der Ansprüche 1, 2 und 24, dadurch gekennzeichnet, dass man E-2-Amino-4-methyl-5-phosphono-3-pentensäureethylester oder ein pharmazeutisch verwendbares Salz davon herstellt.

39. Verfahren gemäss einem der Ansprüche 3, 5, 6, 8, 15, 16, 18, 20 und 23, dadurch gekennzeichnet, dass man E-2-Amino-4-methyl-5-phosphono-3-pentensaureethylester herstellt.

40. Verfahren gemäss einem der Ansprüche 1, 2 und 4-24, dadurch gekennzeichnet, dass man E-2-Amino-4-methyl-5-phosphono-3-pentensäure oder ein pharmazeutisch verwendbares Salz davon herstellt.

41. Verfahren gemäss Anspruch 26, dadurch gekennzeichnet, dass man E-2-Amino-4-methyl-5-phosphono-3-pentensäure oder ein pharmazeutisch verwendbares Salz davon herstellt.

42. Verfahren gemäss der Ansprüche 1, 2 und 4-24, dadurch gekennzeichnet, dass man E-2-Amino-4-methyl-5-phosphono-3-pentensäuremethylester, E-2-Amino-4-methyl-5-phosphono-3-pentensäure-n-propylester, E-2-Amino-4-methyl-5-phosphono-3-pentensäure-n-butylester, E-2-Amino-4-methyl-5-phosphono-3-pentensäure-iso-butylester, E-2-Amino-4-methyl- 5-phosphono-3-pentensäure-n-pentylester, E-2-Amino-4-methyl-5-phosphono3-pentensäure-n-hexylester oder jeweils ein pharmazeutisch verwendbares Salz davon herstellt.

43. Verfahren gemäss einem der Ansprüche 3, 5, 6 8, 15, 16, 18, 20 und 23, dadurch gekennzeichnet, dass man E-2-Amino-4-methyl-5-phosphono3-pentensäuremethylester herstellt, E-2-Amino-4-methyl-5-phosphono-3-pentensäure-n-propylester, E-2-Amino-4-methyl-5-phosphono-3-pentensäure-n-butylester, E-2-Amino-4-methyl-5-phosphono-3-pentensäure-iso-butylester, E-2-Amino-4-methyl-5-phosphono-3-pentensäure-n-pentylester oder E-2-Amino-4-methyl-5-phosphono-3-pentensäure-n-hexylester oder jeweils ein pharmazeutisch verwendbares Salz davon herstellt.

44. Verfahren gemäss Anspruch 1 zur Herstellung von E-2-Amino-4-phosphonomethyl-3,6-heptadiensäure und ihrer pharmazeutisch verwendbaren Salze, dadurch gekennzeichnet, dass man E-2-Formylamino-4-diethylphosphonomethyl-3,6-heptadiensäuremethylester in Dichlormethan mit Trimethyljodsilan umsetzt und das Primärprodukt mit Salzsäure behandelt.

45. Verfahren gemäss einem der Ansprüche 1-44, dadurch gekennzeichnet, dass man dasjenige Enantiomere herstellt, in dem das C-Atom, welches die Aminogruppe $R_6$ trägt, R-Konfiguration besitzt.

46. Verfahren gemäss einem der Ansprüche 1, 2 und 4-24, dadurch gekennzeichnet, dass man (2R)-E-2-Amino-4-methyl-5-phosphono-3-pentensäure oder ein pharmazeutisch verwendbares Salz davon herstellt.

47. Verfahren gemäss Anspruch 26, dadruch gekennzeichnet, dass man (2R)-E-2-Amino-4-methyl-5-phosphono-3-pentensäure oder ein pharmazeutisch verwendbares Salz davon herstellt.

48. Verfahren gemäss einem der Ansprüche 1, 2 und 4-24, dadurhc gekennzeichnet, dass man (2R)-E-2-Amino-4-methyl-5-phosphono-3-pentensäureethylester oder ein pharmazeutisch verwendbares Salz davon herstellt.

49. Verfahren gemäss einem der Ansprüche 3, 5, 6, 8, 12, 15, 18, 20 und 23, dadurch gekennzeichnet, dass man (2R)-E-2-Amino-4-methyl-5-phosphono-3-pentensäureethylester oder ein pharmazeutisch verwendbares Salz davon herstellt.

50. Verfahren zur Herstellung pharmazeutischer Präparate, dadurch gekennzeichnet, dass man (2R)-E-2-Amino-4-methyl-5-phosphono-3-pentensäureethylester oder ein pharmazeutisch verwendbares Salz davon mit üblichen pharmazeutischen Hilfsstoffen vermischt.